# EUROPEAN PATENT SPECIFICATION

(11) **EP 2 330 200 B1**
(45) Date of publication and mention of the grant of the patent: **05.04.2017**
(21) Application number: 10178934.5
(22) Date of filing: 23.12.2005
(51) Int. Cl.: C07K 14/79, C07K 14/395, C12N 9/90, C12N 15/81

(54) **GENE EXPRESSION TECHNIQUE**
Genexpressionstechnik
Technique d'expression génique

(30) Priority: 23.12.2004 WO PCT/GB2004/005435; 23.12.2004 WO PCT/GB2004/005462
(43) Date of publication of application: 08.06.2011
(62) Divisional of application: 05823505.2
(73) Proprietor: Albumedix A/S, 2880 Kgs. Lyngby (DK)
(72) Inventor: SHUTTLEWORTH, Gillian, Nottingham, Nottinghamshire, NG2 6DW (GB); SLEEP, Darrell, Nottingham, Nottinghamshire NG2 5DS (GB); FINNIS, Christopher, John, Arthur, Nottingham, Nottinghamshire NG7 1JB (GB)
(74) Representative: Williams, Rachel Clare

(56) References cited:
- WO-A1-93/25676
- WO-A1-2005/061718
- WO-A2-01/72783
- HJELMQVIST LARS ET AL: "ORMDL proteins are a conserved new family of endoplasmic reticulum membrane proteins", GENOME BIOLOGY, BIOMED CENTRAL LTD., LONDON, GB, vol. 3, no. 6, 14 May 2002 (2002-05-14), XP021012719, ISSN: 1465-6906, DOI: 10.1186/GB-2002-3-6-RESEARCH0027
- BAO W-G ET AL: "Secretion of human proteins from yeast: stimulation by duplication of polyubiquitin and protein disulfide isomerase genes in Kluyveromyces lactis", GENE: AN INTERNATIONAL JOURNAL ON GENES AND GENOMES, ELSEVIER SCIENCE PUBLISHERS, BARKING, GB, vol. 272, no. 1-2, 11 July 2001 (2001-07-11), pages 103-110, XP004274844, ISSN: 0378-1119
- SINA GHAEMMAGHAMI ET AL: 'Global analysis of protein expression in yeast' NATURE vol. 425, no. 6959, 16 October 2003, pages 737 - 741, XP055038190 DOI: 10.1038/nature02046 ISSN: 0028-0836
- KAROLYNN J HSU ET AL: "Functional analysis of the impact of ORMDL3 expression on inflammation and activation of the unfolded protein response in human airway epithelial cells", ALLERGY, ASTHMA & CLINICAL IMMUNOLOGY, BIOMED CENTRAL LTD, LONDON, UK, vol. 9, no. 1, 1 February 2013 (2013-02-01), page 4, XP021148428, ISSN: 1710-1492, DOI: 10.1186/1710-1492-9-4
- Sina Ghaemmaghami ET AL: "Global analysis of protein expression in yeast", Nature, vol. 425, no. 6959, 16 October 2003 (2003-10-16), pages 737-741, XP055038190, ISSN: 0028-0836, DOI: 10.1038/nature02046

## Description

### FIELD OF THE INVENTION

The present application relates to gene expression techniques.

### BACKGROUND OF THE INVENTION

The listing or discussion of a prior-published document in this specification should not necessarily be taken as an acknowledgement that the document is part of the state of the art or is common general knowledge.

The class of proteins known as chaperones has been defined by Hartl (1996, Nature, 381, 571-580) as a protein that binds to and stabilises an otherwise unstable conformer of another protein and, by controlled binding and release, facilitates its correct fate *in vivo*, be it folding, oligomeric assembly, transport to a particular subcellular compartment, or disposal by degradation.

BiP (also known as GRP78, Ig heavy chain binding protein and Kar2p in yeast) is an abundant ~70kDa chaperone of the hsp 70 family, resident in the endoplasmic reticulum (ER), which amongst other functions, serves to assist in transport in the secretory system and fold proteins.

Protein disulphide isomerase (PDI) is a chaperone protein, resident in the ER that is involved in the catalysis of disulphide bond formation during the posttranslational processing of proteins.

Studies of the secretion of both native and foreign proteins have shown that transit from the ER to the Golgi is the rate-limiting step. Evidence points to a transient association of the BiP with normal proteins and a more stable interaction with mutant or misfolded forms of a protein. As a result, BiP may play a dual role in solubilising folding precursors and preventing the transport of unfolded and unassembled proteins. Robinson and Wittrup, 1995, Biotechnol. Prog. 11, 171-177, have examined the effect of foreign protein secretion on BiP (Kar2p) and PDI protein levels in *Saccharomyces cerevisiae* and found that prolonged constitutive expression of foreign secreted proteins reduces soluble BiP and PDI to levels undetectable by Western analysis. The lowering of ER chaperone and foldase levels as a consequence of heterologous protein secretion has important implications for attempts to improve yeast expression/secretion systems.

Expression of chaperones is regulated by a number of mechanisms, including the unfolded protein response (UPR). WO01/72783 discloses methods for increasing the amount of protein secreted by a cell for example by providing a cell which contains a heterologous nucleic acid encoding a protein having unfolded protein response modulating activity and a heterologous nucleic acid encoding a protein of interest to be secreted.

Using recombinant techniques, multiple PDI gene copies have been shown to increase PDI protein levels in a host cell (Farquhar et al, 1991, Gene, 108, 81-89).

Co-expression of the gene encoding PDI and a gene encoding a heterologous disulphide-bonded protein was first suggested in WO 93/25676, published on 23 December 1993, as a means of increasing the production of the heterologous protein. WO 93/25676 reports that the recombinant expression of antistasin and tick anticoagulant protein can be increased by co-expression with PDI.

This strategy has been exploited to increase the recombinant expression of other types of protein.

Robinson et al, 1994, Bio/Technology, 12, 381-384 reported that a recombinant additional PDI gene copy in *Saccharomyces cerevisiae* could be used to increase the recombinant expression of human platelet derived growth factor (PDGF) B homodimer by ten-fold and *Schizosacharomyces pombe* acid phosphatase by fourfold.

Hayano et al, 1995, FEBS Letters, 377, 505-511 described the co-expression of human lysozyme and PDI in yeast. Increases of around 30-60% in functional lysozyme production and secretion were observed.

Shusta et al, 1998, Nature Biotechnology, 16, 773-777 reported that the recombinant expression of single-chain antibody fragments (scFv) in *Saccharomyces cerevisiae* could be increased by between 2-8 fold by over-expressing PDI in the host cell.

Bao & Fukuhara, 2001, Gene, 272, 103-110 reported that the expression and secretion of recombinant human serum albumin (rHSA) in the yeast *Kluyveromyces lactis* could be increased by 15-fold or more by co-expression with an additional recombinant copy of the yeast PDI gene (*KIPDI1*).

In order to produce co-transformed yeast comprising both a PDI gene and a gene for a heterologous protein, WO 93/25676 taught that the two genes could be chromosomally integrated; one could be chromosomally integrated and one present on a plasmid; each gene could be introduced on a different plasmid; or both genes could be introduced on the same plasmid. WO 93/25676 exemplified expression of antistasin from the plasmid pKH4α2 in yeast strains having a chromosomally integrated additional copy of a PDI gene (Examples 16 and 17); expression of antistasin from the vector K991 with an additional PDI gene copy being present on a multicopy yeast shuttle vector named YEp24 (Botstein et al, 1979, Gene, 8, 17-24) (Example 20); and expression of both the antistasin and the PDI genes from the yeast shuttle vector pC1/1 (Rosenberg et al, 1984, Nature, 312, 77-80) under control of the GAL10 and GAL1 promoters, respectively. Indeed, Robinson and Wittrup, 1995, *op. cit*., also used the GAL1-GAL10 intergenic region to express erythropoietin and concluded that production yeast strains for the secretion of heterologous proteins should be constructed using tightly repressible, inducible promoters, otherwise the negative effects of sustained secretion (i.e. lowered detectable BiP and PDI) would be dominant after the many generations of cell growth required to fill a large-scale fermenter.

Subsequent work in the field has identified chromosomal integration of transgenes as the key to maximising recombinant protein production.

Robinson *et al*, 1994, *op. cit*., obtained the observed increases in expression of PDGF and *S. pombe* acid phosphatase using an additional chromosomally integrated PDI gene copy. Robinson *et al* reported that attempts to use the multi-copy 2µm expression vector to increase PDI protein levels had had a detrimental effect on heterologous protein secretion.

Hayano *et al*, 1995, *op. cit.* described the introduction of genes for human lysozyme and PDI into a yeast host each on a separate linearised integration vector, thereby to bring about chromosomal integration.

Shusta *et al*, 1998, *op. cit*., reported that in yeast systems, the choice between integration of a transgene into the host chromosome versus the use of episomal expression vectors can greatly affect secretion and, with reference to Parekh & Wittrup, 1997, Biotechnol. Prog., 13, 117-122, that stable integration of the scFv gene into the host chromosome using a δ integration vector was superior to the use of a 2µm-based expression plasmid. Parekh & Wittrup, *op. cit*., had previously taught that the expression of bovine pancreatic trypsin inhibitor (BPTI) was increased by an order of magnitude using a δ integration vector rather than a 2µm-based expression plasmid. The 2µm-based expression plasmid was said to be counter-productive for the production of heterologous secreted protein.

Bao & Fukuhara, 2001, *op. cit*., reported that "It was first thought that the *KlPDI1* gene might be directly introduced into the multi-copy vector that carried the rHSA expression cassette. However, such constructs were found to severely affect yeast growth and plasmid stability. This confirmed our previous finding that the *KlPDI1* gene on a multi-copy vector was detrimental to growth of *K. lactis* cells (Bao *et al*, 2000)". Bao et al, 2000, Yeast, 16, 329-341, as referred to in the above-quoted passage of Bao & Fukuhara, reported that the *KlPDI1* gene had been introduced into *K. lactis* on a multi-copy plasmid, pKan707, and that the presence of the plasmid caused the strain to grow poorly. Bao *et al* concluded that over-expression of the *KlPDI1* gene was toxic to *K. lactis* cells. In the light of the earlier findings in Bao *et al*, Bao & Fukuhara chose to introduce a single duplication of *KlPDI1* on the host chromosome.

Against this background, we had previously surprisingly demonstrated that, contrary to the suggestions in the prior art, when the genes for a chaperone protein and a heterologous protein are co-expressed on a 2µm-family multi-copy plasmid in yeast, the production of the heterologous protein is substantially increased.

Our earlier application, which has been published as WO 2005/061718, from which this application claims priority, disclosed a method for producing heterologous protein comprising:
(a) providing a host cell comprising a 2µm-family plasmid, the plasmid comprising a gene encoding a protein comprising the sequence of a chaperone protein and a gene encoding a heterologous protein;
(b) culturing the host cell in a culture medium under conditions that allow the expression of the gene encoding the chaperone protein and the gene encoding a heterologous protein;
(c) purifying the thus expressed heterologous protein from the culture medium; and
(d) optionally, lyophilising the thus purified protein.

As discussed above, Bao et al, 2000, Yeast, 16, 329-341 reported that over-expression of the *K. lactis* PDI gene *KlPDI1* was toxic to *K. lactis* cells. Against this background we have surprisingly found that, not only is it possible to over-express PDI and other chaperones without the detrimental effects reported in Bao *et al*, but that two different chaperones can be recombinantly over-expressed in the same cell and, rather than being toxic, can increase the expression of proteins, including heterologous proteins, to levels higher than the levels obtained by individual expression of either of the chaperones. This was not expected. On the contrary, in light of the teaching of Bao *et al*, one would think that over-expression of two chaperones would be even more toxic than the over-expression of one. Moreover, in light of some initial findings which are also reported below in the present application, it was expected that the increases in heterologous protein expression obtained by co-expression with a single chaperone would be at the maximum level possible for the cell system used. Therefore, it was particularly surprising to find that yet further increases in protein expression could be obtained by co-expression of two different chaperones with the protein.

### SUMMARY OF THE INVENTION

The main aspect of the invention provides a method for producing a desired protein (such as a desired heterologous protein), comprising:
(a) providing a fungal or yeast host cell comprising a first recombinant gene encoding the protein comprising the sequence of Orm2p or a variant thereof having equivalent chaperone-like activity and a second gene, optionally a second recombinant gene, encoding a desired protein (such as a desired heterologous protein), with the proviso that the first and second genes are not both present within the host cell on the same 2µm-family plasmid; and
   culturing the host cell in a culture medium under conditions that allow the expression of the first and second genes,
   wherein
   Orm2p has the following sequence: and the variant of Orm2p has at least 70% sequence identity to: and the fragment of Orm2p has at least 70% of the complete sequence of:

The method may, or may not, further comprise the step of purifying the thus expressed desired protein (such as a desired heterologous protein) from the cultured host cell or the culture medium; and optionally, lyophilising the thus purified protein; and optionally formulating the purified desired protein (such as a desired heterologous protein) with a carrier or diluent; and optionally presenting the thus formulated protein in a unit dosage form.

The host cell may, or may not, further comprise a further recombinant gene encoding a protein comprising the sequence of an alternative chaperone to Orm2p or a variant thereof.

Either or both of the first and second genes of the invention may or may not be recombinant genes that are expressed from a plasmid, and optionally from the same plasmid, provided that where both genes are expressed from the same plasmid then that plasmid is not a 2µm-family plasmid. A further recombinant gene encoding a protein comprising the sequence of an alternative chaperone to Orm2p or a variant thereof may, or may not, also be expressed from a plasmid, optionally from the same plasmid as either or both of the first and second recombinant genes. Except where both of the first and second genes are recombinant genes that are co-expressed from the same plasmid then either one may, or may not, be individually expressed from a 2µm-family plasmid, such as the 2µm plasmid. Alternatively, one or both of the first and second genes of the invention may, or may not, be integrated into the chromosome of the host cell. The further recombinant gene encoding a protein comprising the sequence of an alternative chaperone to Orm2p or a variant thereof may, or may not, be integrated into the chromosome of the host cell, irrespective of whether or not the first and second genes are expressed from a plasmid or are chromosomally integrated.

The main aspect of the present invention also provides, a fungal or yeast host cell comprising (i) a plasmid comprising a first recombinant gene encoding a protein comprising the sequence of Orm2p or a variant or fragment thereof having equivalent chaperone-like activity, and (ii) and a second gene, such as a recombinant gene, encoding a desired protein (such as a desired heterologous protein), with the proviso that the first and second genes are not present within the host cell on the same 2µm-family plasmid,
wherein
Orm2p has the following sequence: and the variant of Orm2p has at least 70% sequence identity to: and the fragment of Orm2p has at least 70% of the complete sequence of:

The main aspect of the present invention also provides for the use of a recombinant gene encoding a protein comprising the sequence of Orm2p or a variant or fragment thereof having equivalent chaperone-like activity to increase the expression of a desired protein in a fungal or yeast host cell, with the proviso that the recombinant gene encoding a protein comprising the sequence of Orm2p or a variant or fragment thereof and the gene encoding the desired protein are not co-expressed within the host cell from the same 2µm-family plasmid
wherein
Orm2p has the following sequence: and the variant of Orm2p has at least 70% sequence identity to: and the fragment of Orm2p has at least 70% of the complete sequence of:

The main aspect of the present invention also provides for the use of a nucleic acid sequence encoding the protein Orm2p or a variant thereof to increase the production, in a host cell (such as a host cell as defined above), of a desired protein (such as a desired heterologous protein) encoded by a gene, such as a recombinant gene, in the host cell by co-expression of the nucleic acid sequence and the gene within the host cell (but optionally not including co-expression of these genes from the same 2µm-family plasmid). Either or both of the nucleic acid sequence and the gene encoding the desired protein may, or may not, be expressed from a plasmid within the host cell, and optionally from the same plasmid. In the manner discussed above, the host cell may, or may not, further comprise a recombinant gene encoding an alternative chaperone to Orm2p or a variant thereof, which may, or may not, be located on a plasmid within the host cell, optionally on the same plasmid as either or both of the nucleic acid sequence and a gene encoding the desired protein. Suitable plasmids include a 2µm-family plasmid, such as the 2µm plasmid.

The main aspect of the present invention also provides for the use of a plasmid as an expression vector to increase the production of a heterologous protein by providing a recombinant gene encoding the heterologous protein and a gene encoding Orm2p or a variant thereof on the same plasmid, optionally with the proviso that the plasmid is not a 2µm-family plasmid. The plasmid may, or may not, further comprise a gene encoding an alternative chaperone to Orm2p or a variant thereof.

The main aspect of the present invention also provides a plasmid, optionally an expression plasmid, comprising a first gene encoding the protein Orm2p or a variant or fragment thereof and a second gene encoding a heterologous protein, as discussed above, optionally with the proviso that the plasmid is not a 2µm-family plasmid. The plasmid may, or may not, further comprise a third gene encoding an alternative chaperone to Orm2p or a variant thereof. In a preferred embodiment, the third gene encodes a protein comprising the sequence of protein disulphide isomerase.

Furthermore, as a first aspect there is provided a method for producing a desired protein, such as a heterologous protein, comprising providing a host cell comprising a first recombinant gene encoding a protein comprising the sequence of a first chaperone protein, a second recombinant gene encoding a protein comprising the sequence of a second chaperone protein and a third gene (optionally the third gene being recombinant) encoding the desired protein (optionally a heterologous protein), wherein the first and second chaperones are different; and culturing the host cell in a culture medium under conditions that allow the expression of the first, second and third genes.

Optionally the thus expressed desired protein may, or may not, be purified from the cultured host cell or the culture medium.

Optionally, the thus purified desired protein may, or may not, be lyophilised.

The method may, or may not, further comprise the step of formulating the purified desired protein with a carrier or diluent and optionally presenting the thus formulated protein in a unit dosage form, in the manner discussed above.

The term "recombinant gene" includes nucleic acid sequences that operate independently as "stand alone" expressible sequences to produce an encoded protein or, in the alternative, nucleic acid sequences introduced that operate in combination with endogenous sequences (such as by integration into an endogenous sequence so as to produce a nucleic acid sequence that is different to the endogenous sequence) within the host to cause increased expression of a target protein.

A "recombinant gene" is typically a gene that is not naturally found in the context used. For example, a gene that is integrated, at an integration site, into the chromosome of a host organism can be said to be a "recombinant gene" if it comprises a sequence that does not naturally occur at the integration site. Thus, the "recombinant gene" may, or may not, comprise a non-natural sequence in the coding, regulatory or any other region of the gene, or may, or may not, comprise the sequence of a naturally occurring gene but be introduced into the chromosome of a host organism at an integration site at which that sequence does not naturally occur. The same issues apply, *mutatis mutandis*, to the insertion of a "recombinant gene" into a plasmid.

The terms "chromosomally integrated" and "integrated into the chromosome of the host cell" are well recognised terms of the art. For avoidance of doubt, these terms include the integration of polynucleotide sequences in any inheritable nuclear material that naturally occurs in a host cell, other than for naturally occurring plasmids. Thus, a polynucleotide sequence that is "integrated into the chromosome of the host cell" may, or may not, be integrated into the chromosome of a procaryotic (such as a bacterial) cell, or into any part of the genome of a eucaryotic cell, such as into nuclear genetic material including the chromosome (or, one of the chromosomes), the mitochondrial genome or the chloroplast genome.

The first and second chaperones may, or may not, each individually, be one of the specifically listed chaperones as discussed below, and are a combination of chaperones that, when co-expressed in the same host cell, provide at least an additive effect to the increase in expression of the desired protein. By "additive effect" we mean that the level of expression of the desired protein in the host cell is higher when the first and second recombinant genes are simultaneously co-expressed with the third gene as compared to the same system wherein (i) the first recombinant gene is co-expressed with the third gene in the absence of the expression of the second recombinant gene and (ii) the second recombinant gene is co-expressed with the third gene in the absence of the expression of the first recombinant gene.

The term "chaperone" as used herein refers to a protein that binds to and stabilises an otherwise unstable conformer of another protein, and by controlled binding and release, facilitates its correct fate *in vivo*, be it folding, oligomeric assembly, transport to a particular subcellular compartment, or disposal by degradation. Accordingly a chaperone is also a protein that is involved in protein folding, or which has chaperone activity or is involved in the unfolded protein response. Chaperone proteins of this type are known in the art, for example in the Stanford Genome Database (SGD), http://db.yeastgenome.org or http://www.yeastgenome.org. Preferred chaperones are eucaryotic chaperones, especially preferred chaperones are yeast chaperones, including *AHA1*, *CCT2, CCT3*, *CCT4*, *CCT5*, *CCT6*, *CCT7*, *CCT8*, *CNS1*, *CPR3*, *CPR6*, *ERO1*, *EUG1*, *FMO1*, *HCH1*, *HSP10*, *HSP12*, *HSP104*, *HSP26, HSP30*, *HSP42*, *HSP60*, *HSP78*, *HSP82, JEM1*, *MDJ1*, *MDJ2*, *MPD1*, *MPD2*, *PDI1*, *PFD1*, *ABC1*, *APJ1*, *ATP11*, *ATP12*, *BTT1*, *CDC37, CPR7*, *HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *ORM1*, *ORM2*, *PER1*, *PTC2*, *PSE1*, *UBI4* and *HAC1* or a truncated intronless *HAC1* (Valkonen et al. 2003, Applied Environ. Micro., 69, 2065), as well as *TIM9*, *PAM18* (also known as *TIM14*) and *TCP1* (also known as *CCT1)*

A chaperone useful in the practice of the present invention may, or may not, be:
- a heat shock protein, such as a protein that is a member of the hsp70 family of proteins (including Kar2p, SSA and SSB proteins, for example proteins encoded by *SSA1*, *SSA2*, *SSA3*, *SSA4*, *SSB1* and *SSB2*), a protein that is a member of the HSP90-family, or a protein that is a member of the HSP40-family or proteins involved in their modulation (e.g. Sil1p), including DNA-J and DNA-J-like proteins (e.g. Jem1p, Mdj2p);
- a protein that is a member of the karyopherin/importin family of proteins, such as the alpha or beta families of karyopherin/importin proteins, for example the karyopherin beta protein PSE1;
- a protein that is a member of the ORMDL family described by Hjelmqvist et al, 2002, Genome Biology, 3(6), research0027.1-0027.16, such as Orm2p.
- a protein that is naturally located in the endoplasmic reticulum or elsewhere in the secretory pathway, such as the golgi. For example, a protein that naturally acts in the lumen of the endoplasmic reticulum (ER), particularly in secretory cells. such as PDI
- a protein that is transmembrane protein anchored in the ER, such as a member of the ORMDL family described by Hjelmqvist *et al*, 2002, *supra*, (for example, Orm2p);
- a protein that acts in the cytosol, such as the hsp70 proteins, including SSA and SSB proteins, for example protein production *SSA1, SSA2, SSA3, SSA4, SSB1* and *SSB2;*
- a protein that acts in the nucleus, the nuclear envelope and/or the cytoplasm, such as Pse1p;
- a protein that is "essential" to the viability of the cell, such as PDI, or a protein encoded by one of the following genes: *CCT2, CCT3*, *CCT4, CCT5*, *CCT6*, *CCT7*, *CCT8*, *CNS1*, *ERO1*, *HSP10*, *HSP60*, *PDI1*, *CDC37, KAR2*, *MGE1*, *MRS11*, *NOB1*, *SSC1*, *TIM9, PAM18* and *TCP1*, or a protein that is an essential karyopherin protein, such as Pselp;
- a protein that is involved in sulphydryl oxidation or disulphide bond formation, breakage or isomerization, or a protein that catalyses thiol:disulphide interchange reactions in proteins, particularly during the biosynthesis of secretory and cell surface proteins, such as protein disulphide isomerases (e.g. Pdi1p, Mpd1p), homologues (e.g. Euglp) and/or related proteins (e.g. Mpd2p, Fmo1p, Ero1p);
- a protein that is involved in protein synthesis, assembly or folding, such as PDI and Ssalp;
- a protein that binds preferentially or exclusively to unfolded, rather than mature protein, such as the hsp70 proteins, including SSA and SSB proteins, for example proteins encoded by *SSA1, SSA2, SSA3, SSA4, SSB1* and *SSB2*;
- a protein that prevents aggregation of precursor proteins in the cytosol, such as the hsp70 proteins, including SSA and SSB proteins, for example proteins encoded *by SSA1, SSA2, SSA3, SSA4, SSB1* and *SSB2;*
- a protein that binds to and stabilises damaged proteins, for example Ssa1p;
- a protein that is involved in the unfolded protein response or provides for increased resistance to agents (such as tunicamycin and dithiothreitol) that induce the unfolded protein response, such as a member of the ORMDL family described by Hjelmqvist *et al*, 2002, *supra* (for example, Orm2p) or proteins involved in the response to stress (e.g. Ubi4p);
- a protein that is a co-chaperone and/or a protein indirectly involved in protein folding and/or the unfolded protein response (e.g. hsp104p, Mdj1p);
- a protein that is involved in the nucleocytoplasmic transport of macromolecules, such as Pselp;
- a protein that mediates the transport of macromolecules across the nuclear membrane by recognising nuclear location sequences and nuclear export sequences and interacting with the nuclear pore complex, such as PSE1;
- a protein that is able to reactivate ribonuclease activity against RNA of scrambled ribonuclease as described in as described in EP 0 746 611 and Hillson et al, 1984, Methods Enzymol., 107, 281-292, such as PDI;
- a protein that has an acidic pI (for example, 4.0-4.5), such as PDI;
- a protein that is a member of the Hsp70 family, and optionally possesses an N-terminal ATP-binding domain and a C-terminal peptide-binding domain, such as Ssalp.
- a protein that is a peptidyl-prolyl cis-trans isomerases (e.g. Cpr3p, Cpr6p);
- a protein that is a homologue of known chaperones (e.g. Hsp10p);
- a protein that is a mitochondrial chaperone (e.g Cpr3p);
- a protein that is a cytoplasmic or nuclear chaperone (e.g Cns1p);
- a protein that is a membrane-bound chaperone (e.g. Orm2p, Fmo1p);
- a protein that has chaperone activator activity or chaperone regulatory activity (e.g. Aha1p, Hac1p, Hch1p);
- a protein that transiently binds to polypeptides in their immature form to cause proper folding transportation and/or secretion, including proteins required for efficient translocation into the endoplasmic reticulum (e.g. Lhs1p) or their site of action within the cell (e.g. PseIp);
- a protein that is a involved in protein complex assembly and/or ribosome assembly (e.g. Atp11p, PseIp, Nob1p);
- a protein of the chaperonin T-complex (e.g. Cct2p);
- a protein of the prefoldin complex (e.g. Pfd1p);
- a mitochondrial intermembrane space protein such as Tim9p;
- a protein that can form a complex, *in vivo*, with Mrs11p/Tim10p, such as Tim9p;
- a protein that is involved in the mediation of import and insertion of polytopic inner membrane proteins, such as Tim9p;
- a protein that can prevent the aggregation of incoming proteins, such as Tim9p;
- a protein that can be a functional constituent of the mitochondrial import motor associated with presequence translocase (along with Ssclp, Tim44p, Mge1p and Pam16p) such as Pam18p;
- a protein that can stimulate the ATPase activity of Ssclp, such as to drive mitochondrial import, such as Pam18p;
- a protein that contains a J domain, such as Pam18p;
- a protein that can act as an alpha subunit of chaperonin-containing T-complex, which mediates protein folding in the cytosol, such as Tcp1p;
- a protein that can play a role in the in maintenance of an actin cytoskeleton, such as Tcp1p; or
- a protein that is, or is a homolog to, a *Drosophilia melanogaster* or mouse tailless complex polypeptide, such as Tcp1p.

A preferred chaperone is protein disulphide isomerase (PDI) or a fragment or variant thereof having an equivalent ability to catalyse the formation of disulphide bonds within the lumen of the endoplasmic reticulum (ER). By "PDI" we include any protein having the ability to reactivate the ribonuclease activity against RNA of scrambled ribonuclease as described in EP 0 746 611 and Hillson et al, 1984, Methods Enzymol., 107, 281-292.

PDI is an enzyme which typically catalyzes thiol:disulphide interchange reactions, and is a major resident protein component of the ER lumen in secretory cells. A body of evidence suggests that it plays a role in secretory protein biosynthesis (Freedman, 1984, Trends Biochem. Sci., 9, 438-41) and this is supported by direct cross-linking studies *in situ* (Roth and Pierce, 1987, Biochemistry, 26, 4179-82). The finding that microsomal membranes deficient in PDI show a specific defect in cotranslational protein disulphide (Bulleid and Freedman, 1988, Nature, 335, 649-51) implies that the enzyme functions as a catalyst of native disulphide bond formation during the biosynthesis of secretory and cell surface proteins. This role is consistent with what is known of the enzyme's catalytic properties *in vitro*; it catalyzes thiol: disulphide interchange reactions leading to net protein disulphide formation, breakage or isomerization, and can typically catalyze protein folding and the formation of native disulphide bonds in a wide variety of reduced, unfolded protein substrates (Freedman et al., 1989, Biochem. Soc. Symp., 55, 167-192). PDI also functions as a chaperone since mutant PDI lacking isomerase activity accelerates protein folding (Hayano et al, 1995, FEBS Letters, 377, 505-511). Recently, sulphydryl oxidation, not disulphide isomerisation was reported to be the principal function of Protein Disulphide Isomerase in *S. cerevisiae* (Solovyov et al., 2004, J. Biol. Chem., 279 (33) 34095-34100). The DNA and amino acid sequence of the enzyme is known for several species (Scherens et al, 1991, Yeast, 7, 185-193; Farquhar et al, 1991, Gene, 108, 81-89; EP074661; EP0293793; EP0509841) and there is increasing information on the mechanism of action of the enzyme purified to homogeneity from mammalian liver (Creighton et al, 1980, J. Mol. Biol., 142, 43-62; Freedman et al, 1988, Biochem. Soc. Trans., 16, 96-9; Gilbert, 1989, Biochemistry, 28, 7298-7305; Lundstrom and Holmgren, 1990, J. Biol. Chem., 265, 9114-9120; Hawkins and Freedman, 1990, Biochem. J., 275, 335-339). Of the many protein factors currently implicated as mediators of protein folding, assembly and translocation in the cell (Rothman, 1989, Cell, 59, 591-601), PDI has a well-defined catalytic activity.

The deletion or inactivation of the endogenous PDI gene in a host results in the production of an inviable host. In other words, the endogenous PDI gene is an "essential" gene.

PDI is readily isolated from mammalian tissues and the homogeneous enzyme is a homodimer (2x57 kD) with characteristically acidic pI (4.0-4.5) (Hillson *et al*, 1984, *op. cit*.)*.* The enzyme has also been purified from wheat and from the alga *Chlamydomonas reinhardii* (Kaska et al, 1990, Biochem. J., 268, 63-68), rat (Edman et al, 1985, Nature, 317, 267-270), bovine (Yamauchi et al, 1987, Biochem. Biophys. Res. Comm., 146, 1485-1492), human (Pihlajaniemi et al, 1987, EMBO J., 6, 643-9), yeast (Scherens *et al*, *supra*; Farquhar *et al*, *op. cit*.) and chick (Parkkonen et al, 1988, Biochem. J., 256, 1005-1011). The proteins from these vertebrate species show a high degree of sequence conservation throughout and all show several overall features first noted in the rat PDI sequence (Edman *et al*., 1985, *op. cit*.).

Preferred PDI sequences include those from humans and those from yeast species, such as *S. cerevisiae.*

A yeast protein disulphide isomerase precursor, PDI1, can be found as Genbank accession no. CAA42373 or BAA00723 and has a sequence of 522 amino acids as described in WO 2005/061718.

An alternative yeast protein disulphide isomerase sequence can be found as Genbank accession no. CAA38402, which has a sequence of 530 amino acids as described in WO 2005/061718.

The alignment of these sequences (the sequence of Genbank accession no. CAA42373 or BAA00723 first, the sequence of Genbank accession no. CAA38402 second) in WO 2005/061718, shows that the differences between these two sequences are a single amino acid difference at position 114 (highlighted in bold) and that the sequence defined by Genbank accession no. CAA38402 contains the additional amino acids EADAEAEA at positions 506-513.

Variants and fragments of the above PDI sequences, and variants of other naturally occurring PDI sequences are also included in the present invention. A "variant", in the context of PDI, refers to a protein wherein at one or more positions there have been amino acid insertions, deletions, or substitutions, either conservative or non-conservative, provided that such changes result in a protein whose basic properties, for example enzymatic activity (type of and specific activity), thermo stability, activity in a certain pH-range (pH-stability) have not significantly been changed. "Significantly" in this context means that one skilled in the art would say that the properties of the variant may, or may not, still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" typically has at least 25%, at least 50%, at least 60% or at least 70%, preferably at least 80%, more preferably at least 90%, even more preferably at least 95%, yet more preferably at least 99%, most preferably at least 99.5% sequence identity to the polypeptide from which it is derived.

The percent sequence identity between two polypeptides may be determined using suitable computer programs, as discussed below. Such variants may, or may not, be natural or made using the methods of protein engineering and site-directed mutagenesis as are well known in the art.

A "fragment", in the context of PDI, refers to a protein wherein at one or more positions there have been deletions. Thus the fragment may, or may not, comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature PDI protein. Particularly preferred fragments of PDI protein comprise one or more whole domains of the desired protein.

A fragment or variant of PDI may, or may not, be a protein that, when expressed recombinantly in a host cell, can complement the deletion of the endogenously encoded PDI gene in the host cell, such as *S. cerevisiae*, and may, or may not, for example, be a naturally occurring homolog of PDI, such as a homolog encoded by another organism, such as another yeast or other fungi, or another eucaryote such as a human or other vertebrate, or animal or by a plant.

Where the first chaperone is PDI, particularly mammalian or yeast PDI, then in one embodiment the second chaperone is not an hsp70 chaperone protein (such as yeast KAR2, HSP70, BiP, SSA1-4, SSB1, SSC1, SSD1 or a eucaryotic hsp70 protein such as HSP68, HSP72, HSP73, HSC70, clathrin uncoating ATPase, IgG heavy chain binding protein (BiP), glucose-regulated proteins 75, 78 and 80 (GPR75, GRP78 and GRP80) and the like). Specifically in one embodiment the first chaperone is not yeast PDI when the second chaperone is yeast KAR2. Specifically in another embodiment the first chaperone is not mammalian PDI when the second chaperone is mammalian BiP.

Alternatively, where the first and second chaperones are, for example, PDI, particularly mammalian or yeast PDI, and an hsp70 chaperone protein as described above, respectively, then the desired protein may be a heterologous protein that may or may not be a protein selected from -
- mammalian gene products such as enzymes, cytokines, growth factors, hormones, vaccines, antibodies and the like; erythropoietin, insulin, somatotropin, growth hormone releasing factor, platelet derived growth factor, epidermal growth factor, transforming growth factor α, transforming growth factor β, epidermal growth factor, fibroblast growth factor, nerve growth factor, insulin-like growth factor I, insulin-like growth factor II, clotting Factor VIII, superoxide dismutase, α-interferon, γ-interferon, interleukin-1, interleukin-2, interleukin-3, interleukin-4, interleukin-5, interleukin-6, granulocyte colony stimulating factor, multi-lineage colony stimulating activity, granulocyte-macrophage stimulating factor, macrophage colony stimulating factor, T cell growth factor, lymphotoxin and the like; or human gene products;
- any gene product which can be used as a vaccine, including any structural, membrane-associated, membrane-bound or secreted gene product of a mammalian pathogen, including viruses, bacteria, single-celled or multi-celled parasites which can infect or attack a mammal, in particular viruses such as human immunodeficiency virus (HIV), *R. rickettsii*, vaccinia, Shigella, poliovirus, adenovirus, influenza, hepatitis A, hepatitis B, dengue virus, Japanese B encephalitis, *Varicella roster*, cytomegalovirus, hepatitis A, rotavirus, as well as vaccines against viral diseases like Lyme disease, measles, yellow fever, mumps, rabies, herpes, influenza, parainfluenza and the like; or bacteria such as *Vibrio cholerae*, *Salmonella typhi*, *Bordetella pertussis*, *Streptococcus pneumoniae*, *Hemophilus influenza, Clostridium tetani*, *Corynebacterium diphtheriae*, *Mycobacterium leprae*, *Neisseriaqonorrhoeae*, *Neisseriameningitidis*, *Coccidioides immitis* and the like.

Another preferred chaperone is a protein comprising the sequence of a protein encoded by the gene *SSA1*, or a fragment or variant thereof having an equivalent chaperone-like activity. *SSA1*, also known as YG100, is located on chromosome I of the *S. cerevisiae* genome and is 1.93-kbp in size.

One published protein sequence of the protein encoded by the gene *SSA1* is as described in WO 2005/061718.

A published coding sequence for *SSA1* is also described in WO 2005/061718, although it will be appreciated that the sequence can be modified by degenerate substitutions to obtain alternative nucleotide sequences which encode an identical protein product.

The protein Ssalp belongs to the Hsp70 family of proteins and is resident in the cytosol. Hsp70s possess the ability to perform a number of chaperone activities; aiding protein synthesis, assembly and folding; mediating translocation of polypeptides to various intracellular locations, and resolution of protein aggregates (Becker & Craig, 1994, Eur. J. Biochem. 219, 11-23). Hsp70 genes are highly conserved, possessing an N-terminal ATP-binding domain and a C-terminal peptide-binding domain. Hsp70 proteins interact with the peptide backbone of, mainly unfolded, proteins. The binding and release of peptides by hsp70 proteins is an ATP-dependent process and accompanied by a conformational change in the hsp70 (Becker & Craig, 1994, *supra*).

Cytosolic hsp70 proteins are particularly involved in the synthesis, folding and secretion of proteins (Becker & Craig, 1994, *supra*). In *S. cerevisiae* cytosolic hsp70 proteins have been divided into two groups; SSA (SSA 1-4) and SSB (SSB 1 and 2) proteins, which are functionally distinct from each other. The SSA family is "essential" in that at least one protein from the group must be active to maintain cell viability (Becker & Craig, 1994, *supra*). Cytosolic hsp70 proteins bind preferentially to unfolded and not mature proteins. This suggests that they prevent the aggregation of precursor proteins, by maintaining them in an unfolded state prior to being assembled into multimolecular complexes in the cytosol and/or facilitating their translocation to various organelles (Becker & Craig, 1994, *supra*). SSA proteins are particularly involved in posttranslational biogenesis and maintenance of precursors for translocation into the endoplasmic reticulum and mitochondria (Kim et al., 1998, Proc. Natl. Acad. Sci. USA. 95, 12860-12865; Ngosuwan et al., 2003, J. Biol. Chem. 278 (9), 7034-7042). Ssalp has been shown to bind damaged proteins, stabilising them in a partially unfolded form and allowing refolding or degradation to occur (Becker & Craig, 1994, *supra*; Glover & Lindquist, 1998, Cell. 94, 73-82).

Demolder et al, 1994, J. Biotechnol., 32, 179-189 reported that over-expression of *SSA1* in yeast provided for increases in the expression of a recombinant chromosomally integrated gene encoding human interferon-β. There is no suggestion that increases in heterologous gene expression could be achieved if *SSA1* and human interferon-β were to be encoded by recombinant genes on the same plasmid. In fact, in light of more recent developments in the field of over-expression of chaperones in yeast (e.g. Robinson *et al*, 1994, *op. cit.;* Hayano *et al*, 1995, *op. cit.;* Shusta *et al*, 1998, *op. cit;* Parekh & Wittrup, 1997, *op. cit*.; Bao & Fukuhara, 2001, *op. cit.;* and Bao *et al*, 2000, *op. cit* ) the skilled person would have been disinclined to express *SSA1* from a 2µm-family plasmid at all, much less to express both *SSA1* and a heterologous protein from a 2µm-family plasmid in order to increase the expression levels of a heterologous protein.

Variants and fragments of is a protein comprising the sequence of a protein encoded by the gene *SSA1* are also included in the present invention. A "variant", in the context of a protein encoded by the gene *SSA1*, refers to a protein having the sequence of native Ssalp other than at one or more positions where there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" of Ssalp typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native Ssalp.

The percent sequence identity between two polypeptides may be determined using suitable computer programs, as discussed below. Such variants may, or may not, be natural or made using the methods of protein engineering and site-directed mutagenesis as are well known in the art.

A "fragment", in the context of Ssalp, refers to a protein having the sequence of native Ssalp other than for at one or more positions where there have been deletions. Thus the fragment may, or may not, comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature Ssalp protein. Particularly preferred fragments of SSA1 protein comprise one or more whole domains of the desired protein.

A fragment or variant of Ssalp may, or may not, be a protein that, when expressed recombinantly in a host cell, such as *S. cerevisiae*, can complement the deletion of the endogenously encoded *SSA1* gene (or homolog thereof) in the host cell and may, or may not, for example, be a naturally occurring homolog of Ssa1p, such as a homolog encoded by another organism, such as another yeast or other fungi, or another eucaryote such as a human or other vertebrate, or animal or by a plant.

Another preferred chaperone is protein comprising the sequence of a protein encoded by the *PSE1* gene, or a fragment or variant thereof having equivalent chaperone-like activity.

*PSE1,* also known as *KAP121*, is an essential gene, located on chromosome XIII.

A published protein sequence for the protein Pse1p is as described in WO 2005/061718.

A published nucleotide coding sequence of *PSE1* is also described in WO 2005/061718, although it will be appreciated that the sequence can be modified by degenerate substitutions to obtain alternative nucleotide sequences which encode an identical protein product.

The *PSE1* gene is 3.25-kbp in size. Pse1p is involved in the nucleocytoplasmic transport of macromolecules (Seedorf & Silver, 1997, Proc. Natl. Acad. Sci. USA. 94, 8590-8595). This process occurs via the nuclear pore complex (NPC) embedded in the nuclear envelope and made up of nucleoporins (Ryan & Wente, 2000, Curr. Opin. Cell Biol. 12, 361-371). Proteins possess specific sequences that contain the information required for nuclear import, nuclear localisation sequence (NLS) and export, nuclear export sequence (NES) (Pemberton et al., 1998, Curr. Opin. Cell Biol. 10, 392-399). Pse1p is a karyopherin/importin, a group of proteins, which have been divided up into α and β families. Karyopherins are soluble transport factors that mediate the transport of macromolecules across the nuclear membrane by recognising NLS and NES, and interact with and the NPC (Seedorf & Silver, 1997, *supra*; Pemberton *et al*., 1998, *supra*; Ryan & Wente, 2000, *sura*)*.* Translocation through the nuclear pore is driven by GTP hydrolysis, catalysed by the small GTP-binding protein, Ran (Seedorf & Silver, 1997, *sura*)*.* Pse1p has been identified as a karyopherin (3. 14 karyopherin β proteins have been identified in *S. cerevisiae*, of which only 4 are "essential". This is perhaps because multiple karyopherins may mediate the transport of a single macromolecule (Isoyama et al., 2001, J. Biol. Chem. 276 (24), 21863-21869). Pse1p is localised to the nucleus, at the nuclear envelope, and to a certain extent to the cytoplasm. This suggests the protein moves in and out of the nucleus as part of its transport function (Seedorf & Silver, 1997, *supra*). Pselp is involved in the nuclear import of transcription factors (Isoyama *et* al., 2001, *supra*; Ueta et al., 2003, J. Biol. Chem. 278 (50), 50120-50127), histones (Mosammaparast et al., 2002, J. Biol. Chem. 277 (1), 862-868), and ribosomal proteins prior to their assembly into ribosomes (Pemberton *et al*., 1998, *sura*)*.* It also mediates the export of mRNA from the nucleus. Karyopherins recognise and bind distinct NES found on RNA-binding proteins, which coat the RNA before it is exported from the nucleus (Seedorf & Silver, 1997, Pemberton *et al*., 1998, *supra*).

As nucleocytoplasmic transport of macromolecules is essential for proper progression through the cell cycle, nuclear transport factors, such as Pselp are novel candidate targets for growth control (Seedorf & Silver, 1997, *supra*)*.*

Overexpression of Pselp (protein secretion enhancer) in *S. cerevisiae* has also been shown to increase endogenous protein secretion levels of a repertoire of biologically active proteins (Chow *et al*., 1992; J. Cell. Sci. 101 (3), 709-719). There is no suggestion that increases in heterologous gene expression could be achieved if Pse1p and a heterologous protein were both to be encoded by recombinant genes on the same plasmid. In fact, in light of more recent developments in the over-expression of chaperones in yeast (e.g. Robinson *et al*, 1994, *op. cit.;* Hayano *et al*, 1995, *op. cit.;* Shusta *et al*, 1998, *op. cit;* Parekh & Wittrup, 1997, *op. cit*.; Bao & Fukuhara, 2001, *op. cit.;* and Bao *et al*, 2000, *op. cit*) the skilled person would not have attempted to over-express a *PSE1* gene from a 2µm-family plasmid at all, much less to express both Pse1p and a heterologous protein from a 2µm-family plasmid in order to increase the expression levels of a heterologous protein.

Variants and fragments of Pse1p are also included in the present invention. A "variant", in the context of Pselp, refers to a protein having the sequence of native Pse1p other than for at one or more positions where there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" of Pse1p typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native Pse1p.

The percent sequence identity between two polypeptides may be determined using suitable computer programs, as discussed below. Such variants may, or may not, be natural or made using the methods of protein engineering and site-directed mutagenesis as are well known in the art.

A "fragment", in the context of Pse1p, refers to a protein having the sequence of native Pse1p other than for at one or more positions where there have been deletions. Thus the fragment may, or may not, comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature Pse1p protein. Particularly preferred fragments of Pse1p protein comprise one or more whole domains of the desired protein.

A fragment or variant of Pse1p may, or may not, be a protein that, when expressed recombinantly in a host cell, such as *S. cerevisiae*, can complement the deletion of the endogenous *PSE1* gene in the host cell and may, or may not, for example, be a naturally occurring homolog of Pse1p, such as a homolog encoded by another organism, such as another yeast or other fungi, or another eucaryote such as a human or other vertebrate, or animal or by a plant.

Another preferred chaperone is a protein comprising the sequence of a protein encoded by the *ORM2* gene, or a fragment or variant thereof having equivalent chaperone-like activity.

*ORM2*, also known as YLR350W, is located on chromosome XII (positions 828729 to 829379) of the *S. cerevisiae* genome and encodes an evolutionarily conserved protein with similarity to the yeast protein Orm1p. Hjelmqvist et al, 2002, Genome Biology, 3(6), research 0027.1-0027.16 reports that *ORM2* belongs to gene family comprising three human genes (*ORMDL1*, *ORMDL2* and *ORMDL3*) as well as homo logs in microsporidia, plants, *Drosophila*, urochordates and vertebrates. The *ORMDL* genes are reported to encode transmembrane proteins anchored in the proteins endoplasmic reticulum (ER).

The protein Orm2p is required for resistance to agents that induce the unfolded protein response. Hjelmqvist et al, 2002 (*supra*) reported that a double knockout of the two *S. cerevisiae ORMDL* homologs (*ORM1* and *ORM2*) leads to a decreased growth rate and greater sensitivity to tunicamycin and dithiothreitol.

One published sequence of Orm2p is as described in WO 2005/061718.

The above protein is encoded in *S. cerevisiae* by the coding nucleotide sequence also as described in WO 2005/061718, although it will be appreciated that the sequence can be modified by degenerate substitutions to obtain alternative nucleotide sequences which encode an identical protein product.

Variants and fragments of Orm2p are also included in the present invention. A "variant", in the context of Orm2p, refers to a protein having the sequence of native Orm2p other than for at one or more positions where there have been amino acid insertions, deletions, or substitutions, either conservative or non-conservative, provided that such changes result in a protein whose basic properties, for example enzymatic activity (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" of Orm2p typically has at least 25%, at least 50%, at least 60% or at least 70%, preferably at least 80%, more preferably at least 90%, even more preferably at least 95%, yet more preferably at least 99%, most preferably at least 99.5% sequence identity to the sequence of native Orm2p.

The percent sequence identity between two polypeptides may be determined using suitable computer programs, as discussed below. Such variants may, or may not, be natural or made using the methods of protein engineering and site-directed mutagenesis as are well known in the art.

A "fragment", in the context of Orm2p, refers to a protein having the sequence of native Orm2p other than for at one or more positions where there have been deletions. Thus the fragment may, or may not, comprise at most 5, 10, 20, 30, 40 or 50%, typically up to 60%, more typically up to 70%, preferably up to 80%, more preferably up to 90%, even more preferably up to 95%, yet more preferably up to 99% of the complete sequence of the full mature Orm2p protein. Particularly preferred fragments of Orm2p protein comprise one or more whole domains of the desired protein.

A fragment or variant of Orm2p may, or may not, be a protein that, when expressed recombinantly in a host cell, such as *S. cerevisiae*, can complement the deletion of the endogenous *ORM2* gene in the host cell and may, or may not, for example, be a naturally occurring homolog of Orm2p, such as a homolog encoded by another organism, such as another yeast or other fungi, or another eucaryote such as a human or other vertebrate, or animal or by a plant.

A gene encoding a protein comprising the sequence of a chaperone may, or may not, be formed in a like manner to that discussed below for genes encoding heterologous proteins, with particular emphasis on combinations of ORFs and regulatory regions.

Thus, one preferred chaperone is protein disulphide isomerase; another preferred chaperone is Orm2p or a fragment or variant thereof. In a particularly preferred embodiment, the first and second chaperones are protein disulphide isomerase and Orm2p or a fragment or variant thereof.

Further preferred combinations for the first and second chaperones, respectively, may, or may not, be encoded by the genes *AHA1* and *CCT2; AHA1* and *CCT3*; *AHA1* and *CCT4*; *AHA1* and *CCT5*; *AHA1* and *CCT6*; *AHA1* and *CCT7*; *AHA1* and *CCT8*; *AHA1* and *CNS1*; *AHA1* and *CPR3*; *AHA1* and *CPR6*; *AHA1* and *ERO1*; *AHA1* and *EUG1*; *AHA1* and *FMO1*; *AHA1* and *HCH1*; *AHA1* and *HSP10*; *AHA1* and *HSP12*; *AHA1* and *HSP104*; *AHA1* and *HSP26; AHA1* and *HSP30*; *AHA1* and *HSP42*; *AHA1* and *HSP60*; *AHA1* and *HSP78*; *AHA1* and *HSP82*; *AHA1* and *JEM1*; *AHA1* and *MDJ1*; *AHA1* and *MDJ2*; *AHA1* and *MPDP1*; *AHA1* and *MPD2*; *AHA1* and *PDI1*; *AHA1* and *PFD1*; *AHA1* and *ABC1*; *AHA1* and *APJ1*; *AHA1 and ATP11*; *AHA1* and *ATP12*; *AHA1* and *BTT1*; *AHA1* and *CDC37; AHA1* and *CPR7*; *AHA1* and *HSC82*; *AHA1* and *KAR2*; *AHA1* and *LHS1*; *AHA1* and *MGE1*; *AHA1* and *MRS11*; *AHA1* and *NOB1*; *AHA1* and *ECM10*; *AHA1* and *SSA1*; *AHA1* and *SSA2*; *AHA1* and *SSA3*; *AHA1* and *SSA4*; *AHA1* and *SSC1*; *AHA1* and *SSE2*; *AHA1* and *SIL1*; *AHA1* and *SLS1*; *AHA1* and *ORM1*; *AHA1* and *ORM2*; *AHA1* and *PER1*; *AHA1* and *PTC2*; *AHA1* and *PSE1*; *AHA1* and *UBI4*; *AHA1* and *HAC1* or a truncated intronless *HAC1*; *CCT2* and *CCT3*; *CCT2* and *CCT4*; *CCT2* and *CCT5*; *CCT2* and *CCT6*; *CCT2* and *CCT7*; *CCT2* and *CCT8*; *CCT2* and *CNS1*; *CCT2* and *CPR3*; *CCT2* and *CPR6*; *CCT2* and *ERO1*; *CCT2* and *EUG1*; *CCT2* and *FMO1*; *CCT2* and *HCH1*; *CCT2* and *HSP1O*; *CCT2* and *HSP12*; *CCT2* and *HSP104*; *CCT2* and *HSP26*; *CCT2* and *HSP30*; *CCT2* and *HSP42; CCT2* and *HSP60*; *CCT2* and *HSP78*; *CCT2* and *HSP82; CCT2* and *JEM1*; *CCT2* and *MDJ1*; *CCT2* and *MDJ2*; *CCT2* and *MPD1*; *CCT2* and *MPD2*; *CCT2* and *PDI1*; *CCT2 and PFD1*; *CCT2* and *ABC1*; *CCT2* and *APJ1*; *CCT2* and *ATP11*; *CCT2 and ATP12*; *CCT2* and *BTT1*; *CCT2* and *CDC37; CCT2* and *CPR7*; *CCT2* and *HSC82*; *CCT2* and *KAR2*; *CCT2* and *LHS1*; *CCT2* and *MGE1*; *CCT2* and *MRS11*; *CCT2* and *NOB1*; *CCT2* and *ECM10*; *CCT2* and *SSA1*; *CCT2* and *SSA2*; *CCT2* and *SSA3*; *CCT2* and *SSA4*; *CCT2* and *SSC1*; *CCT2* and *SSE2*; *CCT2* and *SIL1*; *CCT2* and *SLS1*; *CCT2* and *ORM1*; *CCT2* and *ORM2*; *CCT2* and *PER1*; *CCT2* and *PTC2*; *CCT2* and *PSE1*; *CCT2* and *UBI4*; *CCT2* and *HAC1* or a truncated intronless *HAC1*; *CCT3* and *CCT4*; *CCT3* and *CCT5*; *CCT3* and *CCT6; CCT3* and *CCT7*; *CCT3* and *CCT8*; *CCT3* and *CNS1*; *CCT3* and *CPR3*; *CCT3* and *CPR6*; *CCT3* and *ERO1*; *CCT3* and *EUG1*; *CCT3* and *FMO1*; *CCT3* and *HCH1*; *CCT3* and *HSP10*; *CCT3* and *HSP12*; *CCT3* and *HSP104*; *CCT3* and *HSP26*; *CCT3* and *HSP30*; *CCT3* and *HSP42; CCT3* and *HSP60*; *CCT3* and *HSP78; CCT3* and *HSP82*; *CCT3* and *JEM1*; *CCT3* and *MDJ1*; *CCT3* and *MDJ2*; *CCT3* and *MPD1*; *CCT3* and *MPD2*; *CCT3* and *PDI1*; *CCT3* and *PFD1*; *CCT3* and *ABC1*; *CCT3* and *APJ1*; *CCT3* and *ATP11*; *CCT3* and *ATP12*; *CCT3* and *BTT1*; *CCT3* and *CDC37*; *CCT3* and *CPR7*; *CCT3* and *HSC82*; *CCT3* and *KAR2*; *CCT3* and *LHS1*; *CCT3* and *MGE1*; *CCT3* and *MRS11*; *CCT3* and *NOB1*; *CCT3* and *ECM10*; *CCT3* and *SSA1*; *CCT3* and *SSA2*; *CCT3* and *SSA3*; *CCT3* and *SSA4*; *CCT3* and *SSC1*; *CCT3* and *SSE2*; *CCT3* and *SIL1*; *CCT3* and *SLS1*; *CCT3* and *ORM1*; *CCT3* and *ORM2*; *CCT3* and *PER1*; *CCT3* and *PTC2*; *CCT3* and *PSE1*; *CCT3* and *UBI4*; *CCT3* and *HAC1* or a truncated intronless *HAC1*; *CCT4* and *CCT5*; *CCT4* and *CCT6*; *CCT4* and *CCT7*; *CCT4* and *CCT8*; *CCT4* and *CNS1*; *CCT4* and *CPR3*; *CCT4* and *CPR6*; *CCT4* and *ENRO1*; *CCT4* and *EUG1*; *CCT4* and *FMO1*; *CCT4* and *HCH1*; *CCT4* and *HSP10*; *CCT4* and *HSP12*; *CCT4* and *HSP104*; *CCT4* and *HSP26; CCT4* and *HSP30*; *CCT4* and *HSP42; CCT4* and *HSP60*; *CCT4* and *HSP78*; *CCT4* and *HSP82; CCT4* and *JEM1*; *CCT4* and *MDJ1*; *CCT4* and *MDJ2*; *CCT4* and *MPD1*; *CCT4* and *MPD2*; *CCT4* and *PDI1*; *CCT4* and *PFD1*; *CCT4* and *ABC1*; *CCT4* and *APJ1*; *CCT4* and *ATP11*; *CCT4* and *ATP12*; *CCT4* and *BTT1*; *CCT4* and *CDC37; CCT4* and *CPR7; CCT4* and *HSC82; CCT4* and *KAR2*; *CCT4* and *LHS1*; *CCT4* and *MGE1*; *CCT4* and *MRS11*; *CCT4* and *NOB1*; *CCT4* and *ECM10*; *CCT4* and *SSA1*; *CCT4* and *SSA2*; *CCT4* and *SSA3*; *CCT4* and *SSA4*; *CCT4* and *SSC1*; *CCT4* and *SSE2*; *CCT4* and *SIL1*; *CCT4* and *SLS1*; *CCT4* and *ORM1*; *CCT4* and *ORM2*; *CCT4* and *PER1*; *CCT4* and *PTC2*; *CCT4* and *PSE1*; *CCT4* and *UBI4*; *CCT4* and *HAC1* or a truncated intronless *HAC1*; *CCT5* and *CCT6*; *CCT5* and *CCT7*; *CCT5* and *CCT8*; *CCT5* and *CNS1*; *CCT5* and *CPR3*; *CCT5* and *CPR6*; *CCT5* and *ERO1*; *CCT5* and *EUG1*; *CCT5* and *FMO1*; *CCT5* and *HCH1*; *CCT5* and *HSP10*; *CCT5* and *HSP12*; *CCT5* and *HSP104*; *CCT5* and *HSP26*; *CCT5* and *HSP30*; *CCT5* and *HSP42*; *CCT5* and *HSP60*; *CCT5* and *HSP78; CCT5* and *HSP82*; *CCT5* and *JEM1*; *CCT5* and *MDJ1*; *CCT5* and *MDJ2*; *CCT5* and *MPD1*; *CCT5* and *MPD2*; *CCT5* and *PDI1*; *CCT5* and *PFD1*; *CCT5* and *ABC1*; *CCT5* and *APJ1*; *CCT5* and *ATP11*; *CCT5* and *ATP12*; *CCT5* and *BTT1*; *CCT5* and *CDC37; CCT5* and *CPR7; CCT5* and *HSC82; CCT5* and *KAR2*; *CCT5* and *LHS1*; *CCT5* and *MGE1*; *CCT5* and *MRS11*; *CCT5* and *NOB1*; *CCT5* and *ECM10*; *CCT5* and *SSA1*; *CCT5* and *SSA2*; *CCT5* and *SSA3*; *CCT5* and *SSA4*; *CCT5* and *SSC1*; *CCT5* and *SSE2*; *CCT5* and *SIL1*; *CCT5* and *SLS1*; *CCT5* and *ORM1*; *CCT5* and *ORM2*; *CCT5* and *PER1*; *CCT5* and *PTC2; CCT5* and *PSE1*; *CCT5* and *UBI4*; *CCT5* and *HAC1* or a truncated intronless *HAC1*; *CCT6* and *CCT7*; *CCT6* and *CCT8*; *CCT6* and *CNS1*; *CCT6* and *CPR3*; *CCT6* and *CPR6*; *CCT6* and *ERO1*; *CCT6* and *EUG1*; *CCT6* and *FMO1*; *CCT6* and *HCH1*; *CCT6* and *HSP10*; *CCT6* and *HSP12*; *CCT6* and *HSP104*; *CCT6* and *HSP26; CCT6* and *HSP30*; *CCT6* and *HSP42; CCT6* and *HSP60*; *CCT6* and *HSP78*; *CCT6* and *HSP82; CCT6* and *JEM1*; *CCT6* and *MDJ1*; *CCT6* and *MDJ2*; *CCT6* and *MPD1*; *CCT6* and *MPD2*; *CCT6* and *PDI1*; *CCT6* and *PFD1*; *CCT6* and *ABC1*; *CCT6* and *APJ1*; *CCT6* and *ATP11*; *CCT6* and *ATP12*; *CCT6* and *BTT1*; *CCT6* and *CDC37; CCT6* and *CPR7*; *CCT6* and *HSC82; CCT6* and *KAR2*; *CCT6* and *LHS1*; *CCT6* and *MGE1*; *CCT6* and *MRS11*; *CCT6* and *NOB1*; *CCT6* and *ECM10*; *CCT6* and *SSA1*; *CCT6* and *SSA2*; *CCT6* and *SSA3*; *CCT6* and *SSA4*; *CCT6* and *SSC1*; *CCT6* and *SSE2*; *CCT6* and *SIL1*; *CCT6* and *SLS1*; *CCT6* and *ORM1*; *CCT6* and *ORM2*; *CCT6* and *PER1*; *CCT6* and *PTC2*; *CCT6* and *PSE1*; *CCT6* and *UB14*; *CCT6* and *HAC1* or a truncated intronless *HAC1*; *CCT7* and *CCT8*; *CCT7* and *CNS1*; *CCT7* and *CPR3*; *CCT7* and *CPR6*; *CCT7* and *ERO1*; *CCT7* and *EUG1*; *CCT7* and *FMO1*; *CCT7* and *HCH1*; *CCT7* and *HSP10*; *CCT7* and *HSP12*; *CCT7* and *HSP104*; *CCT7* and *HSP26*; *CCT7* and *HSP30*; *CCT7* and *HSP42; CCT7* and *HSP60*; *CCT7* and *HSP78*; *CCT7* and *HSP82*; *CCT7* and *JEM1*; *CCT7* and *MDJ1*; *CCT7* and *MDJ2*; *CCT7* and *MPD1*; *CCT7* and *MPD2*; *CCT7* and *PDI1*; *CCT7* and *PFD1*; *CCT7* and *ABC1*; *CCT7* and *APJ1*; *CCT7* and *ATP11*; *CCT7* and *ATP12*; *CCT7* and *BTT1*; *CCT7* and *CDC37; CCT7* and *CPR7*; *CCT7* and *HSC82*; *CCT7* and *KAR2*; *CCT7* and *LHS1*; *CCT7* and *MGE1*; *CCT7* and *MRS11*; *CCT7* and *NOB1*; *CCT7* and *ECM10*; *CCT7* and *SSA1*; *CCT7* and *SSA2*; *CCT7* and *SSA3*; *CCT7* and *SSA4*; *CCT7* and *SSC1*; *CCT7* and *SSE2*; *CCT7* and *SIL1*; *CCT7* and *SLS1*; *CCT7* and *ORM1*; *CCT7* and *ORM2*; *CCT7* and *PER1*; *CCT7* and *PTC2*; *CCT7* and *PSE1*; *CCT7* and *UB14*; *CCT7* and *HAC1* or a truncated intronless *HAC1*; *CCT8* and *CNS1*; *CCT8* and *CPR3*; *CCT8* and *CPR6*; *CCT8* and *ERO1*; *CCT8* and *EUG1*; *CCT8* and *FMO1*; *CCT8* and *HCH1*; *CCT8* and *HSP10*; *CCT8* and *HSP12*; *CCT8* and *HSP104*; *CCT8* and *HSP26*; *CCT8* and *HSP30*; *CCT8* and *HSP42; CCT8* and *HSP60*; *CCT8* and *HSP78*; *CCT8* and *HSP82*; *CCT8* and *JEM1*; *CCT8* and *MDJ1*; *CCT8* and *MDJ2*; *CCT8* and *MPD1*; *CCT8* and *MPD2*; *CCT8* and *PDI1*; *CCT8* and *PFD1*; *CCT8* and *ABC1*; *CCT8* and *APJ1*; *CCT8* and *ATP11*; *CCT8* and *ATP12*; *CCT8* and *BTT1*; *CCT8* and *CDC37; CCT8* and *CPR7*; *CCT8* and *HSC82*; *CCT8* and *KAR2*; *CCT8* and *LHS1*; *CCT8* and *MGE1*; *CCT8* and *MRS11*; *CCT8* and *NOB1*; *CCT8* and *ECM10*; *CCT8* and *SSA1*; *CCT8* and *SSA2*; *CCT8* and *SSA3*; *CCT8* and *SSA4*; *CCT8* and *SSC1*; *CCT8* and *SSE2*; *CCT8* and *SIL1*; *CCT8* and *SLS1*; *CCT8* and *ORM1*; *CCT8* and *ORM2*; *CCT8* and *PER1*; *CCT8* and *PTC2*; *CCT8* and *PSE1*; *CCT8* and *UBI4*; *CCT8* and *HAC1* or a truncated intronless *HAC1*; *CNS1* and *CPR3*; *CNS1* and *CPR6*; *CNS1* and *ERO1*; *CNS1* and *EUG1*; *CNS1* and *FMO1*; *CNS1* and *HCH1*; *CNS1* and *HSP10*; *CNS1* and *HSP12*; *CNS1* and *HSP104*; *CNS1* and *HSP26*; *CNS1* and *HSP30*; *CNS1* and *HSP42; CNS1* and *HSP60*; *CNS1* and *HSP78; CNS1* and *HSP82; CNS1* and *JEM1*; *CNS1* and *MDJ1*; *CNS1* and *MDJ2*; *CNS1* and *MPD1*; *CNS1* and *MPD2*; *CNS1* and *PDI1*; *CNS1* and *PFD1*; *CNS1* and *ABC1*; *CNS1* and *APJ1*; *CNS1* and *ATP11*; *CNS1* and *ATP12*; *CNS1* and *BTT1*; *CNS1* and *CDC37; CNS1* and *CPR7*; *CNS1* and *HSC82*; *CNS1* and *KAR2*; *CNS1* and *LHS1*; *CNS1* and *MGE1*; *CNS1* and *MRS11*; *CNS1* and *NOB1*; *CNS1* and *ECM10*; *CNS1* and *SSA1*; *CNS1* and *SSA2*; *CNS1* and *SSA3*; *CNS1* and *SSA4*; *CNS1* and *SSC1*; *CNS1* and *SSE2*; *CNS1* and *SIL1*; *CNS1* and *SLS1*; *CNS1* and *ORM1*; *CNS1* and *ORM2*; *CNS1* and *PER1*; *CNS1* and *PTC2*; *CNS1* and *PSE1*; *CNS1* and *UBI4*; *CNS1* and *HAC1* or a truncated intronless *HAC1*; *CPR3* and *CPR6*; *CPR3* and *ERO1*; *CPR3* and *EUG1*; *CPR3* and *FMO1*; *CPR3* and *HCH1*; *CPR3* and *HSP10*; *CPR3* and *HSP12*; *CPR3* and *HSP104*; *CPR3* and *HSP26; CPR3* and *HSP30*; *CPR3* and *HSP42*; *CPR3* and *HSP60*; *CPR3* and *HSP78; CPR3* and *HSP82*; *CPR3* and *JEM1*; *CPR3* and *MDJ1*; *CPR3* and *MDJ2*; *CPR3* and *MPD1*; *CPR3* and *MPD2*; *CPR3* and *PDI1*; *CPR3* and *PFD1*; *CPR3* and *ABC1*; *CPR3* and *APJ1*; *CPR3* and *ATP11*; *CPR3* and *ATP12*; *CPR3* and *BTT1*; *CPR3* and *CDC37; CPR3* and *CPR7; CPR3* and *HSC82*; *CPR3* and *KAR2*; *CPR3* and *LHS1*; *CPR3* and *MGE1*; *CPR3* and *MRS11*; *CPR3* and *NOB1*; *CPR3* and *ECM10*; *CPR3* and *SSA1*; *CPR3* and *SSA2*; *CPR3* and *SSA3*; *CPR3* and *SSA4*; *CPR3* and *SSC1*; *CPR3* and *SSE2*; *CPR3* and *SIL1*; *CPR3* and *SLS1*; *CPR3* and *ORM1*; *CPR3* and *ORM2*; *CPR3* and *PER1*; *CPR3* and *PTC2*; *CPR3* and *PSE1*; *CPR3* and *UB14*; *CPR3* and *HAC1* or a truncated intronless *HAC1*; *CPR6* and *ERO1*; *CPR6* and *EUG1*; *CPR6* and *FMO1*; *CPR6* and *HCH1*; *CPR6* and *HSP10*; *CPR6* and *HSP12*; *CPR6* and *HSP104*; *CPR6* and *HSP26*; *CPR6* and *HSP30*; *CPR6* and *HSP42*; *CPR6* and *HSP60*; *CPR6* and *HSP78; CPR6* and *HSP82*; *CPR6* and *JEM1*; *CPR6* and *MDJ1*; *CPR6* and *MDJ2*; *CPR6* and *MPD1*; *CPR6* and *MPD2*; *CPR6* and *PDI1*; *CPR6* and *PFD1*; *CPR6* and *ABC1*; *CPR6* and *APJ1*; *CPR6* and *ATP11*; *CPR6* and *ATP12*; *CPR6* and *BTT1*; *CPR6* and *CDC37; CPR6* and *CPR7*; *CPR6* and *HSC82*; *CPR6* and *KAR2*; *CPR6* and *LHS1*; *CPR6* and *MGE1*; *CPR6* and *MRS11*; *CPR6* and *NOB1*; *CPR6* and *ECM10*; *CPR6* and *SSA1*; *CPR6* and *SSA2*; *CPR6* and *SSA3*; *CPR6* and *SSA4*; *CPR6* and *SSC1*; *CPR6* and *SSE2*; *CPR6* and *SIL1*; *CPR6* and *SLS1*; *CPR6* and *ORM1*; *CPR6* and *ORM2*; *CPR6* and *PER1*; *CPR6* and *PTC2*; *CPR6* and *PSE1*; *CPR6* and *UBI4*; *CPR6* and *HAC1* or a truncated intronless *HAC1*; *ERO1* and *EUG1*; *ERO1* and *FMO1*; *ERO1* and *HCH1*; *ERO1* and *HSP10*; *ERO1* and *HSP12*; *ERO1* and *HSP104*; *ERO1* and *HSP26*; *ERO1* and *HSP30*; *ERO1* and *HSP42; ERO1* and *HSP60*; *ERO1* and *HSP78*; *ERO1* and *HSP82*; *ERO1* and *JEM1*; *ERO1* and *MDJ1*; *ERO1* and *MDJ2*; *ERO1* and *MPD1*; *ERO1* and *MPD2*; *ERO1* and *PDI1*; *ERO1* and *PFD1*; *ERO1* and *ABC1*; *ERO1* and *APJ1*; *ERO1* and *ATP11*; *ERO1* and *ATP12*; *ERO1* and *BTT1*; *ERO1* and *CDC37; ERO1* and *CPR7*; *ERO1* and *HSC82; ERO1* and *KAR2*; *ERO1* and *LHS1*; *ERO1* and *MGE1*; *ERO1* and *MRS11*; *ERO1* and *NOB1*; *ERO1* and *ECM10*; *ERO1* and *SSA1*; *ERO1* and *SSA2*; *ERO1* and *SSA3*; *ERO1* and *SSA4*; *ERO1* and *SSC1*; *ERO1* and *SSE2*; *ERO1* and *SIL1*; *ERO1* and *SLS1*; *ERO1* and *ORM1*; *ERO1* and *ORM2*; *ERO1* and *PER1*; *ERO1* and *PTC2*; *ERO1* and *PSE1*; *ERO1* and *UB14*; *ERO1* and *HAC1* or a truncated intronless *HAC1*; *EUG1* and *FMO1*; *EUG1* and *HCH1*; *EUG1* and *HSP10*; *EUG1* and *HSP12*; *EUG1* and *HSP104*; *EUG1* and *HSP26*; *EUG1* and *HSP30*; *EUG1* and *HSP42*; *EUG1* and *HSP60*; *EUG1* and *HSP78*; *EUG1* and *HSP82; EUG1* and *JEM1*; *EUG1* and *MDJ1*; *EUG1* and *MDJ2*; *EUG1* and *MPD1*; *EUG1* and *MPD2*; *EUG1* and *PDI1*; *EUG1* and *PFD1*; *EUG1* and *ABC1*; *EUG1* and *APJ1*; *EUG1* and *ATP11*; *EUG1* and *ATP12*; *EUG1* and *BTT1*; *EUG1* and *CDC37*; *EUG1* and *CPR7*; *EUG1* and *HSC82*; *EUG1* and *KAR2*; *EUG1* and *LHS1*; *EUG1* and *MGE1*; *EUG1* and *MRS11*; *EUG1* and *NOB1*; *EUG1* and *ECM10*; *EUG1* and *SSA1*; *EUG1* and *SSA2*; *EUG1* and *SSA3*; *EUG1* and *SSA4*; *EUG1* and *SSC1*; *EUG1* and *SSE2*; *EUG1* and *SIL1*; *EUG1* and *SLS1*; *EUG1* and *ORM1*; *EUG1* and *ORM2*; *EUG1* and *PER1*; *EUG1* and *PTC2*; *EUG1* and *PSE1*; *EUG1* and *UBI4*; *EUG1* and *HAC1* or a truncated intronless *HAC1*; *FMO1* and *HCH1*; *FMO1* and *HSP10*; *FMO1* and *HSP12*; *FMO1* and *HSP104*; *FMO1* and *HSP26*; *FMO1* and *HSP30*; *FMO1* and *HSP42*; *FMO1* and *HSP60*; *FMO1* and *HSP78*; *FMO1* and *HSP82*; *FMO1* and *JEM1*; *FMO1* and *MDJ1*; *FMO1* and *MDJ2*; *FMO1* and *MPD1*; *FMO1* and *MPD2*; *FMO1* and *PDI1*; *FMO1* and *PFD1*; *FMO1* and *ABC1*; *FMO1* and *APJ1*; *FMO1* and *ATP11*; *FMO1* and *ATP12*; *FMO1* and *BTT1*; *FMO1* and *CDC37*; *FMO1* and *CPR7*; *FMO1* and *HSC82*; *FMO1* and *KAR2*; *FMO1* and *LHS1*; *FMO1* and *MGE1*; *FMO1* and *MRS11*; *FMO1* and *NOB1*; *FMO1* and *ECM10*; *FMO1* and *SSA1*; *FMO1* and *SSA2*; *FMO1* and *SSA3*; *FMO1* and *SSA4*; *FMO1* and *SSC1*; *FMO1* and *SSE2*; *FMO1* and *SIL1*; *FMO1* and *SLS1*; *FMO1* and *ORM1*; *FMO1* and *ORM2*; *FMO1* and *PER1*; *FMO1* and *PTC2*; *FMO1* and *PSE1*; *FMO1* and *UB14*; *FMO1* and *HAC1* or a truncated intronless *HAC1*; *HCH1* and *HSP10*; *HCH1* and *HSP12*; *HCH1* and *HSP104*; *HCH1* and *HSP26*; *HCH1* and *HSP30*; *HCH1* and *HSP42; HCH1* and *HSP60*; *HCH1* and *HSP78; HCH1* and *HSP82*; *HCH1* and *JEM1*; *HCH1* and *MDJ1*; *HCH1* and *MDJ2*; *HCH1* and *MPD1*; *HCH1* and *MPD2*; *HCH1* and *PDI1*; *HCH1* and *PFD1*; *HCH1* and *ABC1*; *HCH1* and *APJ1*; *HCH1* and *ATP11*; *HCH1* and *ATP12*; *HCH1* and *BTT1*; *HCH1* and *CDC37*; *HCH1* and *CPR7*; *HCH1* and *HSC82*; *HCH1* and *KAR2*; *HCH1* and *LHS1*; *HCH1* and *MGE1*; *HCH1* and *MRS11*; *HCH1* and *NOB1*; *HCH1* and *ECM10*; *HCH1* and *SSA1*; *HCH1* and *SSA2*; *HCH1* and *SSA3*; *HCH1* and *SSA4*; *HCH1* and *SSC1*; *HCH1* and *SSE2*; *HCH1* and *SIL1*; *HCH1* and *SLS1*; *HCH1* and *ORM1*; *HCH1* and *ORM2*; *HCH1* and *PER1*; *HCH1* and *PTC2*; *HCH1* and *PSE1*; *HCH1* and *UBI4*; *HCH1* and *HAC1* or a truncated intronless *HAC1*; *HSP10* and *HSP12*; *HSP10* and *HSP104*; *HSP10* and *HSP26*; *HSP10* and *HSP30*; *HSP10* and *HSP42; HSP10* and *HSP60*; *HSP10* and *HSP78*; *HSP10* and *HSP82*; *HSP10* and *JEM1*; *HSP10* and *MDJ1*; *HSP10* and *MDJ2*; *HSP10* and *MPD1*; *HSP10* and *MPD2*; *HSP10* and *PDI1*; *HSP10* and *PFD1*; *HSP10* and *ABC1*; *HSP10* and *APJ1*; *HSP10* and *ATP11*; *HSP10* and *ATP12*; *HSP10* and *BTT1*; *HSP10* and *CDC37*; *HSP10* and *CPR7*; *HSP10* and *HSC82*; *HSP10* and *KAR2*; *HSP10* and *LHS1*; *HSP10* and *MGE1*; *HSP10* and *MRS11*; *HSP10* and *NOB1*; *HSP10* and *ECM10*; *HSP10* and *SSA1*; *HSP10* and *SSA2*; *HSP10* and *SSA3*; *HSP10* and *SSA4*; *HSP10* and *SSC1*; *HSP10* and *SSE2*; *HSP10* and *SIL1*; *HSP10* and *SLS1*; *HSP10* and *ORM1*; *HSP10* and *ORM2*; *HSP10* and *PER1*; *HSP10* and *PTC2*; *HSP10* and *PSE1*; *HSP10* and *UB14*; *HSP10* and *HAC1* or a truncated intronless *HAC1*; *HSP12* and *HSP104*; *HSP12* and *HSP26*; *HSP12* and *HSP30*; *HSP12* and *HSP42*; *HSP12* and *HSP60*; *HSP12* and *HSP78*; *HSP12* and *HSP82*; *HSP12* and *JEM1*; *HSP12* and *MDJ1*; *HSP12* and *MDJ2*; *HSP12* and *MPD1*; *HSP12* and *MPD2*; *HSP12* and *PDI1*; *HSP12* and *PFD1*; *HSP12* and *ABC1*; *HSP12* and *APJ1*; *HSP12* and *ATP11*; *HSP12* and *ATP12*; *HSP12* and *BTT1*; *HSP12* and *CDC37*; *HSP12* and *CPR7*; *HSP12* and *HSC82*; *HSP12* and *KAR2*; *HSP12* and *LHS1*; *HSP12* and *MGE1*; *HSP12* and *MRS11*; *HSP12* and *NOB1*; *HSP12* and *ECM10*; *HSP12* and *SSA1*; *HSP12* and *SSA2*; *HSP12* and *SSA3*; *HSP12* and *SSA4*; *HSP12* and *SSC1*; *HSP12* and *SSE2*; *HSP12* and *SIL1*; *HSP12* and *SLS1*; *HSP12* and *ORM1*; *HSP12* and *ORM2*; *HSP12* and *PER1*; *HSP12* and *PTC2*; *HSP12* and *PSE1*; *HSP12* and *UBI4*; *HSP12* and *HAC1* or a truncated intronless *HAC1*; *HSP104* and *HSP26*; *HSP104* and *HSP30*; *HSP104* and *HSP42*; *HSP104* and *HSP60*; *HSP104* and *HSP78*; *HSP104* and *HSP82*; *HSP104* and *JEM1*; *HSP104* and *MDJ1*; *HSP104* and *MDJ2*; *HSP104* and *MPD1*; *HSP104* and *MPD2*; *HSP104* and *PDI1*; *HSP104* and *PFD1*; *HSP104* and *ABC1*; *HSP104* and *APJ1*; *HSP104* and *ATP11*; *HSP104* and *ATP12*; *HSP104* and *BTT1*; *HSP104* and *CDC37; HSP104* and *CPR7*; *HSP104* and *HSC82*; *HSP104* and *KAR2*; *HSP104* and *LHS1*; *HSP104* and *MGE1*; *HSP104* and *MRS11*; *HSP104* and *NOB1*; *HSP104* and *ECM10*; *HSP104* and *SSA1*; *HSP104* and *SSA2*; *HSP104* and *SSA3*; *HSP104* and *SSA4*; *HSP104* and *SSC1*; *HSP104* and *SSE2*; *HSP104* and *SIL1*; *HSP104* and *SLS1*; *HSP104* and *ORM1*; *HSP104* and *ORM2*; *HSP104* and *PER1*; *HSP104* and *PTC2*; *HSP104* and *PSE1*; *HSP104* and *UBI4*; *HSP104* and *HAC1* or a truncated intronless *HAC1*; *HSP26* and *HSP30*; *HSP26* and *HSP42; HSP26* and *HSP60*; *HSP26* and *HSP78*; *HSP26* and *HSP82*; *HSP26* and *JEM1*; *HSP26* and *MDJ1*; *HSP26* and *MDJ2*; *HSP26* and *MPD1*; *HSP26* and *MPD2*; *HSP26* and *PDI1*; *HSP26* and *PFD1*; *HSP26* and *ABC1*; *HSP26* and *APJ1*; *HSP26* and *ATP11*; *HSP26* and *ATP12*; *HSP26* and *BTT1*; *HSP26* and *CDC37; HSP26* and *CPR7*; *HSP26* and *HSC82*; *HSP26* and *KAR2*; *HSP26* and *LHS1*; *HSP26* and *MGE1*; *HSP26* and *MRS11*; *HSP26* and *NOB1*; *HSP26* and *ECM10*; *HSP26* and *SSA1*; *HSP26* and *SSA2*; *HSP26* and *SSA3*; *HSP26* and *SSA4*; *HSP26* and *SSC1*; *HSP26* and *SSE2*; *HSP26* and *SIL1*; *HSP26* and *SLS1*; *HSP26* and *ORM1*; *HSP26* and *ORM2*; *HSP26* and *PER1*; *HSP26* and *PTC2*; *HSP26* and *PSE1*; *HSP26* and *UBI4*; *HSP26* and *HAC1* or a truncated intronless *HAC1*; *HSP30* and *HSP42*; *HSP30* and *HSP60*; *HSP30* and *HSP78*; *HSP30* and *HSP82*; *HSP30* and *JEM1*; *HSP30* and *MDJ1*; *HSP30* and *MDJ2*; *HSP30* and *MPD1*; *HSP30* and *MPD2*; *HSP30* and *PDI1*; *HSP30* and *PFD1*; *HSP30* and *ABC1*; *HSP30* and *APJ1*; *HSP30* and *ATP11*; *HSP30* and *ATP12*; *HSP30* and *BTT1*; *HSP30* and *CDC37*; *HSP30* and *CPR7*; *HSP30* and *HSC82*; *HSP30* and *KAR2*; *HSP30* and *LHS1*; *HSP30* and *MGE1*; *HSP30* and *MRS11*; *HSP30* and *NOB1*; *HSP30* and *ECM10*; *HSP30* and *SSA1*; *HSP30* and *SSA2*; *HSP30* and *SSA3*; *HSP30* and *SSA4*; *HSP30* and *SSC1*; *HSP30* and *SSE2*; *HSP30* and *SIL1*; *HSP30* and *SLS1*; *HSP30* and *ORM1*; *HSP30* and *ORM2*; *HSP30* and *PER1*; *HSP30* and *PTC2*; *HSP30* and *PSE1*; *HSP30* and *UBI4*; *HSP30* and *HAC1* or a truncated intronless *HAC1*; *HSP42* and *HSP60*; *HSP42* and *HSP78*; *HSP42* and *HSP82*; *HSP42* and *JEM1*; *HSP42* and *MDJ1*; *HSP42* and *MDJ2*; *HSP42* and *MPD7*; *HSP42* and *MPD2*; *HSP42* and *PDI1*; *HSP42* and *PFD1*; *HSP42* and *ABC1*; *HSP42* and *APJ1*; *HSP42* and *ATP11*; *HSP42* and *ATP12*; *HSP42* and *BTT1*; *HSP42* and *CDC37; HSP42* and *CPR7*; *HSP42* and *HSC82*; *HSP42* and *KAR2*; *HSP42* and *LHS1*; *HSP42* and *MGE1*; *HSP42* and *MRS11*; *HSP42* and *NOB1*; *HSP42* and *ECM10*; *HSP42* and *SSA1*; *HSP42* and *SSA2*; *HSP42* and *SSA3*; *HSP42* and *SSA4*; *HSP42* and *SSC1*; *HSP42* and *SSE2*; *HSP42* and *SIL1*; *HSP42* and *SLS1*; *HSP42* and *ORM1*; *HSP42* and *ORM2*; *HSP42* and *PER1*; *HSP42* and *PTC2*; *HSP42* and *PSE1*; *HSP42* and *UBI4*; *HSP42* and *HAC1* or a truncated intronless *HAC1*; *HSP60* and *HSP78*; *HSP60* and *HSP82; HSP60* and *JEM1*; *HSP60* and *MDJ1*; *HSP60* and *MDJ2*; *HSP60* and *MPD7*; *HSP60* and *MPD2*; *HSP60* and *PDI1*; *HSP60* and *PFD1*; *HSP60* and *ABC1*; *HSP60* and *APJ1*; *HSP60* and *ATP11*; *HSP60* and *ATP12*; *HSP60* and *BTT1*; *HSP60* and *CDC37; HSP60* and *CPR7; HSP60* and *HSC82; HSP60* and *KAR2*; *HSP60* and *LHS1*; *HSP60* and *MGE1*; *HSP60* and *MRS11*; *HSP60* and *NOB1*; *HSP60* and *ECM10*; *HSP60* and *SSA1*; *HSP60* and *SSA2*; *HSP60* and *SSA3*; *HSP60* and *SSA4*; *HSP60* and *SSC1*; *HSP60* and *SSE2*; *HSP60* and *SIL1*; *HSP60* and *SLS1*; *HSP60* and *ORM1*; *HSP60* and *ORM2*; *HSP60* and *PER1*; *HSP60* and *PTC2*; *HSP60* and *PSE1*; *HSP60* and *UBI4*; *HSP60* and *HAC1* or a truncated intronless *HAC1*; *HSP78* and *HSP82*; *HSP78* and *JEM1*; *HSP78* and *MDJ1*; *HSP78* and *MDJ2*; *HSP78* and *MPD7*; *HSP78* and *MPD2*; *HSP78* and *PDI1*; *HSP78* and *PFD1*; *HSP78* and *ABC1*; *HSP78* and *APJ1*; *HSP78* and *ATP11*; *HSP78* and *ATP12*; *HSP78* and *BTT1*; *HSP78* and *CDC37; HSP78* and *CPR7*; *HSP78* and *HSC82*; *HSP78* and *KAR2*; *HSP78* and *LHS1*; *HSP78* and *MGE1*; *HSP78* and *MRS11*; *HSP78* and *NOB1*; *HSP78* and *ECM10*; *HSP78* and *SSA1*; *HSP78* and *SSA2*; *HSP78* and *SSA3*; *HSP78* and *SSA4*; *HSP78* and *SSC1*; *HSP78* and *SSE2*; *HSP78* and *SIL1*; *HSP78* and *SLS1*; *HSP78* and *ORM1*; *HSP78* and *ORM2*; *HSP78* and *PER1*; *HSP78* and *PTC2*; *HSP78* and *PSE1*; *HSP78* and *UBI4*; *HSP78* and *HAC1* or a truncated intronless *HAC1*; *HSP82* and *JEM1*; *HSP82* and *MDJ1*; *HSP82* and *MDJ2*; *HSP82* and *MPD7*; *HSP82* and *MPD2*; *HSP82* and *PDI1*; *HSP82* and *PFD1*; *HSP82* and *ABC1*; *HSP82* and *APJ1*; *HSP82* and *ATP11*; *HSP82* and *ATP12*; *HSP82* and *BTT1*; *HSP82* and *CDC37; HSP82* and *CPR7*; *HSP82* and *HSC82*; *HSP82* and *KAR2*; *HSP82* and *LHS1*; *HSP82* and *MGE1*; *HSP82* and *MRS11*; *HSP82* and *NOB1*; *HSP82* and *ECM10*; *HSP82* and *SSA1*; *HSP82* and *SSA2*; *HSP82* and *SSA3*; *HSP82* and *SSA4*; *HSP82* and *SSC1*; *HSP82* and *SSE2*; *HSP82* and *SIL1*; *HSP82* and *SLS1*; *HSP82* and *ORM1*; *HSP82* and *ORM2*; *HSP82* and *PER1*; *HSP82* and *PTC2*; *HSP82* and *PSE1*; *HSP82* and *UBI4*; *HSP82* and *HAC1* or a truncated intronless *HAC1*; *JEM1* and *MDJ1*; *JEM1* and *MDJ2*; *JEM1* and *MPD7*; *JEM1* and *MPD2*; *JEM1* and *PDI1*; *JEM1* and *PFD1*; *JEM1* and *ABC1*; *JEM1* and *APJ1*; *JEM1* and *ATP11*; *JEM1* and *ATP12*; *JEM1* and *BTT1*; *JEM1* and *CDC37; JEM1* and *CPR7*; *JEM1* and *HSC82; JEM1* and *KAR2*; *JEM1* and *LHS1*; *JEM1* and *MGE1*; *JEM1* and *MRS11*; *JEM1* and *NOB1*; *JEM1* and *ECM10*; *JEM1* and *SSA1*; *JEM1* and *SSA2*; *JEM1* and *SSA3*; *JEM1* and *SSA4*; *JEM1* and *SSC1*; *JEM1* and *SSE2*; *JEM1* and *SIL1*; *JEM1* and *SLS1*; *JEM1* and *ORM1*; *JEM1* and *ORM2*; *JEM1* and *PER1*; *JEM1* and *PTC2*; *JEM1* and *PSE1*; *JEM1* and *UBI4*; *JEM1* and *HAC1* or a truncated intronless *HAC1*; *MDJ1* and *MDJ2*; *MDJ1* and *MPD7*; *MDJ1* and *MPD2*; *MDJ1* and *PDI1*; *MDJ1* and *PFD1*; *MDJ1* and *ABC1*; *MDJ1* and *APJ1*; *MDJ1* and *ATP11*; *MDJ1* and *ATP12*; *MDJ1* and *BTT1*; *MDJ1* and *CDC37; MDJ1* and *CPR7*; *MDJ1* and *HSC82*; *MDJ1* and *KAR2*; *MDJ1* and *LHS1*; *MDJ1* and *MGE1*; *MDJ1* and *MRS11*; *MDJ1* and *NOB1*; *MDJ1* and *ECM10*; *MDJ1* and *SSA1*; *MDJ1* and *SSA2*; *MDJ1* and *SSA3*; *MDJ1* and *SSA4*; *MDJ1* and *SSC1*; *MDJ1* and *SSE2*; *MDJ1* and *SIL1*; *MDJ1* and *SLS1*; *MDJ1* and *ORM1*; *MDJ1* and *ORM2*; *MDJ1* and *PER1*; *MDJ1* and *PTC2*; *MDJ1* and *PSE1*; *MDJ1* and *UBI4*; *MDJ1* and *HAC1* or a truncated intronless *HAC1*; *MDJ2* and *MPD7*; *MDJ2* and *MPD2*; *MDJ2* and *PDI1*; *MDJ2* and *PFD1*; *MDJ2* and *ABC1*; *MDJ2* and *APJ1*; *MDJ2* and *ATP11*; *MDJ2* and *ATP12*; *MDJ2* and *BTT1*; *MDJ2* and *CDC37; MDJ2* and *CPR7*; *MDJ2* and *HSC82*; *MDJ2* and *KAR2*; *MDJ2* and *LHS1*; *MDJ2* and *MGE1*; *MDJ2* and *MRS11*; *MDJ2* and *NOB1*; *MDJ2* and *ECM10*; *MDJ2* and *SSA1*; *MDJ2* and *SSA2*; *MDJ2* and *SSA3*; *MDJ2* and *SSA4*; *MDJ2* and *SSC1*; *MDJ2* and *SSE2*; *MDJ2* and *SIL1*; *MDJ2* and *SLS1*; *MDJ2* and *ORM1*; *MDJ2* and *ORM2*; *MDJ2* and *PER1*; *MDJ2* and *PTC2*; *MDJ2* and *PSE1*; *MDJ2* and *UBI4*; *MDJ2* and *HAC1* or a truncated intronless *HAC1*; *MPD1* and *MPD2*; *MPD1* and *PDI1*; *MPD1* and *PFD1*; *MPD1* and *ABC1*; *MPD1* and *APJ1*; *MPD1* and *ATP11*; *MPD1* and *ATP12*; *MPD1* and *BTT1*; *MPD1* and *CDC37; MPD1* and *CPR7*; *MPD1* and *HSC82*; *MPD1* and *KAR2*; *MPD1* and *LHS1*; *MPD1* and *MGE1*; *MPD1* and *MRS11*; *MPD1* and *NOB1*; *MPD1* and *ECM10*; *MPD1* and *SSA1*; *MPD1* and *SSA2*; *MPD1* and *SSA3*; *MPD1* and *SSA4*; *MPD1* and *SSC1*; *MPD1* and *SSE2*; *MPD1* and *SIL1*; *MPD1* and *SLS1*; *MPD1* and *ORM1*; *MPD1* and *ORM2*; *MPD1* and *PER1*; *MPD1* and *PTC2*; *MPD1* and *PSE1*; *MPD1* and *UBI4*; *MPD1* and *HAC1* or a truncated intronless *HAC1*; *MPD2* and *PDI1*; *MPD2* and *PFD1*; *MPD2* and *ABC1; MPD2* and *APJ1*; *MPD2* and *ATP11*; *MPD2* and *ATP12*; *MPD2* and *BTT1*; *MPD2* and *CDC37; MPD2* and *CPR7; MPD2* and *HSC82; MPD2* and *KAR2*; *MPD2* and *LHS1*; *MPD2* and *MGE1*; *MPD2* and *MRS11*; *MPD2* and *NOB1*; *MPD2* and *ECM10*; *MPD2* and *SSA1*; *MPD2* and *SSA2*; *MPD2* and *SSA3*; *MPD2* and *SSA4*; *MPD2* and *SSC1*; *MPD2* and *SSE2*; *MPD2* and *SIL1*; *MPD2* and *SLS1*; *MPD2* and *ORM1*; *MPD2* and *ORM2*; *MPD2* and *PER1*; *MPD2* and *PTC2*; *MPD2* and *PSE1*; *MPD2* and *UBI4*; *MPD2* and *HAC1* or a truncated intronless *HAC1*; *PDI1* and *PFD1*; *PDI1* and *ABC1*; *PDI1* and *APJ1*; *PDI1* and *ATP11*; *PDI1* and *ATP12*; *PDI1* and *BTT1*; *PDI1* and *CDC37; PDI1* and *CPR7*; *PDI1* and *HSC82*; *PDI1* and *KAR2*; *PDI1* and *LHS1*; *PDI1* and *MGE1*; *PDI1* and *MRS11*; *PDI1* and *NOB1*; *PDI1* and *ECM10*; *PDI1* and *SSA1*; *PDI1* and *SSA2*; *PDI1* and *SSA3*; *PDI1* and *SSA4*; *PDI1* and *SSC1*; *PDI1* and *SSE2*; *PDI1* and *SIL1*; *PDI1* and *SLS1*; *PDI1* and *ORM1*; *PDI1* and *ORM2*; *PDI1* and *PER1*; *PDI1* and *PTC2*; *PDI1* and *PSE1*; *PDI1* and *UBI4*; *PDI1* and *HAC1* or a truncated intronless *HAC1*; *PFD1* and *ABC1*; *PFD1* and *APJ1*; *PFD1* and *ATP11*; *PFD1* and *ATP12*; *PFD1* and *BTT1*; *PFD1* and *CDC37; PFD1* and *CPR7*; *PFD1* and *HSC82; PFD1* and *KAR2*; *PFD1* and *LHS1*; *PFD1* and *MGE1*; *PFD1* and *MRS11*; *PFD1* and *NOB1*; *PFD1* and *ECM10*; *PFD1* and *SSA1*; *PFD1* and *SSA2*; *PFD1* and *SSA3*; *PFD1* and *SSA4*; *PFD1* and *SSC1*; *PFD1* and *SSE2*; *PFD1* and *SIL1*; *PFD1* and *SLS1*; *PFD1* and *ORM1*; *PFD1* and *ORM2*; *PFD1* and *PER1*; *PFD1* and *PTC2*; *PFD1* and *PSE1*; *PFD1* and *UBI4*; *PFD1* and *HAC1* or a truncated intronless *HAC1*; *ABC1* and *APJ1*; *ABC1* and *ATP11*; *ABC1* and *ATP12*; *ABC1* and *BTT1*; *ABC1* and *CDC37*; *ABC1* and *CPR7*; *ABC1* and *HSC82*; *ABC1* and *KAR2*; *ABC1* and *LHS1*; *ABC1* and *MGE1*; *ABC1* and *MRS11*; *ABC1* and *NOB1*; *ABC1* and *ECM10*; *ABC1* and *SSA1*; *ABC1* and *SSA2*; *ABC1* and *SSA3*; *ABC1* and *SSA4*; *ABC1* and *SSC1*; *ABC1* and *SSE2*; *ABC1* and *SIL1*; *ABC1* and *SLS1*; *ABC1* and *ORM1*; *ABC1* and *ORM2*; *ABC1* and *PER1*; *ABC1* and *PTC2*; *ABC1* and *PSE1*; *ABC1* and *UBI4*; *ABC1* and *HAC1* or a truncated intronless *HAC1*; *APJ1* and *ATP11*; *APJ1* and *ATP12*; *APJ1* and *BTT1*; *APJ1* and *CDC37; APJ1* and *CPR7*; *APJ1* and *HSC82; APJ1* and *KAR2*; *APJ1* and *LHS1*; *APJ1* and *MGE1*; *APJ1* and *MRS11*; *APJ1* and *NOB1*; *APJ1* and *ECM10*; *APJ1* and *SSA1*; *APJ1* and *SSA2*; *APJ1* and *SSA3*; *APJ1* and *SSA4*; *APJ1* and *SSC1*; *APJ1* and *SSE2; APJ1* and *SIL1*; *APJ1* and *SLS1*; *APJ1* and *ORM1*; *APJ1* and *ORM2*; *APJ1* and *PER1*; *APJ1* and *PTC2; APJ1* and *PSE1*; *APJ1* and *UBI4*; *APJ1* and *HAC1* or a truncated intronless *HAC1*; *ATP11* and *ATP12*; *ATP11* and *BTT1*; *ATP11* and *CDC37; ATP11* and *CPR7; ATP11* and *HSC82; ATP11* and *KAR2*; *ATP11* and *LHS1*; *ATP11* and *MGE1*; *ATP11* and *MRS11*; *ATP11* and *NOB1*; *ATP11* and *ECM10*; *ATP11* and *SSA1*; *ATP11* and *SSA2*; *ATP11* and *SSA3*; *ATP11* and *SSA4*; *ATP11* and *SSC1*; *ATP11* and *SSE2; ATP11* and *SIL1*; *ATP11* and *SLS1*; *ATP11* and *ORM1*; *ATP11* and *ORM2*; *ATP11* and *PER1*; *ATP11* and *PTC2*; *ATP11* and *PSE1*; *ATP11* and *UBI4*; *ATP11* and *HAC1* or a truncated intronless *HAC1*; *ATP12* and *BTT1*; *ATP12* and *CDC37*; *ATP12* and *CPR7*; *ATP12* and *HSC82*; *ATP12* and *KAR2*; *ATP12* and *LHS1*; *ATP12* and *MGE1*; *ATP12* and *MRS11*; *ATP12* and *NOB1*; *ATP12* and *ECM10*; *ATP12* and *SSA1*; *ATP12* and *SSA2*; *ATP12* and *SSA3*; *ATP12* and *SSA4*; *ATP12* and *SSC1*; *ATP12* and *SSE2*; *ATP12* and *SIL1*; *ATP12* and *SLS1*; *ATP12* and *ORM1*; *ATP12* and *ORM2*; *ATP12* and *PER1*; *ATP12* and *PTC2*; *ATP12* and *PSE1*; *ATP12* and *UBI4*; *ATP12* and *HAC1* or a truncated intronless *HAC1*; *BTT1* and *CDC37*; *BTT1* and *CPR7*; *BTT1* and *HSC82*; *BTT1* and *KAR2*; *BTT1* and *LHS1*; *BTT1* and *MGE1*; *BTT1* and *MRS11*; *BTT1* and *NOB1*; *BTT1* and *ECM10*; *BTT1* and *SSA1*; *BTT1* and *SSA2*; *BTT1* and *SSA3*; *BTT1* and *SSA4*; *BTT1* and *SSC1*; *BTT1* and *SSE2*; *BTT1* and *SIL1*; *BTT1* and *SLS1*; *BTT1* and *ORM1*; *BTT1* and *ORM2*; *BTT1* and *PER1*; *BTT1* and *PTC2*; *BTT1* and *PSE1*; *BTT1* and *UBI4*; *BTT1* and *HAC1* or a truncated intronless *HAC1*; *CDC37* and *CPR7*; *CDC37* and *HSC82*; *CDC37* and *KAR2*; *CDC37* and *LHS1*; *CDC37* and *MGE1*; *CDC37* and *MRS11*; *CDC37* and *NOB1*; *CDC37* and *ECM10*; *CDC37* and *SSA1*; *CDC37* and *SSA2*; *CDC37* and *SSA3*; *CDC37* and *SSA4*; *CDC37* and *SSC1*; *CDC37* and *SSE2*; *CDC37* and *SIL1*; *CDC37* and *SLS1*; *CDC37* and *ORM1*; *CDC37* and *ORM2*; *CDC37* and *PER1*; *CDC37* and *PTC2*; *CDC37* and *PSE1*; *CDC37* and *UBI4*; *CDC37* and *HAC1* or a truncated intronless *HAC1*; *CPR7* and *HSC82*; *CPR7* and *KAR2*; *CPR7* and *LHS1*; *CPR7* and *MGE1*; *CPR7* and *MRS11*; *CPR7* and *NOB1*; *CPR7* and *ECM10*; *CPR7* and *SSA1*; *CPR7* and *SSA2*; *CPR7* and *SSA3*; *CPR7* and *SSA4*; *CPR7* and *SSC1*; *CPR7* and *SSE2*; *CPR7* and *SIL1*; *CPR7* and *SLS1*; *CPR7* and *ORM1*; *CPR7* and *ORM2*; *CPR7* and *PER1*; *CPR7* and *PTC2*; *CPR7* and *PSE1*; *CPR7* and *UBI4*; *CPR7* and *HAC1* or a truncated intronless *HAC1*; *HSC82* and *KAR2*; *HSC82* and *LHS1*; *HSC82* and *MGE1*; *HSC82* and *MRS11*; *HSC82* and *NOB1*; *HSC82* and *ECM10*; *HSC82* and *SSA1*; *HSC82* and *SSA2*; *HSC82* and *SSA3*; *HSC82* and *SSA4*; *HSC82* and *SSC1*; *HSC82* and *SSE2*; *HSC82* and *SIL1*; *HSC82* and *SLS1*; *HSC82* and *ORM1*; *HSC82* and *ORM2*; *HSC82* and *PER1*; *HSC82* and *PTC2*; *HSC82* and *PSE1*; *HSC82* and *UBI4*; *HSC82* and *HAC1* or a truncated intronless *HAC1*; *KAR2* and *LHS1*; *KAR2* and *MGE1*; *KAR2* and *MRS11*; *KAR2* and *NOB1*; *KAR2* and *ECM10*; *KAR2* and *SSA1*; *KAR2* and *SSA2*; *KAR2* and *SSA3*; *KAR2* and *SSA4*; *KAR2* and *SSC1*; *KAR2* and *SSE2*; *KAR2* and *SIL1*; *KAR2* and *SLS1*; *KAR2* and *ORM1*; *KAR2* and *ORM2*; *KAR2* and *PER1*; *KAR2* and *PTC2*; *KAR2* and *PSE1*; *KAR2* and *UBI4*; *KAR2* and *HAC1* or a truncated intronless *HAC1*; *LHS1* and *MGE1*; *LHS1* and *MRS11*; *LHS1* and *NOB1*; *LHS1* and *ECM10*; *LHS1* and *SSA1*; *LHS1* and *SSA2*; *LHS1* and *SSA3*; *LHS1* and *SSA4*; *LHS1* and *SSC1*; *LHS1* and *SSE2*; *LHS1* and *SIL1*; *LHS1* and *SLS1*; *LHS1* and *ORM1*; *LHS1* and *ORM2*; *LHS1* and *PER1*; *LHS1* and *PTC2*; *LHS1* and *PSE1*; *LHS1* and *UBI4*; *LHS1* and *HAC1* or a truncated intronless *HAC1*; *MGE1* and *MRS11*; *MGE1* and *NOB1*; *MGE1* and *ECM10*; *MGE1* and *SSA1*; *MGE1* and *SSA2*; *MGE1* and *SSA3*; *MGE1* and *SSA4*; *MGE1* and *SSC1*; *MGE1* and *SSE2*; *MGE1* and *SIL1*; *MGE1* and *SLS1*; *MGE1* and *ORM1*; *MGE1* and *ORM2*; *MGE1* and *PER1*; *MGE1* and *PTC2*; *MGE1* and *PSE1*; *MGE1* and *UBI4*; *MGE1* and *HAC1* or a truncated intronless *HAC1*; *MRS11* and *NOB1*; *MRS11* and *ECM10*; *MRS11* and *SSA1*; *MRS11* and *SSA2*; *MRS11* and *SSA3*; *MRS11* and *SSA4*; *MRS11* and *SSC1*; *MRS11* and *SSE2*; *MRS11* and *SIL1*; *MRS11* and *SLS1*; *MRS11* and *ORM1*; *MRS11* and *ORM2*; *MRS11* and *PER1*; *MRS11* and *PTC2*; *MRS11* and *PSE1*; *MRS11* and *UBI4*; *MRS11* and *HAC1* or a truncated intronless *HAC1*; *NOB1* and *ECM10*; *NOB1* and *SSA1*; *NOB1* and *SSA2*; *NOB1* and *SSA3*; *NOB1* and *SSA4*; *NOB1* and *SSC1*; *NOB1* and *SSE2*; *NOB1* and *SIL1*; *NOB1* and *SLS1*; *NOB1* and *ORM1*; *NOB1* and *ORM2*; *NOB1* and *PER1*; *NOB1* and *PTC2*; *NOB1* and *PSE1*; *NOB1* and *UBI4*; *NOB1* and *HAC1* or a truncated intronless *HAC1*; *ECM10* and *SSA1*; *ECM10* and *SSA2*; *ECM10* and *SSA3*; *ECM10* and *SSA4*; *ECM10* and *SSC1*; *ECM10* and *SSE2*; *ECM10* and *SIL1*; *ECM10* and *SLS1*; *ECM10* and *ORM1*; *ECM10* and *ORM2*; *ECM10* and *PER1*; *ECM10* and *PTC2*; *ECM10* and *PSE1*; *ECM10* and *UBI4*; *ECM10* and *HAC1* or a truncated intronless *HAC1*; *SSA1* and *SSA2*; *SSA1* and *SSA3*; *SSA1* and *SSA4*; *SSA1* and *SSC1*; *SSA1* and *SSE2*; *SSA1* and *SIL1*; *SSA1* and *SLS1*; *SSA1* and *ORM1*; *SSA1* and *ORM2*; *SSA1* and *PER1*; *SSA1* and *PTC2; SSA1* and *PSE1*; *SSA1* and *UBI4*; *SSA1* and *HAC1* or a truncated intronless *HAC1*; *SSA2* and *SSA3*; *SSA2* and *SSA4*; *SSA2* and *SSC1*; *SSA2* and *SSE2*; *SSA2* and *SIL1*; *SSA2* and *SLS1*; *SSA2* and *ORM1*; *SSA2* and *ORM2*; *SSA2* and *PER1*; *SSA2* and *PTC2*; *SSA2* and *PSE1*; *SSA2* and *UBI4*; *SSA2* and *HAC1* or a truncated intronless *HAC1*; *SSA3* and *SSA4*; *SSA3* and *SSC1*; *SSA3* and *SSE2*; *SSA3* and *SIL1*; *SSA3* and *SLS1*; *SSA3* and *ORM1*; *SSA3* and *ORM2*; *SSA3* and *PER1*; *SSA3* and *PTC2; SSA3* and *PSE1*; *SSA3* and *UBI4*; *SSA3* and *HAC1* or a truncated intronless *HAC1*; *SSA4* and *SSC1*; *SSA4* and *SSE2*; *SSA4* and *SIL1*; *SSA4* and *SLS1*; *SSA4* and *ORM1*; *SSA4* and *ORM2*; *SSA4* and *PER1*; *SSA4* and *PTC2*; *SSA4* and *PSE1*; *SSA4* and *UBI4*; *SSA4* and *HAC1* or a truncated intronless *HAC1*; *SSC1* and *SSE2*; *SSC1* and *SIL1*; *SSC1* and *SLS1*; *SSC1* and *ORM1*; *SSC1* and *ORM2*; *SSC1* and *PER1*; *SSC1* and *PTC2*; *SSC1* and *PSE1*; *SSC1* and *UBI4*; *SSC1* and *HAC1* or a truncated intronless *HAC1*; *SSE2* and *SIL1*; *SSE2* and *SLS1*; *SSE2* and *ORM1*; *SSE2* and *ORM2*; *SSE2* and *PER1*; *SSE2* and *PTC2*; *SSE2* and *PSE1*; *SSE2* and *UBI4*; *SSE2* and *HAC1* or a truncated intronless *HAC1*; *SIL1* and *SLS1*; *SIL1* and *ORM1*; *SIL1* and *ORM2*; *SIL1* and *PER1*; *SIL1* and *PTC2*; *SIL1* and *PSE1*; *SIL1* and *UBI4*; *SIL1* and *HAC1* or a truncated intronless *HAC1*; *SLS1* and *ORM1*; *SLS1* and *ORM2*; *SLS1* and *PER1*; *SLS1* and *PTC2*; *SLS1* and *PSE1*; *SLS1* and *UBI4*; *SLS1* and *HAC1* or a truncated intronless *HAC1*; *ORM1* and *ORM2*; *ORM1* and *PER1*; *ORM1* and *PTC2*; *ORM1* and *PSE1*; *ORM1* and *UBI4*; *ORM1* and *HAC1* or a truncated intronless *HAC1*; *ORM2* and *PER1*; *ORM2* and *PTC2*; *ORM2* and *PSE1*; *ORM2* and *UBI4*; *ORM2* and *HAC1* or a truncated intronless *HAC1*; *PERI* and *PTC2*; *PERI* and *PSE1*; *PERI* and *UBI4*; *PERI* and *HAC1* or a truncated intronless *HAC1*; *PTC2* and *PSE1*; *PTC2* and *UBI4*; *PTC2* and *HAC1* or a truncated intronless *HAC1*; *PSE1* and *UBI4*; *PSE1* and *HAC1* or a truncated intronless *HAC1*; *UBI4* and *HAC1* or a truncated intronless *HAC1*; *TIM9* and *AHA1; TIM9* and *CCT2*; *TIM9* and *CCT3*; *TIM9* and *CCT4*; *TIM9* and *CCT5*; *TIM9* and *CCT6*; *TIM9* and *CCT7*; *TIM9* and *CCT8*; *TIM9* and *CNS1*; *TIM9* and *CPR3*; *TIM9* and *CPR6*; *TIM9* and *ERO1*; *TIM9* and *EUG1*; *TIM9* and *FMO1*; *TIM9* and *HCH1*; *TIM9* and *HSP10*; *TIM9* and *HSP12*; *TIM9* and *HSP104*; *TIM9* and *HSP26*; *TIM9* and *HSP30*; *TIM9* and *HSP42*; *TIM9* and *HSP60*; *TIM9* and *HSP78; TIM9* and *HSP82*; *TIM9* and *JEM1*; *TIM9* and *MDJ1*; *TIM9* and *MDJ2*; *TIM9* and *MPD1*; *TIM9* and *MPD2*; *TIM9* and *PDI1*; *TIM9* and *PFD1*; *TIM9* and *ABC1*; *TIM9* and *APJ1*; *TIM9* and *ATP11*; *TIM9* and *ATP12*; *TIM9* and *BTT1*; *TIM9* and *CDC37; TIM9* and *CPR7*; *TIM9* and *HSC82*; *TIM9* and *KAR2*; *TIM9* and *LHS1*; *TIM9* and *MGE1*; *TIM9* and *MRS11*; *TIM9* and *NOB1*; *TIM9* and *ECM10*; *TIM9* and *SSA1*; *TIM9* and *SSA2*; *TIM9* and *SSA3*; *TIM9* and *SSA4*; *TIM9* and *SSC1*; *TIM9* and *SSE2*; *TIM9* and *SIL1*; *TIM9* and *SLS1*; *TIM9* and *ORM1*; *TIM9* and *ORM2*; *TIM9* and *PER1*; *TIM9* and *PTC2*; *TIM9* and *PSE1*; *TIM9* and *UBI4*; *TIM9* and *HAC1* or a truncated intronless *HAC1*; *PAM18* and *AHA1*; *PAM18* and *CCT2*; *PAM18* and *CCT3*; *PAM18* and *CCT4*; *PAM18* and *CCT5*; *PAM18* and *CCT6*; *PAM18* and *CCT7*; *PAM18* and *CCT8*; *PAM18* and *CNS1*; *PAM18* and *CPR3*; *PAM18* and *CPR6*; *PAM18* and *ERO1*; *PAM18* and *EUG1*; *PAM18* and *FMO1*; *PAM18* and *HCH1*; *PAM18* and *HSP10*; *PAM18* and *HSP12*; *PAM18* and *HSP104*; *PAM18* and *HSP26*; *PAM18* and *HSP30*; *PAM18* and *HSP42*; *PAM18* and *HSP60*; *PAM18* and *HSP78; PAM18* and *HSP82*; *PAM18* and *JEM1*; *PAM18* and *MDJ1*; *PAM18* and *MDJ2*; *PAM18* and *MPD1*; *PAM18* and *MPD2*; *PAM18* and *PDI1*; *PAM18* and *PFD1*; *PAM18* and *ABC1*; *PAM18* and *APJ1*; *PAM18* and *ATP11*; *PAM18* and *ATP12*; *PAM18* and *BTT1*; *PAM18* and *CDC37; PAM18* and *CPR7*; *PAM18* and *HSC82*; *PAM18* and *KAR2*; *PAM18* and *LHS1*; *PAM18* and *MGE1*; *PAM18* and *MRS11*; *PAM18* and *NOB1*; *PAM18* and *ECM10*; *PAM18* and *SSA1*; *PAM18* and *SSA2*; *PAM18* and *SSA3*; *PAM18* and *SSA4*; *PAM18* and *SSC1*; *PAM18* and *SSE2*; *PAM18* and *SIL1*; *PAM18* and *SLS1*; *PAM18* and *ORM1*; *PAM18* and *ORM2*; *PAM18* and *PER1*; *PAM18* and *PTC2*; *PAM18* and *PSE1*; *PAM18* and *UBI4*; *PAM18* and *HAC1* or a truncated intronless *HAC1*; *TCP1* and *AHA1*; *TCP1* and *CCT2*; *TCP1* and *CCT3*; *TCP1* and *CCT4; TCP1* and *CCT5*; *TCP1* and *CCT6*; *TCP1* and *CCT7*; *TCP1* and *CCT8*; *TCP1* and *CNS1*; *TCP1* and *CPR3*; *TCP1* and *CPR6*; *TCP1* and *ERO1*; *TCP1* and *EUG1*; *TCP1* and *FMO1*; *TCP1* and *HCH1*; *TCP1* and *HSP10*; *TCP1* and *HSP12*; *TCP1* and *HSP104*; *TCP1* and *HSP26; TCP1* and *HSP30*; *TCP1* and *HSP42*; *TCP1* and *HSP60*; *TCP1* and *HSP78; TCP1* and *HSP82; TCP1* and *JEM1*; *TCP1* and *MDJ1*; *TCP1* and *MDJ2*; *TCP1* and *MPD1*; *TCP1* and *MPD2*; *TCP1* and *PDI1*; *TCP1* and *PFD1*; *TCP1* and *ABC1*; *TCP1* and *APJ1*; *TCP1* and *ATP11*; *TCP1* and *ATP12*; *TCP1* and *BTT1*; *TCP1* and *CDC37; TCP1* and *CPR7; TCP1* and *HSC82; TCP1* and *KAR2*; *TCP1* and *LHS1*; *TCP1* and *MGE1*; *TCP1* and *MRS11*; *TCP1* and *NOB1*; *TCP1* and *ECM10*; *TCP1* and *SSA1*; *TCP1* and *SSA2*; *TCP1* and *SSA3*; *TCP1* and *SSA4*; *TCP1* and *SSC1*; *TCP1* and *SSE2*; *TCP1* and *SIL1*; *TCP1* and *SLS1*; *TCP1* and *ORM1*; *TCP1* and *ORM2*; *TCP1* and *PER1*; *TCP1* and *PTC2; TCP1* and *PSE1*; *TCP1* and *UBI4*; *TCP1* and *HAC1* or a truncated intronless *HAC1*; *TIM9* and *PAM18*; *TIM9* and *TCP1*; *or PAM18* and *TCP1*.

The first, second and third recombinant genes may, or may not, each individually be present on a plasmid within the host cell (which may, or may not, be a 2µm-family plasmid, as discussed above) or be chromosomally integrated within the genome of the host cell. It will be appreciated that any combination of plasmid and chromosomally integrated first, second and third recombinant genes may be used. For example, the first, second and third recombinant genes may, or may not, each individually be present on a plasmid, and this may, or may not, be either the same plasmid or different plasmids. Alternatively, the first recombinant gene may, or may not, be present on a plasmid, and second and third recombinant genes may, or may not, be chromosomally integrated within the genome of the host cell. Alternatively, the first and second recombinant genes may, or may not, be present on a plasmid and the third recombinant gene may, or may not, be chromosomally integrated within the genome of the host cell. Alternatively, the first and third recombinant genes may, or may not, be present on a plasmid and the second recombinant gene may, or may not, be chromosomally integrated within the genome of the host cell. Alternatively, the first and second recombinant gene may, or may not, be chromosomally integrated within the genome of the host cell and the third recombinant gene may, or may not, be present on a plasmid. Alternatively, the first, second and third recombinant genes may, or may not, each individually be chromosomally integrated within the genome of the host cell.

Plasmids used for this purpose may, or may not, be plasmids, such as 2µm-family plasmids, as defined below. Thus, in one embodiment, a method according to the first aspect does not involve a host cell in which the first, second and third recombinant genes are all present on the 2µm-family plasmid.

Accordingly, as a second aspect, there is provided a plasmid wherein the plasmid comprises two different genes (the first and second recombinant genes) encoding different chaperones. In one preferred embodiment, the plasmid may, or may not, further comprise a gene encoding a heterologous protein (the third recombinant gene), such as a heterologous protein as described above. A plasmid according to the second aspect may, or may not, be a 2µm-family plasmid.

A third aspect provides for the use of the plasmid of the second aspect as an expression vector to increase the production of a desired protein, including as heterologous protein, such as a fungal (optionally yeast) or vertebrate protein. The desired protein may, or may not, be encoded by a recombinant gene that is present as part of the plasmid, or present in the host cell on a different plasmid, or present in the host cell as a transgene that is integrated in the host cell's chromosome.

A fourth aspect provides a host cell comprising a plasmid as defined above. The host cell may, or may not, further comprise a recombinant gene encoding a desired heterologous protein. Where the recombinant gene that encodes the desired heterologous protein (the "third recombinant gene") is not present as part of the same plasmid that encodes the first and second chaperones, then the host cell may, or may not, comprise the third recombinant gene on a different plasmid, or as a transgene that is integrated in the host cell's chromosome.

As a fifth aspect, there is provided a host cell which comprises the first, second and third recombinant genes. The first, second and third recombinant genes may, or may not, each individually be present on a plasmid within the host cell (which may, or may not, be a 2µm-family plasmid, as discussed above) or be chromosomally integrated within the genome of the host cell. It will be appreciated that any combination of plasmid and chromosomally integrated first, second and third recombinant genes may be used, as discussed above. Thus, the host cell may, or may not, comprise the first, second and third recombinant genes each individually present on a plasmid, and this may, or may not, be either the same plasmid or different plasmids. Alternatively, the host cell may, or may not, comprise the first recombinant gene on a plasmid, and second and third recombinant genes chromosomally integrated within the genome of the host cell. Alternatively, the host cell may, or may not, comprise the first and second recombinant genes on a plasmid and the third recombinant gene chromosomally integrated within the genome of the host cell. Alternatively, the host cell may, or may not, comprise the first and third recombinant genes on a plasmid and the second recombinant gene chromosomally integrated within the genome of the host cell. Alternatively, the host cell may, or may not, comprise the first and second recombinant genes chromosomally integrated within the genome of the host cell and the third recombinant gene present on a plasmid. Alternatively, the host cell may, or may not, comprise the first, second and third recombinant genes each individually chromosomally integrated within the genome of the host cell.

### The 2µm-family plasmids:

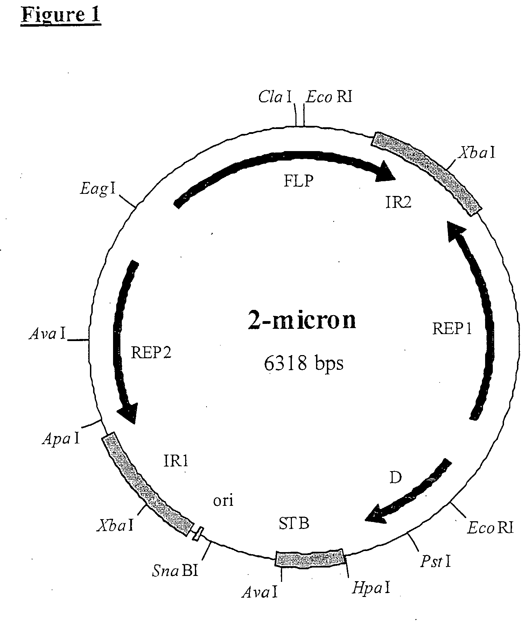

For the purposes of the present invention, a plasmid may, or may not, be a 2µm-family plasmid. Certain closely related species of budding yeast have been shown to contain naturally occurring circular double stranded DNA plasmids. These plasmids, collectively termed 2µm-family plasmids, include pSR1, pSB3 and pSB4 from *Zygosaccharomyces rouxii* (formerly classified as *Zygosaccharomyces bisporus*), plasmids pSB1 and pSB2 from *Zygosaccharomyces bailii*, plasmid pSM1 from *Zygosaccharomyces fermentati*, plasmid pKD1 from *Kluyveromyces drosphilarum*, an un-named plasmid from *Pichia membranaefaciens* (hereinafter "pPM1") and the 2µm plasmid (such as shown in Figure 1) and variants (such as Scp1, Scp2 and Scp3) from *Saccharomyces cerevisiae* (Volkert, et al., 1989, Microbiological Reviews, 53, 299; Murray et al., 1988, J. Mol. Biol. 200, 601; Painting, et al., 1984, J. Applied Bacteriology, 56, 331).

As a family of plasmids these molecules share a series of common features in that they typically possess two inverted repeats on opposite sides of the plasmid, have a similar size around 6-kbp (range 4757 to 6615-bp), three open reading frames, one of which encodes for a site specific recombinase (*FLP*) and an autonomously replicating sequence (*ARS*), also known as an origin of replication (*ori*), located close to the end of one of the inverted repeats. (Futcher, 1988, Yeast, 4, 27; Murray *et al.*, *op. cit.*, and Toh-e et al., 1986, Basic Life Sci. 40, 425). Despite their lack of discernible DNA sequence homology, their shared molecular architecture and the conservation of function of the three open reading frames have demonstrated a common ancestral link between the family members.

The above naturally occurring 2µm-family plasmids may, or may not, be used in the present invention, but this invention is not limited to the use of naturally occurring 2µm-family plasmids. For the purposes of this invention, a 2µm-family plasmid may, or may not, be as described below.

A 2µm-family plasmid is a circular, double stranded, DNA plasmid. It is typically small, such as between 3,000 to 10,000 bp, optionally between 4,500 to 7000 bp, excluding recombinantly inserted sequences.

A 2µm-family plasmid typically comprises at least three open reading frames ("ORFs") that each encodes a protein that functions in the stable maintenance of the 2µm-family plasmid as a multicopy plasmid. The proteins encoded by the three ORFs can be designated *FLP, REP1* and *REP2.* Where a 2µm-family plasmid comprises not all three of the ORFs encoding *FLP, REP1* and *REP2* then ORFs encoding the missing protein(s) should be supplied in *trans*, either on another plasmid or by chromosomal integration.

A "*FLP*" protein is a protein capable of catalysing the site-specific recombination between inverted repeat sequences recognised by *FLP.* The inverted repeat sequences are termed *FLP* recombination target (FRT) sites and each is typically present as part of a larger inverted repeat (see below). Preferred *FLP* proteins comprise the sequence of the *FLP* proteins encoded by one of plasmids pSR1, pSB1, pSB2, pSB3, pSB4, pSM1, pKD1, pPM1 and the 2µm plasmid, for example as described in Volkert *et al*, *op. cit.*, Murray *et al*, *op. cit.*, and Painting *et al.*, *op. cit.* Variants and fragments of these *FLP* proteins are also included in the present invention. "Fragments" and "variants" are those which retain the ability of the native protein to catalyse the site-specific recombination between the same FRT sequences. Such variants and fragments will usually have at least 50%, 60%, 70%, 80%, 90%, 95%, 98%, 99%, or more, homology with an *FLP* protein encoded by one of plasmids pSR1, pSB1, pSB2, pSB3, pSB4, pSM1, pKD1, pPM1 and the 2µm plasmid. Different *FLP* proteins can have different FRT sequence specificities. A typical FRT site may, or may not, comprise a core nucleotide sequence flanked by inverted repeat sequences. In the 2µm plasmid, the FRT core sequence is 8 nucleotides in length and the flanking inverted repeat sequences are 13 nucleotides in length (Volkert *et al*, *op. cit.*). However the FRT site recognised by any given *FLP* protein may, or may not, be different to the 2µm plasmid FRT site.

*REP1* and *REP2* are proteins involved in the partitioning of plasmid copies during cell division, and may, or may not, also have a role in the regulation of *FLP* expression. Considerable sequence divergence has been observed between *REP1* proteins from different 2µm-family plasmids, whereas no sequence alignment is possible between *REP2* proteins derived from different 2µm-family plasmids. Preferred *REP1* and *REP2* proteins comprise the sequence of the *REP1* and *REP2* proteins encoded by one of plasmids pSR1, pSB1, pSB2, pSB3, pSB4, pSM1, pKD1, pPM1 and the 2µm plasmid, for example as described in Volkert *et al*, *op. cit.*, Murray *et al*, *op. cit.*, and Painting *et al*, *op. cit.* Variants and fragments of these *REP1* and *REP2* proteins are also included in the present invention. "Fragments" and "variants" of *REP1* and *REP2* are those which, when encoded by the plasmid in place of the native ORF, do not substantially disrupt the stable multicopy maintenance of the plasmid within a suitable yeast population. Such variants and fragments of *REP1* and *REP2* will usually have at least 5%, 10%, 20%, 30%, 40%, 50%, 60%, 70%, 80%, 90%, 95%, 98%, 99%, or more, homology with a *REP1* and *REP2* protein, respectively, as encoded by one of plasmids pSR1, pSB1, pSB2, pSB3, pSB4, pSM1, pKD1, pPM1 and the 2µm plasmid.

The *REP1* and *REP2* proteins encoded by the ORFs on the plasmid must be compatible. It is preferred that the *REP1* and *REP2* proteins have the sequences of *REP1* and *REP2* proteins encoded by the same naturally occurring 2µm-family plasmid, such as pSR1, pSB1, pSB2, pSB3, pSB4, pSM1, pKD1, pPM1 and the 2µm plasmid, or variant or fragments thereof.

A 2µm-family plasmid typically comprises two inverted repeat sequences. The inverted repeats may be any size, so long as they each contain an FRT site (see above). The inverted repeats are typically highly homologous. They may, or may not, share greater than 50%, 60%, 70%, 80%, 90%, 95%, 96%, 97%, 98%, 99%, 99.5% or more sequence identity. In a preferred embodiment they are identical.

Typically the inverted repeats are each between 200 to 1000 bp in length. Preferred inverted repeat sequences may, or may not, each have a length of from 200 to 300 bp, 300 to 400 bp, 400 to 500 bp, 500 to 600 bp, 600 to 700 bp, 700 to 800 bp, 800 to 900 bp, or 900 to 1000 bp. Particularly preferred inverted repeats are those of the plasmids pSR1 (959 bp), pSB1 (675 bp), pSB2 (477 bp), pSB3 (391 bp), pSM1 (352 bp), pKD1 (346 bp), the 2µm plasmid (599 bp), pSB4 or pPM1.

The sequences of the inverted repeats may, or may not, be varied. However, the sequences of the FRT site in each inverted repeat should be compatible with the specificity of the *FLP* protein encoded by the plasmid, thereby to enable the encoded *FLP* protein to act to catalyse the site-specific recombination between the inverted repeat sequences of the plasmid. Recombination between inverted repeat sequences (and thus the ability of the *FLP* protein to recognise the FRT sites with the plasmid) can be determined by methods known in the art. For example, a plasmid in a yeast cell under conditions that favour *FLP* expression can be assayed for changes in the restriction profile of the plasmid which would result from a change in the orientation of a region of the plasmid relative to another region of the plasmid. The detection of changes in restriction profile indicate that the *FLP* protein is able to recognise the FRT sites in the plasmid and therefore that the FRT site in each inverted repeat are compatible with the specificity of the *FLP* protein encoded by the plasmid.

In a particularly preferred embodiment, the sequences of inverted repeats, including the FRT sites, are derived from the same 2µm-family plasmid as the ORF encoding the *FLP* protein, such as pSR1, pSB1, pSB2, pSB3, pSB4, pSM1, pKD1, pPM1 or the 2µm plasmid.

The inverted repeats are typically positioned with the 2µm-family plasmid such that the two regions defined between the inverted repeats (e.g. such as defined as UL and US in the 2µm plasmid) are of approximately similar size, excluding exogenously introduced sequences such as transgenes. For example, one of the two regions may, or may not, have a length equivalent to at least 40%, 50%, 60%, 70%, 80%, 90%, 95% or more, up to 100%, of the length of the other region.

A 2µm-family plasmid typically comprises the ORF that encodes *FLP* and one inverted repeat (arbitrarily termed "IR1" to distinguish it from the other inverted repeat mentioned in the next paragraph) juxtaposed in such a manner that IR1 occurs at the distal end of the *FLP* ORF, without any intervening coding sequence, for example as seen in the 2µm plasmid. By "distal end" in this context we mean the end of the *FLP* ORF opposite to the end from which the promoter initiates its transcription. In a preferred embodiment, the distal end of the *FLP* ORF overlaps with IR1.

A 2µm-family plasmid typically comprises the ORF that encodes *REP2* and the other inverted repeat (arbitrarily termed "IR2" to distinguish it from IR1 mentioned in the previous paragraph) juxtaposed in such a manner that IR2 occurs at the distal end of the *REP2* ORF, without any intervening coding sequence, for example as seen in the 2µm plasmid. By "distal end" in this context we mean the end of the *REP2* ORF opposite to the end from which the promoter initiates its transcription.

In one embodiment, the ORFs encoding *REP2* and *FLP* may, or may not, be present on the same region of the two regions defined between the inverted repeats of the 2µm-family plasmid, which region may be the bigger or smaller of the regions (if there is any inequality in size between the two regions).

In one embodiment, the ORFs encoding *REP2* and *FLP* may, or may not, be transcribed from divergent promoters.

Typically, the regions defined between the inverted repeats (e.g. such as defined as UL and US in the 2µm plasmid) of a 2µm-family plasmid may, or may not, comprise not more than two endogenous genes that encode a protein that functions in the stable maintenance of the 2µm-family plasmid as a multicopy plasmid. Thus in a preferred embodiment, one region of the plasmid defined between the inverted repeats may, or may not, comprise not more than the ORFs encoding *FLP* and *REP2*; *FLP* and *REP1*; or *REP1* and *REP2,* as endogenous coding sequence.

A 2µm-family plasmid typically comprises an origin of replication (also known as an "autonomously replicating sequence - "ARS"), which is typically bidirectional. Any appropriate ARS sequence can be present. Consensus sequences typical of yeast chromosomal origins of replication may, or may not, be appropriate (Broach et al, 1982, Cold Spring Harbor Symp. Quant. Biol., 47, 1165-1174; Williamson, Yeast, 1985, 1, 1-14). Preferred ARSs include those isolated from pSR1, pSB1, pSB2, pSB3, pSB4, pSM1, pKD1, pPM1 and the 2µm plasmid.

Thus, a preferred 2µm-family plasmid may, or may not, comprise ORFs encoding *FLP, REP1* and *REP2,* two inverted repeat sequences each inverted repeat comprising an FRT site compatible with the encoded *FLP* protein, and an ARS sequence. Preferably the FRT sites are derived from the same 2µm-family plasmid as the sequence of the encoded *FLP* protein. More preferably the sequences of the encoded *REP1* and *REP2* proteins are derived from the same 2µm-family plasmid as each other. Even more preferably, the FRT sites are derived from the same 2µm-family plasmid as the sequence of the encoded *FLP, REP1* and *REP2* proteins. Yet more preferably, the sequences of the ORFs encoding *FLP, REP1* and *REP2,* and the sequence of the inverted repeats (including the FRT sites) are derived from the same 2µm-family plasmid. Furthermore, the ARS site may, or may not, be derived from the same 2µm-family plasmid as one or more of the ORFs of *FLP*, *REP1* and *REP2*, and the sequence of the inverted repeats (including the FRT sites).

The term "derived from" includes sequences having an identical sequence to the sequence from which they are derived. However, variants and fragments thereof, as defined above, are also included. For example, an *FLP* gene having a sequence derived from the *FLP* gene of the 2µm plasmid may, or may not, have a modified promoter or other regulatory sequence compared to that of the naturally occurring gene. Additionally or alternatively, an *FLP* gene having a sequence derived from the *FLP* gene of the 2µm plasmid may, or may not, have a modified nucleotide sequence in the open reading frame which may, or may not, encode the same protein as the naturally occurring gene, or may, or may not, encode a modified *FLP* protein. The same considerations apply to other sequences on a 2µm-family plasmid having a sequence derived from a particular source.

Optionally, a 2µm-family plasmid may, or may not, comprise a region derived from the *STB* region (also known as REP3) of the 2µm plasmid, as defined in Volkert *et al*, *op. cit.* The *STB* region in a 2µm-family plasmid of the invention may, or may not, comprise two or more tandem repeat sequences, such as three, four, five or more. Alternatively, no tandem repeat sequences may be present. The tandem repeats may be any size, such as 10, 20, 30, 40, 50, 60 70, 80, 90, 100 bp or more in length. The tandem repeats in the *STB* region of the 2µm plasmid are 62 bp in length. It is not essential for the sequences of the tandem repeats to be identical. Slight sequence variation can be tolerated. It may, or may not, be preferable to select an *STB* region from the same plasmid as either or both of the *REP1* and *REP2* ORFs. The *STB* region is thought to be a *cis*-acting element and preferably is not transcribed.

Optionally, a 2µm-family plasmid may, or may not, comprise an additional ORF that encodes a protein that functions in the stable maintenance of the 2µm-family plasmid as a multicopy plasmid. The additional protein can be designated RAF or D. ORFs encoding the RAF or D gene can be seen on, for example, the 2µm plasmid and pSM1. Thus a RAF or D ORF can comprise a sequence suitable to encode the protein product of the RAF or D gene ORFs encoded by the 2µm plasmid or pSM1, or variants and fragments thereof. Thus variants and fragments of the protein products of the RAF or D genes of the 2µm plasmid or pSM1 are also included in the present invention. "Fragments" and "variants" of the protein products of the RAF or D genes of the 2µm plasmid or pSM1 are those which, when encoded by the 2µm plasmid or pSM1 in place of the native ORF, do not disrupt the stable multicopy maintenance of the plasmid within a suitable yeast population. Such variants and fragments will usually have at least 5%, 10%, 20%, 30%, 40%, 50%, 60%, 70%, 80%, 90%, 95%, 98%, 99%, or more, homology with the protein product of the RAF or D gene ORFs encoded by the 2µm plasmid or pSM1.

A naturally occurring 2µm-family plasmid may, or may not, be preferred. A naturally occurring 2µm-family plasmid is any plasmid having the features defined above, which plasmid is found to naturally exist in yeast, i.e. has not been recombinantly modified to include heterologous sequence. Optionally the naturally occurring 2µm-family plasmid is selected from pSR1 (Accession No. X02398), pSB3 (Accession No. X02608) or pSB4 as obtained from *Zygosaccharomyces rouxii*, pSB1 or pSB2 (Accession No. NC_002055 or M18274) both as obtained from *Zygosaccharomyces bailli*, pSM1 (Accession No. NC_002054) as obtained from *Zygosaccharomyces fermentati*, pKD1 (Accession No. X03961) as obtained from *Kluyveromyces drosophilarum,* pPM1 from *Pichia membranaefaciens* or, preferably, the 2µm plasmid (Accession No. NC_001398 or J01347) as obtained from *Saccharomyces cerevisiae*. Accession numbers in this paragraph refer to NCBI deposits.

The 2µm plasmid (Figure 1) is a 6,318-bp double-stranded DNA plasmid, endogenous in most *Saccharomyces cerevisiae* strains at 60-100 copies per haploid genome. The 2µm plasmid comprises a small unique (US) region and a large unique (UL) region, separated by two 599-bp inverted repeat sequences. Site-specific recombination of the inverted repeat sequences results in interconversion between the A-form and B-form of the plasmid *in vivo* (Volkert & Broach, 1986, Cell, 46, 541). The two forms of 2µm differ only in the relative orientation of their unique regions.

While DNA sequencing of a cloned 2µm plasmid (also known as Scp1) from *Saccharomyces cerevisiae* gave a size of 6,318-bp (Hartley and Donelson, 1980, Nature, 286, 860), other slightly smaller variants of 2µm, Scp2 and Scp3, are known to exist as a result of small deletions of 125-bp and 220-bp, respectively, in a region known as *STB* (Cameron et al., 1977, Nucl. Acids Res., 4, 1429: Kikuchi, 1983, Cell, 35, 487 and Livingston & Hahne, 1979, Proc. Natl. Acad. Sci. USA, 76, 3727). In one study about 80% of natural *Saccharomyces* strains from around the world contained DNA homologous to 2µm (by Southern blot analysis) (Hollenberg, 1982, Current Topics in Microbiology and Immunobiology, 96, 119). Furthermore, variation (genetic polymorphism) occurs within the natural population of 2µm plasmids found in *S. cerevisiae* and *S. carlsbergensis*, with the NCBI sequence (accession number NC_001398) being one example.

The 2µm plasmid has a nuclear localisation and displays a high level of mitotic stability (Mead et al, 1986, Molecular & General Genetics, 205, 417). The inherent stability of the 2µm plasmid results from a plasmid-encoded copy number amplification and partitioning mechanism, which can be compromised during the development of chimeric vectors (Futcher & Cox, 1984, J. Bacterial., 157, 283; Bachmair & Ruis, 1984, Monatshefte für Chemie, 115, 1229). A yeast strain, which contains a 2µm plasmid is known as [cir⁺], while a yeast strain which does not contain a 2µm plasmid is known as [cir⁰].

The US-region of the 2µm plasmid contains the *REP2* and *FLP* genes, and the UL-region contains the *REP1* and *D* (also known as *RAF*) genes, the *STB*-locus and the origin of replication (Broach & Hicks, 1980, Cell, 21, 501; Sutton & Broach, 1985, Mol. Cell. Biol., 5, 2770). The Flp recombinase binds to FRT-sites (Flp Recognition Target) within the inverted repeats to mediate site-specific recombination, which is essential for natural plasmid amplification and control of plasmid copy number *in vivo* (Senecoff et al, 1985, Proc. Natl. Acad. Sci. U.S.A., 82, 7270; Jayaram, 1985, Proc. Natl. Acad. Sci. U.S.A., 82, 5875). The copy number of 2µm-family plasmids can be significantly affected by changes in Flp recombinase activity (Sleep et al, 2001, Yeast, 18, 403; Rose & Broach, 1990, Methods Enzymol., 185, 234). The Rep1 and Rep2 proteins mediate plasmid segregation, although their mode of action is unclear (Sengupta et al, 2001, J. Bacterial., 183, 2306). They also repress transcription of the *FLP* gene (Reynolds et al, 1987, Mol. Cell. Biol., 7, 3566).

The *FLP* and *REP2* genes of the 2µm plasmid are transcribed from divergent promoters, with apparently no intervening sequence defined between them. The *FLP* and *REP2* transcripts both terminate at the same sequence motifs within the inverted repeat sequences, at 24-bp and 178-bp respectively after their translation termination codons (Sutton & Broach, 1985, Mol. Cell. Biol., 5, 2770).

In the case of *FLP,* the C-terminal coding sequence also lies within the inverted repeat sequence. Furthermore, the two inverted repeat sequences are highly conserved over 599-bp, a feature considered advantageous to efficient plasmid replication and amplification *in vivo*, although only the FRT-sites (less than 65-bp) are essential for site-specific recombination *in vitro* (Senecoff et al, 1985, Proc. Natl. Acad. Sci. U.S.A., 82, 7270; Jayaram, 1985, Proc. Natl. Acad. Sci. U.S.A., 82, 5875; Meyer-Leon et al, 1984, Cold Spring Harbor Symposia On Quantitative Biology, 49, 797). The key catalytic residues of Flp are arginine-308 and tyrosine-343 (which is essential) with strand-cutting facilitated by histidine-309 and histidine 345 (Prasad et al, 1987, Proc. Natl. Acad. Sci. U.S.A., 84, 2189; Chen et al, 1992, Cell, 69, 647; Grainge et al, 2001, J. Mol. Biol., 314, 717).

Two functional domains are described in Rep2. Residues 15-58 form a Rep1-binding domain, and residues 59-296 contain a self-association and STB-binding region (Sengupta et al, 2001, J. Bacterial., 183, 2306).

Chimeric or large deletion mutant derivatives of 2µm which lack many of the essential functional regions of the 2µm plasmid but retain the functional *cis* element *ARS* and *STB*, cannot effectively partition between mother and daughter cells at cell division. Such plasmids can do so if these functions are supplied in *trans*, by for instance the provision of a functional 2µm plasmid within the host, such as a [cir⁺] host.

Genes of interest have previously been inserted into the UL-region of the 2µm plasmid. For example, see plasmid pSAC3U1 in EP 0 286 424 and the plasmid shown in Figure 2 of WO 2005/061718, which includes a β-lactamase gene (for ampicillin resistance), a *LEU2* selectable marker and an oligonucleotide linker, the latter two of which are inserted into a unique *Sna*BI-site within the UL-region of the 2µm-like disintegration vector, pSAC3 (see EP 0 286 424). The *E. coli* DNA between the *Xba*I-sites that contains the ampicillin resistance gene is lost from the plasmid shown in Figure 2 of WO 2005/061718 after transformation into yeast. This is described in Chinery & Hinchliffe, 1989, Curr. Genet., 16, 21 and EP 0 286 424, where these types of vectors are designated "disintegration vectors". Further polynucleotide insertions can be made in a *Not*I-site within a linker (Sleep et al, 1991, Biotechnology (N Y), 9, 183).

Alternative insertion sites in 2µm plasmid are known in the art, including those described in Rose & Broach (1990, Methods Enzymol., 185, 234-279), such as plasmids pCV19, pCV20, CVₙₑₒ, which utilise an insertion at *Eco*RI in *FLP,* plasmids pCV21, pGT41 and pYE which utilise *EcoRI* in *D* as the insertion site, plasmid pHKB52 which utilises *Pst*I in *D* as the insertion site, plasmid pJDB248 which utilises an insertion at *Pst*I in *D* and *EcoRI* in *D,* plasmid pJDB219 in which *Pst*I in *D* and *Eco*RI in *FLP* are used as insertion sites, plasmid G18, plasmid pAB18 which utilises an insertion at *Cla*I in *FLP,* plasmids pGT39 and pA3, plasmids pYT11, pYT14 and pYT11-LEU which use *Pst*I in *D* as the insertion site, and plasmid PTY39 which uses *EcoRI* in *FLP* as the insertion site. Other 2µm plasmids include pSAC3, pSAC3U1, pSAC3U2, pSAC300, pSAC310, pSAC3C1, pSAC3PL1, pSAC3SL4, and pSAC3SC1 are described in EP 0 286 424 and Chinery & Hinchliffe (1989, Curr. Genet., 16, 21-25) which also described *Pst*I, *Eag*I or *Sna*BI as appropriate 2µm insertion sites. Further 2µm plasmids include pAYE255, pAYE316, pAYE443, pAYE522 (Kerry-Williams et al, 1998, Yeast, 14, 161-169), pDB2244 (WO 00/44772), and pAYE329 (Sleep et al, 2001, Yeast, 18, 403-421).

In one preferred embodiment, one or more genes are inserted into a 2µm-family plasmid within an untranscribed region around the ARS sequence. For example, in the 2µm plasmid obtained from *S. cerevisiae*, the untranscribed region around the ARS sequence extends from the end of the D gene to the beginning of ARS sequence. Insertion into *Sna*BI (near the origin of replication sequence ARS) is described in Chinery & Hinchliffe, 1989, Curr. Genet., 16, 21-25. The skilled person will appreciate that gene insertions can also be made in the untranscribed region at neighbouring positions to the *Sna*BI site described in Chinery & Hinchliffe.

In another preferred embodiment, *REP2* and *FLP* genes in a 2µm-family plasmid each have an inverted repeat adjacent to them, and one or more genes are inserted into a 2µm-family plasmid within the region between the first base after the last functional codon of either the *REP2* gene or the *FLP* gene and the last base before the FRT site in the inverted repeat adjacent to said gene. The last functional codon of either a *REP2* gene or a *FLP* gene is the codon in the open reading frame of the gene that is furthest downstream from the promoter of the gene whose replacement by a stop codon will lead to an unacceptable loss of multicopy stability of the plasmid, as defined herein. Thus, disruption of the *REP2* or *FLP* genes at any point downstream of the last functional codon in either gene, by insertion of a polynucleotide sequence insertion, deletion or substitution will not lead to an unacceptable loss of multicopy stability of the plasmid.

For example, the *REP2* gene of the 2µm plasmid can be disrupted after codon 59 and that the *FLP* gene of the 2µm plasmid can be disrupted after codon 344, each without a loss of multicopy stability of the plasmid. The last functional codon in equivalent genes in other 2µm-family plasmids can be determined routinely by making mutants of the plasmids in either the *FLP* or *REP2* genes and following the tests set out herein to determine whether the plasmid retains multicopy stability. Thus, a plasmid insertion site as defined in WO 2005/061719 may, or may not, be used to carry one or more a recombinant genes according to any aspect of the present invention, including those described in the text relating the 'main aspect' of the invention.

One can determine whether a plasmid retains multicopy stability using test such as defined in Chinery & Hinchliffe (1989, Curr. Genet., 16, 21-25). For yeast that do not grow in the non-selective media (YPD, also designated YEPD) defined in Chinery & Hinchliffe (1989, Curr. Genet., 16, 21-25) other appropriate non-selective media might be used. Plasmid stability may be defined as the percentage cells remaining prototrophic for the selectable marker after a defined number of generations. The number of generations will preferably be sufficient to show a difference between a control plasmid, such as pSAC35 or pSAC310, or to show comparable stability to such a control plasmid. The number of generations may be 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100 or more. Higher numbers are preferred. The acceptable plasmid stability might be 1%, 2%, 3%, 4%, 5%, 10%, 15%, 20%, 25%, 30%, 40%, 50%, 60%, 70%, 75%, 80%, 85%, 90%, 95%, 96%, 97%, 98%, 99%, 99.9% or substantially 100%. Higher percentages are preferred. The skilled person will appreciate that, even though a plasmid may have a stability less than 100% when grown on non-selective media, that plasmid can still be of use when cultured in selective media. For example plasmid pDB2711 as described in the examples is only 10% stable when the stability is determined accordingly to test of Example 2 of WO 2005/061719, but provides a 15-fold increase in recombinant transferrin productivity in shake flask culture under selective growth conditions.

Thus one or more gene insertions may, or may not, occur between the first base after the last functional codon of the *REP2* gene and the last base before the FRT site in an inverted repeat adjacent to said gene, preferably between the first base of the inverted repeat and the last base before the FRT site, more preferably at a position after the translation termination codon of the *REP2* gene and before the last base before the FRT site.

Additionally or alternatively one or more gene insertions may, or may not, occur between the first base after the last functional codon of the *FLP* gene and the last base before the FRT site in an inverted repeat adjacent to said gene, preferably between the first base of the inverted repeat and the last base before the FRT site, more preferably between the first base after the end of the *FLP* coding sequence and the last base before the FRT site, such as at the first base after the end of the *FLP* coding sequence.

In one preferred embodiment, where the 2µm-family plasmid is based on the 2µm plasmid of *S. cerevisiae*, it is a disintegration vector as known in the art (for example, see EP 286 424). A disintegration vector may, or may not, be a 2µm plasmid vector comprising a DNA sequence which is intended to be lost by recombination, three 2µm FRT sites, of which one pair of sites is in direct orientation and the other two pairs are in indirect orientation, and a DNA sequence of interest (such as an *E. coli* origin of replication and bacterial selectable marker), the said sequence to be lost being located between the said sites which are in direct orientation.

Thus, the sequence to be lost may, or may not, comprise a selectable marker DNA sequence.

A preferred disintegration vector may, or may not, comprise a complete 2µm plasmid additionally carrying (i) a bacterial plasmid DNA sequence necessary for propagation of the vector in a bacterial host; (ii) an extra 2µm FRT site; and a selectable marker DNA sequence for yeast transformation; the said bacterial plasmid DNA sequence being present and the extra FRT site being created at a restriction site, such as *Xba*I, in one of the two inverted repeat sequences of the 2µm plasmid, the said extra FRT site being in direct orientation in relation to the endogenous FRT site of the said one repeat sequence, and the bacterial plasmid DNA sequence being sandwiched between the extra FRT site and the endogenous FRT site of the said one repeat sequence. In a preferred disintegration vector, all bacterial plasmid DNA sequences may, or may not, be sandwiched as said. A particularly preferred 2µm plasmid vector has substantially the configuration of pSAC3 as shown in EP 286 424.

The term "disintegration vector" as used herein also includes plasmids as defined in US 6,451,559. Thus a disintegration vector may, or may not, be a 2µm vector that, other than DNA sequence encoding non-yeast polypeptides, contains no bacterial (particularly *E. coli*) origin of replication, or more preferably no bacterial (particularly *E. coli*) sequence and preferably all DNA in said vector, other than DNA sequence encoding non-yeast polypeptides, is yeast-derived DNA.

### Desired proteins and other proteins defined by the present application:

The terms "protein" and "desired protein" as used herein include all natural and non-natural proteins, polypeptides and peptides. For the purposes of the present invention, a "heterologous protein" is a protein that is encoded by a "recombinant gene" as described above. The "heterologous protein" may, or may not, be identical in sequence to a protein that is encoded by one of more other genes that naturally occur in the expression system that is used (by "expression system" we include the meaning of a host cell's genome (typically the chromosome) where the "recombinant gene" is chromosomally integrated, or a plasmid where the "recombinant gene" is encoded by a plasmid). For example, in the context of a "heterologous protein" that is encoded by a "recombinant gene" carried on a 2µm-family plasmid, the "heterologous protein" may, or may not, be a protein that is not naturally encoded by a 2µm-family plasmid and can also be described as a "non 2µm-family plasmid protein". For convenience, the terms "heterologous protein" and "non 2µm-family plasmid protein" are used synonymously in this application. Optionally therefore, when encoded by a 2µm-family, the heterologous protein is not a *FLP*, *REP1*, *REP2*, or a RAF/D protein as encoded by any one of pSR1, pSB3 or pSB4 as obtained from *Z*. *rouxii*, pSB1 or pSB2 both as obtained from Z. *bailli*, pSM1 as obtained from *Z*. *fermentati*, pKD1 as obtained from *K. drosophilarum*, pPM1 as obtained from P. *membranaefaciens* or the 2µm plasmid as obtained from *S. cerevisiae.*

A gene encoding a desired heterologous, or other, protein comprises a polynucleotide sequence encoding the heterologous protein (typically according to standard codon usage for any given organism), designated the open reading frame ("ORF"). The gene may, or may not, additionally comprise some polynucleotide sequence that does not encode an open reading frame (termed "non-coding region").

Non-coding region in the gene may, or may not, contain one or more regulatory sequences, operatively linked to the ORF, which allow for the transcription of the open reading frame and/or translation of the resultant transcript.

The term "regulatory sequence" refers to a sequence that modulates (i.e., promotes or reduces) the expression (i.e., the transcription and/or translation) of an ORF to which it is operably linked. Regulatory regions typically include promoters, terminators, ribosome binding sites and the like. The skilled person will appreciate that the choice of regulatory region will depend upon the intended expression system. For example, promoters may, or may not, be constitutive or inducible and may, or may not, be cell- or tissue-type specific or non-specific.

Suitable regulatory regions, may, or may not, be 5bp, 10bp, 15bp, 20bp, 25bp, 30bp, 35bp, 40bp, 45bp, 50bp, 60bp, 70bp, 80bp, 90bp, 100bp, 120bp, 140bp, 160bp, 180bp, 200bp, 220bp, 240bp, 260bp, 280bp, 300bp, 350bp, 400bp, 450bp, 500bp, 550bp, 600bp, 650bp, 700bp, 750bp, 800bp, 850bp, 900bp, 950bp, 1000bp, 1100bp, 1200bp, 1300bp, 1400bp, 1500bp or greater, in length.

Those skilled in the art will recognise that the gene encoding a chaperone, for example PDI, may, or may not, additionally comprise non-coding regions and/or regulatory regions. Such non-coding regions and regulatory regions are not restricted to the native non-coding regions and/or regulatory regions normally associated with the chaperone ORF.

Where the expression system is yeast, such as *Saccharomyces cerevisiae*, suitable promoters for *S. cerevisiae* include those associated with the *PGK1* gene, *GAL1* or *GAL10* genes, *TEF1*, *TEF2*, *PYK1*, *PM47*, *CYC1*, *PHO5*, *TRP1*, *ADH1*, *ADH2*, the genes for glyceraldehyde-3-phosphate dehydrogenase, hexokinase, pyruvate decarboxylase, phosphofructokinase, triose phosphate isomerase, phosphoglucose isomerase, glucokinase, α-mating factor pheromone, α-mating factor pheromone, the *PRB1* promoter, the *PRA1* promoter, the *GPD1* promoter, and hybrid promoters involving hybrids of parts of 5' regulatory regions with parts of 5' regulatory regions of other promoters or with upstream activation sites (e.g. the promoter of EP-A-258 067).

Suitable transcription termination signals are well known in the art. Where the host cell is eucaryotic, the transcription termination signal is optionally derived from the 3' flanking sequence of a eucaryotic gene, which contains proper signals for transcription termination and polyadenylation. Suitable 3' flanking sequences may, or may not, for example, be those of the gene naturally linked to the expression control sequence used, i.e. may, or may not, correspond to the promoter. Alternatively, they may be different. In that case, and where the host is a yeast, optionally *S. cerevisiae*, then the termination signal of the *S. cerevisiae ADH1*, *ADH2*, *CYC1*, or *PGK1* genes are preferred.

It may, or may not, be beneficial for the promoter and open reading frame of the gene, such as a gene encoding the chaperone (e.g. *PDI1*) or a desired protein (such as a heterologous desired protein), to be flanked by transcription termination sequences so that the transcription termination sequences are located both upstream and downstream of the promoter and open reading frame, in order to prevent transcriptional read-through into neighbouring genes, such as 2µm genes, and vice versa.

In one embodiment, the favoured regulatory sequences in yeast, such as *Saccharomyces cerevisiae*, include: a yeast promoter (e.g. the *Saccharomyces cerevisiae PRB1* promoter), as taught in EP 431 880; and a transcription terminator, optionally the terminator from *Saccharomyces ADH1*, as taught in EP 60 057. Optionally, the vector incorporates at least two translation stop codons.

It may, or may not, be beneficial for the non-coding region to incorporate more than one DNA sequence encoding a translational stop codon, such as UAA, UAG or UGA, in order to minimise translational read-through and thus avoid the production of elongated, non-natural fusion proteins. The translation stop codon UAA is preferred.

The term "operably linked" includes within its meaning that a regulatory sequence is positioned within any non-coding region in a gene such that it forms a relationship with an ORF that permits the regulatory region to exert an effect on the ORF in its intended manner. Thus a regulatory region "operably linked" to an ORF is positioned in such a way that the regulatory region is able to influence transcription and/or translation of the ORF in the intended manner, under conditions compatible with the regulatory sequence.

In one preferred embodiment, the desired protein (such as the heterologous desired protein) is secreted. In that case, a sequence encoding a secretion leader sequence which, for example, comprises most of the natural HSA secretion leader, plus a small portion of the *S. cerevisiae* α-mating factor secretion leader as taught in WO 90/01063 may, or may not, be included in the open reading frame.

Alternatively, the desired protein (such as a heterologous desired protein) may, or may not, be intracellular.

The desired protein (such as a heterologous desired protein) may, or may not, comprise the sequence of a eucaryotic protein, or a fragment or variant thereof. Suitable eucaryotes include fungi, plants and animals. In one embodiment the heterologous protein may, or may not, be a fungal protein, such as a yeast protein. In another preferred embodiment the desired protein (such as a heterologous desired protein) may, or may not, be an animal protein. Exemplary animals include vertebrates and invertebrates. Exemplary vertebrates include mammals, such as humans, and non-human mammals.

Thus the desired protein (such as a heterologous desired protein) may, or may not, comprise the sequence of a yeast protein. It may, or may not, for example, comprise the sequence of a yeast protein from the same host from which a 2µm-family plasmid is derived, particularly if the gene encoding the heterologous protein is integrated into said 2µm-family plasmid. Those skilled in the art will recognise that a method, use or plasmid of the invention may, or may not, comprise DNA sequences encoding more than one heterologous protein, more than one chaperone, or more than one heterologous protein and more than one chaperone.

In another embodiment, the desired protein (such as a desired heterologous protein) may, or may not, comprise the sequence of albumin, a monoclonal antibody, an etoposide, a serum protein (such as a blood clotting factor), antistasin, a tick anticoagulant peptide, transferrin, lactoferrin, endostatin, angiostatin, collagens, immunoglobulins or immunoglobulin-based molecules or fragment of either (e.g. a Small Modular ImmunoPharmaceutical™ ("SMIP") or dAb, Fab' fragments, F(ab')2, scAb, scFv or scFv fragment), a Kunitz domain protein (such as those described in WO 03/066824, with or without albumin fusions), interferons, interleukins, IL10, IL11, IL2, interferon α species and sub-species, interferon β species and sub-species, interferon γ species and sub-species, leptin, CNTF, CNTF_{Ax15}, IL1-receptor antagonist, erythropoietin (EPO) and EPO mimics, thrombopoietin (TPO) and TPO mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, α₁-antitrypsin, plasminogen activators, Factor VII, Factor VIII, Factor IX, Factor X and Factor XIII, nerve growth factor, LACI, platelet-derived endothelial cell growth factor (PD-ECGF), glucose oxidase, serum cholinesterase, aprotinin, amyloid precursor protein, inter-alpha trypsin inhibitor, antithrombin III, apo-lipoprotein species, Protein C, Protein S, a metabolite, an antibiotic, or a variant or fragment of any of the above.

A "variant", in the context of the above-listed proteins, refers to a protein wherein at one or more positions there have been amino acid insertions, deletions, or substitutions, either conservative or non-conservative, provided that such changes result in a protein whose basic properties, for example enzymatic activity or receptor binding (type of and specific activity), thermostability, activity in a certain pH-range (pH-stability) have not significantly been changed. "Significantly" in this context means that one skilled in the art would say that the properties of the variant may still be different but would not be unobvious over the ones of the original protein.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

A "variant" typically has at least 25%, at least 50%, at least 60% or at least 70%, preferably at least 80%, more preferably at least 90%, even more preferably at least 95%, yet more preferably at least 99%, most preferably at least 99.5% sequence identity to the polypeptide from which it is derived.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson et al., (1994) Nucleic Acids Res., 22(22), 4673-80). The parameters used may, or may not, be as follows:
- Fast pairwise alignment parameters: K-tuple(word) size; 1, window size; 5, gap penalty; 3, number of top diagonals; 5. Scoring method: x percent.
- Multiple alignment parameters: gap open penalty; 10, gap extension penalty; 0.05.
- Scoring matrix: BLOSUM.

Such variants may, or may not, be natural or made using the methods of protein engineering and site-directed mutagenesis as are well known in the art.

A "fragment", in the context of the above-listed proteins, refers to a protein wherein at one or more positions there have been deletions. Thus the fragment may, or may not, comprise at most 5, 10, 20, 30, 40 or 50% of the complete sequence of the full mature polypeptide. Typically a fragment comprises up to 60%, more typically up to 70%, preferably up to 80%, more preferably up to 90%, even more preferably up to 95%, yet more preferably up to 99% of the complete sequence of the full desired protein. Particularly preferred fragments of a protein comprise one or more whole domains of the protein.

In one particularly preferred embodiment the desired protein (such as a desired heterologous protein) comprises the sequence of albumin or a variant or fragment thereof.

By "albumin" we include a protein comprising the sequence of an albumin protein obtained from any source. Typically the source is mammalian. In one preferred embodiment the serum albumin is human serum albumin ("HSA"). The term "human serum albumin" includes the meaning of a serum albumin having an amino acid sequence naturally occurring in humans, and variants thereof. Optionally the albumin has the amino acid sequence disclosed in WO 90/13653 or a variant thereof. The HSA coding sequence is obtainable by known methods for isolating cDNA corresponding to human genes, and is also disclosed in, for example, EP 73 646 and EP 286 424.

In another preferred embodiment the "albumin" comprises the sequence of bovine serum albumin. The term "bovine serum albumin" includes the meaning of a serum albumin having an amino acid sequence naturally occurring in cows, for example as taken from Swissprot accession number P02769, and variants thereof as defined below. The term "bovine serum albumin" also includes the meaning of fragments of full-length bovine serum albumin or variants thereof, as defined below.

In another preferred embodiment the albumin comprises the sequence of an albumin derived from one of serum albumin from dog (e.g. see Swissprot accession number P49822), pig (e.g. see Swissprot accession number P08835), goat (e.g. as available from Sigma as product no. A2514 or A4164), turkey (e.g. see Swissprot accession number 073860), baboon (e.g. as available from Sigma as product no. A1516), cat (e.g. see Swissprot accession number P49064), chicken (e.g. see Swissprot accession number P19121), ovalbumin (e.g. chicken ovalbumin) (e.g. see Swissprot accession number P01012), donkey (e.g. see Swissprot accession number P39090), guinea pig (e.g. as available from Sigma as product no. A3060, A2639, 05483 or A6539), hamster (e.g. as available from Sigma as product no. A5409), horse (e.g. see Swissprot accession number P35747), rhesus monkey (e.g. see Swissprot accession number Q28522), mouse (e.g. see Swissprot accession number 089020), pigeon (e.g. as defined by Khan et al, 2002, Int. J. Biol. Macromol., 30(3-4),171-8), rabbit (e.g. see Swissprot accession number P49065), rat (e.g. see Swissprot accession number P36953) and sheep (e.g. see Swissprot accession number P14639) and includes variants and fragments thereof as defined below.

Many naturally occurring mutant forms of albumin are known. Many are described in Peters, (1996, All About Albumin: Biochemistry, Genetics and Medical Applications, Academic Press, Inc., San Diego, California, p.170-181). A variant as defined above may, or may not, be one of these naturally occurring mutants.

A "variant albumin" refers to an albumin protein wherein at one or more positions there have been amino acid insertions, deletions, or substitutions, either conservative or non-conservative, provided that such changes result in an albumin protein for which at least one basic property, for example binding activity (type of and specific activity e.g. binding to bilirubin), osmolarity (oncotic pressure, colloid osmotic pressure), behaviour in a certain pH-range (pH-stability) has not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gln; Ser, Thr; Lys, Arg; and Phe, Tyr. Such variants may, or may not, be made by techniques well known in the art, such as by site-directed mutagenesis as disclosed in US Patent No 4,302,386 issued 24 November 1981 to Stevens.

Typically an albumin variant will have more than 40%, usually at least 50%, more typically at least 60%, preferably at least 70%, more preferably at least 80%, yet more preferably at least 90%, even more preferably at least 95%, most preferably at least 98% or more sequence identity with naturally occurring albumin. The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally. The alignment may alternatively be carried out using the Clustal W program (Thompson *et al.*, 1994). The parameters used may, or may not, be as follows:
Fast pairwise alignment parameters: K-tuple(word) size; 1, window size; 5, gap penalty; 3, number of top diagonals; 5. Scoring method: x percent. Multiple alignment parameters: gap open penalty; 10, gap extension penalty; 0.05. Scoring matrix: BLOSUM.

The term "fragment" as used above includes any fragment of full-length albumin or a variant thereof, so long as at least one basic property, for example binding activity (type of and specific activity e.g. binding to bilirubin), osmolarity (oncotic pressure, colloid osmotic pressure), behaviour in a certain pH-range (pH-stability) has not significantly been changed. "Significantly" in this context means that one skilled in the art would say that the properties of the variant may still be different but would not be unobvious over the ones of the original protein. A fragment will typically be at least 50 amino acids long. A fragment may, or may not, comprise at least one whole sub-domain of albumin. Domains of HSA have been expressed as recombinant proteins (Dockal, M. et al., 1999, J. Biol. Chem., 274, 29303-29310), where domain I was defined as consisting of amino acids 1-197, domain II was defined as consisting of amino acids 189-385 and domain III was defined as consisting of amino acids 381-585. Partial overlap of the domains occurs because of the extended α-helix structure (h10-h1) which exists between domains I and II, and between domains II and III (Peters, 1996, *op. cit*., Table 2-4). HSA also comprises six sub-domains (sub-domains IA, IB, IIA, IIB, IIIA and IIIB). Sub-domain IA comprises amino acids 6-105, sub-domain IB comprises amino acids 120-177, sub-domain IIA comprises amino acids 200-291, sub-domain IIB comprises amino acids 316-369, sub-domain IIIA comprises amino acids 392-491 and sub-domain IIIB comprises amino acids 512-583. A fragment may, or may not, comprise a whole or part of one or more domains or sub-domains as defined above, or any combination of those domains and/or sub-domains.

In another particularly preferred embodiment the desired protein (such as a desired heterologous protein) comprises the sequence of transferrin or a variant or fragment thereof. The term "transferrin" as used herein includes all members of the transferrin family (Testa, Proteins of iron metabolism, CRC Press, 2002; Harris & Aisen, Iron carriers and iron proteins, Vol. 5, Physical Bioinorganic Chemistry, VCH, 1991) and their derivatives, such as transferrin, mutant transferrins (Mason et al, 1993, Biochemistry, 32, 5472; Mason et al, 1998, Biochem. J., 330(1), 35), truncated transferrins, transferrin lobes (Mason et al, 1996, Proteins Expr. Purif., 8, 119; Mason et al, 1991, Proteins Expr. Purif., 2, 214), lactoferrin, mutant lactoferrins, truncated lactoferrins, lactoferrin lobes or fusions of any of the above to other peptides, polypeptides or proteins (Shin et al, 1995, Proc. Natl. Acad. Sci. USA, 92, 2820; Ali et al, 1999, J. Biol. Chem., 274, 24066; Mason et al, 2002, Biochemistry, 41, 9448).

The transferrin may, or may not, be human transferrin. The term "human transferrin" is used herein to denote material which is indistinguishable from transferrin derived from a human or which is a variant or fragment thereof. A "variant" includes insertions, deletions and substitutions, either conservative or non-conservative, where such changes do not substantially alter the useful ligand-binding or immunogenic properties of transferrin.

Mutants of transferrin are included in the invention. Such mutants may, or may not, have altered immunogenicity. For example, transferrin mutants may, or may not, display modified (e.g. reduced) glycosylation. The N-linked glycosylation pattern of a transferrin molecule can be modified by adding/removing amino acid glycosylation consensus sequences such as N-X-S/T, at any or all of the N, X, or S/T position. Transferrin mutants may, or may not, be altered in their natural binding to metal ions and/or other proteins, such as transferrin receptor. An example of a transferrin mutant modified in this manner is exemplified below.

We also include naturally-occurring polymorphic variants of human transferrin or human transferrin analogues. Generally, variants or fragments of human transferrin will have at least 5%, 10%, 15%, 20%, 30%, 40% or 50% (preferably at least 80%, 90% or 95%) of human transferrin's ligand binding activity (for example iron-binding), weight for weight. The iron binding activity of transferrin or a test sample can be determined spectrophotometrically by 470nm:280nm absorbance ratios for the proteins in their iron-free and fully iron-loaded states. Reagents should be iron-free unless stated otherwise. Iron can be removed from transferrin or the test sample by dialysis against 0.1 M citrate, 0.1 M acetate, 10mM EDTA pH4.5. Protein should be at approximately 20mg/mL in 100mM HEPES, 10mM NaHCO₃ pH8.0. Measure the 470nm:280nm absorbance ratio of apo-transferrin (Calbiochem, CN Biosciences, Nottingham, UK) diluted in water so that absorbance at 280nm can be accurately determined spectrophotometrically (0% iron binding). Prepare 20mM iron-nitrilotriacetate (FeNTA) solution by dissolving 191mg nitrotriacetic acid in 2mL 1M NaOH, then add 2mL 0.5M ferric chloride. Dilute to 50mL with deionised water. Fully load apo-transferrin with iron (100% iron binding) by adding a sufficient excess of freshly prepared 20mM FeNTA, then dialyse the holo-transferrin preparation completely against 100mM HEPES, 10mM NaHCO₃ pH8.0 to remove remaining FeNTA before measuring the absorbance ratio at 470nm:280nm. Repeat the procedure using test sample, which should initially be free from iron, and compare final ratios to the control.

Additionally, single or multiple heterologous fusions comprising any of the above; or single or multiple heterologous fusions to albumin, transferrin or immunoglobins or a variant or fragment of any of these may, or may not, be used. Such fusions include albumin N-terminal fusions, albumin C-terminal fusions and co-N-terminal and C-terminal albumin fusions as exemplified by WO 01/79271, and transferrin N-terminal fusions, transferrin C-terminal fusions, and co-N-terminal and C-terminal transferrin fusions.

Examples of transferrin fusions are given in US patent applications US2003/0221201 and US2003/0226155, Shin, et al., 1995, Proc Natl Acad Sci USA, 92, 2820, Ali, et al., 1999, J Biol Chem, 274, 24066, Mason, et al., 2002, Biochemistry, 41, 9448.

The skilled person will also appreciate that the open reading frame of any other gene or variant, or part or either, can be utilised as an open reading frame for use with the present invention. For example, the open reading frame may, or may not, encode a protein comprising any sequence, be it a natural protein (including a zymogen), or a variant, or a fragment (which may, or may not, for example, be a domain) of a natural protein; or a totally synthetic protein; or a single or multiple fusion of different proteins (natural or synthetic). Such proteins can be taken, but not exclusively, from the lists provided in WO 01/79258, WO 01/79271, WO 01/79442, WO 01/79443, WO 01/79444 and WO 01/79480, or a variant or fragment thereof. Although these patent applications present the list of proteins in the context of fusion partners for albumin, the present invention is not so limited and, for the purposes of the present invention, any of the proteins listed therein may, or may not, be presented alone or as fusion partners for albumin, the Fc region of immunoglobulin, transferrin, lactoferrin or any other protein or fragment or variant of any of the above, as a desired polypeptide.

The desired protein (such as a desired heterologous protein) may, or may not, be a therapeutically active protein. In other words, it may, or may not, have a recognised medical effect on individuals, such as humans. Many different types of therapeutically active protein are well known in the art.

The desired protein (such as a desired heterologous protein) may, or may not, be a protein that is useful in diagnostic techniques. Many different types of diagnostically useful protein are well known in the art.

The desired protein (such as a desired heterologous protein) may, or may not, be a protein that has no relationship to healthcare. It may, or may not, for example, be a protein that has a utility as an industrial, domestic or nutritional (e.g. as a foodstuff or additive) agent. Many different types of proteins having industrial, domestic and/or nutritional utilities are also well known in the art.

The desired protein (such as a desired heterologous protein) may, or may not, comprise a leader sequence effective to cause secretion in a host cell, such as in a yeast cell.

Numerous natural or artificial polypeptide signal sequences (also called secretion pre regions) have been used or developed for secreting proteins from host cells. The signal sequence directs the nascent protein towards the machinery of the cell that exports proteins from the cell into the surrounding medium or, in some cases, into the periplasmic space. The signal sequence is usually, although not necessarily, located at the N-terminus of the primary translation product and is generally, although not necessarily, cleaved off the protein during the secretion process, to yield the "mature" protein.

In the case of some proteins the entity that is initially secreted, after the removal of the signal sequence, includes additional amino acids at its N-terminus called a "pro" sequence, the intermediate entity being called a "pro-protein". These pro sequences may, or may not, assist the final protein to fold and become functional, and are usually then cleaved off. In other instances, the pro region simply provides a cleavage site for an enzyme to cleave off the pre-pro region and is not known to have another function.

The pro sequence can be removed either during the secretion of the protein from the cell or after export from the cell into the surrounding medium or periplasmic space.

Polypeptide sequences which direct the secretion of proteins, whether they resemble signal (i.e. pre) sequences or pre-pro secretion sequences, are referred to as leader sequences. The secretion of proteins is a dynamic process involving translation, translocation and post-translational processing, and one or more of these steps may not necessarily be completed before another is either initiated or completed.

For production of proteins in eucaryotic species such as the yeasts *Saccharomyces cerevisiae*, *Zygosaccharomyces* species, *Kluyveromyces lactis* and *Pichia pastoris*, known leader sequences include those from the *S. cerevisiae* acid phosphatase protein (Pho5p) (see EP 366 400), the invertase protein (Suc2p) (see Smith et al. (1985) Science, 229, 1219-1224) and heat-shock protein-150 (Hsp150p) (see WO 95/33833). Additionally, leader sequences from the *S. cerevisiae* mating factor alpha-1 protein (MFα-1) and from the human lysozyme and human serum albumin (HSA) protein have been used, the latter having been used especially, although not exclusively, for secreting human albumin. WO 90/01063 discloses a fusion of the MFα-1 and HSA leader sequences, which advantageously reduces the production of a contaminating fragment of human albumin relative to the use of the MFα-1 leader sequence. Modified leader sequences are also disclosed in WO 2004/009819 and in the examples of this application; the reader will appreciate that those leader sequences can be used with proteins other than transferrin. In addition, the natural transferrin leader sequence may, or may not, be used to direct secretion of transferrin and other heterologous proteins.

Where a chaperone that is recombinantly expressed according to the present invention is protein disulphide isomerase, then optionally the desired protein (such as a desired heterologous protein) may, or may not, comprise disulphide bonds in its mature form. Any disulphide bonds may, or may not, be intramolecular and/or intermolecular.

The desired protein (such as a desired heterologous protein) may, or may not, be a commercially useful protein, such as a therapeutically, diagnostically, industrially, domestically or nutritionally useful protein. Some proteins, such as heterologously expressed proteins, are intended to interact with the cell in which they are expressed in order to bring about a beneficial effect on the cell's activities. These proteins are not, in their own right, commercially useful. Commercially useful proteins are proteins that have a utility *ex vivo* of the cell in which they are expressed. Nevertheless, the skilled reader will appreciate that a commercially useful protein may, or may not, also have a biological effect on the host cell expressing it (such as a heterologous protein), but that that effect is not the main or sole reason for expressing the protein therein.

Commercially useful proteins may include proteins that are useful as metabolites or antibiotics, and the like.

In one embodiment it is preferred that the desired protein (such as a desired heterologous protein) is not β-lactamase. In another embodiment it is preferred that the desired protein (such as a desired heterologous protein) is not antistasin. However, the reader will appreciate that neither of these provisos exclude genes encoding either β-lactamase or antistasin from being present in a host cell or on a plasmid of the invention, merely that the gene encoding the desired protein (such as a desired heterologous protein) encodes a protein other than β-lactamase and/or antistasin.

Plasmids useful in the practice of the present invention can, unless specified otherwise, be any type of plasmid. For the purposes of the present invention, references to "plasmids" may, or may not, also include a reference to other types of vectors. It may be appropriate to choose a suitable plasmid based on the host cell system in which it will be used.

Many plasmids and other vectors are known for the transformation of various expression systems, including systems employing: bacteria (e.g. *Bacillus subtilis* or *Escherichia coli*) transformed with, for example, recombinant bacteriophage, plasmid or cosmid DNA expression vectors; yeasts (e.g. *Saccharomyces cerevisiae* or *Pichia pastoris*) transformed with, for example, yeast expression vectors; insect cell systems transformed with, for example, viral expression vectors (e.g. baculovirus); plant cell systems transfected with, for example viral or bacterial expression vectors; animal cell systems, either in cell culture, transgenic or as gene therapy, transfected with, for example, adenovirus expression vectors.

Typical procaryotic vector plasmids are: pUC18, pUC19, pBR322 and pBR329 available from Biorad Laboratories (Richmond, CA, USA); p*Trc*99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Useful yeast plasmid vectors include the 2µm-family plasmids (as described above), as well as pRS403-406 and pRS413-416 which are generally available from Stratagene Cloning Systems (La Jolla, CA 92037, USA). Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps). Other YIps and YCps plasmids may also be used.

Plasmids for use in any aspect of the present invention, including those described in the text relating the 'main aspect' of the invention, can be prepared by modifying plasmids, such as 2µm-family plasmids, known in the art by inserting the required sequences (for example, one or more genes encoding chaperones and/or one or more genes encoding a heterologous protein) using techniques well known in the art such as are described in by Sambrook et al., Molecular Cloning: A Laboratory Manual, 2001, 3rd edition. For example, one such method involves ligation via cohesive ends. Compatible cohesive ends can be generated on a DNA fragment for insertion and plasmid by the action of suitable restriction enzymes. These ends will rapidly anneal through complementary base pairing and remaining nicks can be closed by the action of DNA ligase.

A further method uses synthetic double stranded oligonucleotide linkers and adaptors. DNA fragments with blunt ends are generated by bacteriophage T4 DNA polymerase or *E.coli* DNA polymerase I which remove protruding 3' termini and fill in recessed 3' ends. Synthetic linkers and pieces of blunt-ended double-stranded DNA, which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end. Alternatively a DNA fragment or DNA fragments can be ligated together by the action of DNA ligase in the presence or absence of one or more synthetic double stranded oligonucleotides optionally containing cohesive ends.

Synthetic linkers containing a variety of restriction endonuclease sites are commercially available from a number of sources including Sigma-Genosys Ltd, London Road, Pampisford, Cambridge, United Kingdom.

Appropriate insertion sites in plasmids (such as 2µm-family plasmids) include, but are not limited to, those discussed above.

### Host cells

The present invention also provides a host cell comprising recombinant genes and/or plasmid according to any aspect of the present invention, including those described in the text relating the 'main aspect' of the invention. The host cell may be any type of cell. Many suitable host cell expression systems are known, including bacteria (for example *E. coli* and *Bacillus subtilis*), yeasts (for example *Saccharomyces cerevisiae*, *Pichia pastoris* and *Kluyveromyces lactis*), filamentous fungi (for example *Aspergillus*), plant cells, whole plants, animal cells and insect cells. Bacterial and yeast host cells may, or may not, be preferred. Bacterial host cells may be useful for cloning purposes. Yeast host cells may be useful for expression of genes present in the plasmid.

In one embodiment the host cell may, or may not, be a yeast cell, such as a member of the *Saccharomyces*, *Kluyveromyces*, *Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces,* or *Pichia* genus. Yeast such *Saccharomyces cerevisiae*, *Kluyveromyces lactis*, *Pichia pastoris*, *Pichia membranaefaciens, Zygosaccharomyces rouxii*, *Zygosaccharomyces bailii, Zygosaccharomyces fermentati*, *Kluyveromyces drosphilarum, Pichia methanolica, Hansenula polymorpha* (also known as *Pichia angusta*)*, Arxula adeninivorans, Yarrowia lipolytica, Candida boidinii Candida utilis, Schizosaccharomyces pombe* may, or may not, be preferred. Other suitable yeast may, or may not, include *Debaryomyces hansenii, Xanthophyllomyces dendrorhous, Geotrichum candidum, Ashbya gossypii, Hortaea werneckii, Schwanniomyces occidentalis, Trichosporon domesticum, and*/*or Xanthophyllomyces dendrorhous*,
It is may, or may not, be particularly advantageous to use a yeast deficient in one or more protein mannosyl transferases involved in O-glycosylation of proteins, for instance by disruption of the gene coding sequence, as discussed in WO 2004/083245.

In another embodiment the host cell may, or may not, be an animal cell. For example, the animal cell may, or may not, be a mammalian cell, such as a human cell type.

The host cell type may, or may not, be selected for compatibility with a plasmid type being used. Plasmids obtained from one yeast type can be maintained in other yeast types (Irie et al, 1991, Gene, 108(1), 139-144; Irie et al, 1991, Mol. Gen. Genet., 225(2), 257-265). For example, pSR1 from *Zygosaccharomyces rouxii* can be maintained in *Saccharomyces cerevisiae.* Optionally, the host cell is compatible with a 2µm-family plasmid (see above for a full description of the following plasmids). For example, where the plasmid is based on pSR1, pSB3 or pSB4 then a suitable yeast cell is *Zygosaccharomyces rouxii*; where the plasmid is based on pSB1 or pSB2 then a suitable yeast cell is *Zygosaccharomyces bailli*; where the plasmid is based on pSM1 then a suitable yeast cell is *Zygosaccharomyces fermentati*; where the plasmid is based on pKD1 then a suitable yeast cell is *Kluyveromyces drosophilarum*; where the plasmid is based on pPM1 then a suitable yeast cell is *Pichia membranaefaciens*; where the plasmid is based on the 2µm plasmid then a suitable yeast cell is *Saccharomyces cerevisiae* or *Saccharomyces carlsbergensis.* It is particularly preferred that the plasmid is based on the 2µm plasmid and the yeast cell is *Saccharomyces cerevisiae.*

A 2µm-family plasmid of the invention can be said to be "based on" a naturally occurring plasmid if it comprises one, two or preferably three of the genes *FLP, REP1* and *REP2* having sequences derived from that naturally occurring plasmid.

It may, or may not, be particularly advantageous to use a yeast deficient in one or more protein mannosyl transferases involved in O-glycosylation of proteins, for instance by disruption of the gene coding sequence.

Recombinantly expressed proteins can be subject to undesirable post-translational modifications by the producing host cell. For example, the albumin protein sequence does not contain any sites for N-linked glycosylation and has not been reported to be modified, in nature, by O-linked glycosylation. However, it has been found that recombinant human albumin ("rHA") produced in a number of yeast species can be modified by O-linked glycosylation, generally involving mannose. The mannosylated albumin is able to bind to the lectin Concanavalin A. The amount of mannosylated albumin produced by the yeast can be reduced by using a yeast strain deficient in one or more of the *PMT* genes (WO 94/04687). The most convenient way of achieving this is to create a yeast which has a defect in its genome such that a reduced level of one of the Pmt proteins is produced. For example, there may, or may not, be a deletion, insertion or transposition in the coding sequence or the regulatory regions (or in another gene regulating the expression of one of the *PMT* genes) such that little or no Pmt protein is produced. Alternatively, the yeast could be transformed to produce an anti-Pmt agent, such as an anti-Pmt antibody.

If a yeast other than *S. cerevisiae* is used, disruption of one or more of the genes equivalent to the *PMT* genes of *S. cerevisiae* is also beneficial, e.g. in *Pichia pastoris* or *Kluyveromyces lactis.* The sequence of *PMT1* (or any other *PMT* gene) isolated from *S. cerevisiae* may, or may not, be used for the identification or disruption of genes encoding similar enzymatic activities in other fungal species. The cloning of the *PMT1* homologue of *Kluyveromyces lactis* is described in WO 94/04687.

The yeast may, or may not, have a deletion of the *HSP150* and/or *YAP3* genes as taught respectively in WO 95/33833 and WO 95/23857.

A plasmid as defined above, may, or may not, be introduced into a host through standard techniques. With regard to transformation of procaryotic host cells, see, for example, Cohen et al (1972) Proc. Natl. Acad. Sci. USA 69, 2110 and Sambrook et al (2001) Molecular Cloning, A Laboratory Manual, 3rd Ed. Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. Methods for the transformation of *S. cerevisiae* are taught generally in EP 251 744, EP 258 067 and WO 90/01063. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming cells and is well known in the art for transforming yeast cell, bacterial cells and vertebrate cells. Methods for transformation of yeast by electroporation are disclosed in Becker & Guarente (1990) Methods Enzymol. 194, 182.

Generally, where a plasmid is used, it will transform not all of the hosts and it will therefore be necessary to select for transformed host cells. Thus, a plasmid may, or may not, comprise a selectable marker, including but not limited to bacterial selectable marker and/or a yeast selectable marker. A typical bacterial selectable marker is the β-lactamase gene although many others are known in the art. Yeast selectable marker include *LEU2, TRP1, HIS3, HIS4, URA3, URA5*, *SFA1, ADE2, MET15, LYS5, LYS2, ILV2, FBA1, PSE1, PDI1* and *PGK1.* Those skilled in the art will appreciate that any gene whose chromosomal deletion or inactivation results in an inviable host, so called "essential" genes, can be used as a selective marker if a functional gene is provided on the plasmid, as demonstrated for *PGK1* in *a pgk1* yeast strain (Piper and Curran, 1990, Curr. Genet. 17, 119). Suitable "essential" genes can be found within the Stanford Genome Database (SGD), http:://db.yeastgenome.org). Any "essential" gene product (e.g. a product of one of the *PDI1*, *PSE1*, *PGK1* or *FBA1* genes, and others described elsewhere in this application) which, when deleted or inactivated, does not result in an auxotrophic (biosynthetic) requirement, can be used as a selectable marker on a plasmid in a host cell that, in the absence of the plasmid, is unable to produce that gene product, to achieve increased plasmid stability without the disadvantage of requiring the cell to be cultured under specific selective conditions. By "auxotrophic (biosynthetic) requirement" we include a deficiency which can be complemented by nutrient and other additions or modifications to the growth medium. Cells unable to express functional Pgk1p or Fbalp can, however, be complemented by certain additions to growth media and such gene products may not be preferred "essential proteins" according to the present invention. Therefore, preferred "essential marker genes" in the context of the present invention are those that, when deleted or inactivated in a host cell, result in a deficiency which cannot be complemented by any additions or modifications to the growth medium, expect where those additions or modifications are, for example, a polynucleotide, that can restore the ability of the host cell to express the product of the "essential" marker gene, or the product of the "essential" marker gene itself.

Accordingly, a plasmid as provided by, for use in a method of, or comprised in a host cell of, the present invention may, or may not, comprise more than one selectable marker.

One selection technique involves incorporating into the expression vector a DNA sequence marker, with any necessary control elements, that codes for a selectable trait in the transformed cell. These markers include dihydrofolate reductase, G418 or neomycin resistance for eucaryotic cell culture, and tetracyclin, kanamycin or ampicillin (i.e. β-lactamase) resistance genes for culturing in *E.coli* and other bacteria. Alternatively, the gene for such selectable trait can be on another vector, which is used to co-transform the desired host cell.

Another method of identifying successfully transformed cells involves growing the cells resulting from the introduction of the plasmid, optionally to allow the expression of a recombinant polypeptide (i.e. where a polypeptide which is encoded by a polynucleotide sequence on the plasmid and is not naturally produced by the host). Cells can be harvested and lysed and their DNA or RNA content examined for the presence of the recombinant sequence using a method such as that described by Southern (1975) J. Mol. Biol. 98, 503 or Berent et al (1985) Biotech. 3, 208 or other methods of DNA and RNA analysis common in the art. Alternatively, the presence of a polypeptide in the supernatant of a culture of a transformed cell can be detected using antibodies.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, optionally a monoclonal (clonally homogeneous) culture, or a culture derived from a monoclonal culture, in a nutrient medium. Alternatively, transformed cells may, or may not, represent an industrially/commercially or pharmaceutically useful product and can be used without further purification or can be purified from a culture medium and optionally formulated with a carrier or diluent in a manner appropriate to their intended industrial/commercial or pharmaceutical use, and optionally packaged and presented in a manner suitable for that use. For example, whole cells could be immobilised; or used to spray a cell culture directly on to/into a process, crop or other desired target. Similarly, whole cell, such as yeast cells can be used as capsules for a huge variety of applications, such as fragrances, flavours and pharmaceuticals.

Transformed host cells may, or may not, be cultured for a sufficient time and under appropriate conditions known to those skilled in the art, and in view of the teachings disclosed herein, to permit the expression of one or more recombinant chaperones and a desired protein (such as a desired heterologous protein).

The culture medium may, or may not, be non-selective or may, or may not place a selective pressure on the maintenance of the plasmid.

The thus produced desired protein (such as a desired heterologous protein) may, or may not, be present intracellularly or, if secreted, in the culture medium and/or periplasmic space of the host cell.

### Protein Recovery and Formulation

The step of purifying the thus expressed desired protein (such as a desired heterologous protein) from the cultured host cell or the culture medium optionally comprises cell immobilization, cell separation and/or cell breakage, but always comprises at least one other purification step different from the step or steps of cell immobilization, separation and/or breakage.

Cell immobilization techniques, such as encasing the cells using calcium alginate bead, are well known in the art. Similarly, cell separation techniques, such as centrifugation, filtration (e.g. cross-flow filtration, expanded bed chromatography and the like are well known in the art. Likewise, methods of cell breakage, including beadmilling, sonication, enzymatic exposure and the like are well known in the art.

The at least one other purification step may be any other step suitable for protein purification known in the art. For example purification techniques for the recovery of recombinantly expressed albumin have been disclosed in: WO 92/04367, removal of matrix-derived dye; EP 464 590, removal of yeast-derived colorants; EP 319 067, alkaline precipitation and subsequent application of the albumin to a lipophilic phase; and WO 96/37515, US 5 728 553 and WO 00/44772, which describe complete purification processes.

Proteins other than albumin may be purified from the culture medium by any technique that has been found to be useful for purifying such proteins.

Suitable methods include ammonium sulphate or ethanol precipitation, acid or solvent extraction, anion or cation exchange chromatography, phosphocellulose chromatography, hydrophobic interaction chromatography, affinity chromatography, hydroxylapatite chromatography, lectin chromatography, concentration, dilution, pH adjustment, diafiltration, ultrafiltration, high performance liquid chromatography ("HPLC"), reverse phase HPLC, conductivity adjustment and the like.

In one embodiment, any one or more of the above mentioned techniques may, or may not, be used to further purify the thus isolated protein to a commercially or industrially acceptable level of purity. By commercially or industrially acceptable level of purity, we include the provision of the protein at a concentration of at least 0.01 g.L⁻¹, 0.02 g.L⁻¹, 0.03 g.L⁻¹, 0.04 g.L⁻¹, 0.05 g.L⁻¹, 0.06 g.L⁻¹, 0.07 g.L⁻¹, 0.08 g.L⁻¹, 0.09 g.L⁻¹, 0.1 g.L⁻¹, 0.2 g.L⁻¹, 0.3 g.L⁻¹, 0.4 g.L⁻¹, 0.5 g.L⁻¹, 0.6 g.L⁻¹, 0.7 g.L⁻¹, 0.8 g.L⁻¹, 0.9 g.L⁻¹, 1 g.L⁻¹, 2 g.L⁻¹, 3 g.L⁻¹, 4 g.L⁻¹, 5 g.L⁻¹, 6 g.L⁻¹, 7 g.L⁻¹, 8 g.L⁻¹, 9 g.L⁻¹, 10 g.L⁻¹, 15 g.L⁻¹, 20 g.L⁻¹, 25 g.L⁻¹, 30 g.L⁻¹, 40 g.L⁻¹,50 g.L⁻¹, 60 g.L⁻¹, 70 g.L⁻¹, 80 g.L⁻¹, 90 g.L⁻¹, 100 g.L⁻¹, 150 g.L⁻¹, 200 g.L⁻¹, 250 g.L⁻¹, 300 g.L⁻¹, 350 g.L⁻¹, 400 g.L⁻¹, 500 g.L⁻¹, 600 g.L⁻¹, 700 g.L⁻¹, 800 g.L⁻¹, 900 g.L⁻¹, 1000 g.L⁻¹, or more.

It is preferred that the desired protein (such as a desired heterologous protein) is purified to achieve a pharmaceutically acceptable level of purity. A protein has a pharmaceutically acceptable level of purity if it is essentially pyrogen free and can be administered in a pharmaceutically efficacious amount without causing medical effects not associated with the activity of the protein.

The resulting desired protein (such as a desired heterologous protein) may, or may not, be used for any of its known utilities, which, in the case of albumin, include i.v. administration to patients to treat severe burns, shock and blood loss, supplementing culture media, and as an excipient in formulations of other proteins.

Although it is possible for a therapeutically, diagnostically, industrially, domestically or nutritionally useful desired protein (such as a desired heterologous protein) obtained by a process of the of the invention to be presented or administered alone, it is preferable to present it as a formulation (such as a pharmaceutical formulation, particularly in the case of therapeutically and/or diagnostically useful proteins), together with one or more acceptable carriers or diluents. The carrier(s) or diluent(s) must be "acceptable" in the sense of being compatible with the desired protein and, where the formulation is intended for administration to a recipient, then not deleterious to the recipient thereof Typically, the carriers or diluents will be water or saline which will be sterile and pyrogen free.

Optionally the thus formulated protein will be presented in a unit dosage form, such as in the form of a tablet, capsule, injectable solution or the like.

We have also demonstrated that a plasmid-borne gene encoding a protein comprising the sequence of an "essential" protein can be used to stably maintain the plasmid in a host cell that, in the absence of the plasmid, does not produce the "essential" protein. This has the advantage of ensuring the genetic stability of the organism in the chosen culture conditions, and thereby improving the reproducibility and reliability of individual cultures, and furthermore enables prolonged culture without reduced productivity due to plasmid loss.

A preferred "essential" protein is a chaperone which may or may not provide the further advantage that, as well as acting as a selectable marker to increase plasmid stability, its expression simultaneously increases the expression of one or more desired proteins, such as a heterologous protein encoded by a recombinant gene, within the host cell. This system is advantageous because it allows the user to minimise the number of recombinant genes that need to be carried by a plasmid. For example, typical prior art plasmids carry marker genes (such as those as described above) that enable the plasmid to be stably maintained during host cell culturing process. Such marker genes need to be retained on the plasmid in addition to any further genes that are required to achieve a desired effect. However, the ability of plasmids to incorporate exogenous DNA sequences is limited and it is therefore advantageous to minimise the number of sequence insertions required to achieve a desired effect. Moreover, some marker genes (such as auxotrophic marker genes) require the culturing process to be conducted under specific conditions in order to obtain the effect of the marker gene. Such specific conditions may not be optimal for cell growth or protein production, or may require inefficient or unduly expensive growth systems to be used.

Thus, it is possible to use a recombinant gene that encodes a protein comprising the sequence of an "essential" protein as a plasmid-borne gene to increase plasmid stability, where the plasmid is present within a cell that, in the absence of the plasmid, is unable to produce the "essential" protein. It will be appreciated that the question of whether or not a protein is "essential" will depend on the system in which it is used; it is possible that a protein that is not "essential" in one host organism might become "essential" when one or more other genes is deleted, disrupted, inactivated, modified or affected in that same host, and thereby be used as an "essential" plasmid-borne gene, as described above; likewise whether or not a protein is "essential" may depend on certain physical conditions, such as pH, temperature and/or oxygen levels under which the host cell is cultured..

It is preferred that the "essential protein" is one that, when its encoding gene(s) in a host cell are deleted or inactivated, does not result in the host cell developing an auxotrophic (biosynthetic) requirement. By "auxotrophic (biosynthetic) requirement" we include a deficiency that can be complemented by additions or modifications to the growth medium, in particular additions of, or modifications to, the nutrient composition of the growth medium. Thus, the "essential protein" would be an auxotrophic marker protein if the inactivation of its encoding gene, in a host cell, resulted in the production of an auxotrophic mutant, i.e. a mutant organism that, in order to grow and survive, requires a particular additional nutrient that the normal (unmutated strain) does not - it is preferred that the "essential protein" is not an auxotrophic marker protein. Therefore, an "essential marker gene" which encodes an "essential protein", in the context of the present invention is one that, when deleted or inactivated in a host cell, results in a deficiency which cannot be complemented by additions or modifications, typically nutrient additions or modifications, to the growth medium, expect where those additions or modifications are, for example, a polynucleotide, that can restore the ability of the host cell to express the product of the "essential" marker gene, or the product of the "essential" marker gene itself. In other words, it may, or may not, be preferred if the "essential protein" is not a protein that, in nature, is involved in the metabolic conversion of nutrients by a host cell. The advantage of this system is that the "essential marker gene" can be used as a selectable marker on a plasmid in host cell that, in the absence of the plasmid, is unable to produce that gene product, to achieve increased plasmid stability without the disadvantage of requiring the cell to be cultured under specific selective (e.g. selective nutrient) conditions. Therefore, the host cell can be cultured under conditions that do not have to be adapted for any particular marker gene, without losing plasmid stability. For example, host cells produced using this system can be cultured in non-selective media such as complex or rich media, and under non-selective growth conditions (e.g. such as pH, temperature and/or oxygen levels), which may be more economical, and/or more supportive growth media/conditions, than the minimal media and/or specifically adapted growth conditions that are commonly used to give auxotrophic, and other, marker genes their effect.

The cell may, or may not, for example, have the endogenous copy (or copies) of the gene (or genes) encoding the "essential" protein deleted or otherwise inactivated.

It is particularly preferred if the "essential protein" is an "essential" chaperone, as this can provide the dual advantage of improving plasmid stability without the need for selective growth conditions and increasing the production of desired proteins, such as endogenously encoded or a heterologous proteins, in the host cell. This system also has the advantage that it minimises the number of recombinant genes that need to be carried by the plasmid if one chooses to use over-expression of an "essential" chaperone to increase protein production by the host cell.

Preferred "essential proteins" for use in this aspect of the invention include the "essential" chaperones encoded by the genes *PDI1* and *PSE1* which, when the endogenous gene(s) encoding these proteins are deleted or inactivated in a host cell, do not result in the host cell developing an auxotrophic (biosynthetic) requirement.

Preferred "essential" chaperones are eucaryotic chaperones, especially preferred "essential" chaperones are yeast chaperones, including chaperones comprising the sequence of proteins encoded by a gene selected from *CCT2*, *CCT3*, *CCT4*, *CCT5*, *CCT6*, *CCT7*, *CCT8*, *CNS1*, *ERO1* (in the absence of diamide), *HSP10*, *HSP60*, *PDI1*, *CDC37*, *KAR2*, *MGE1*, *MRS11*, *NOB1*, *SSC1*, *PSE1*, *TIM9*, *PAM18* and *TCP1.*

It is noted that a host cell that is mutated to inactive *ERO1* can be complemented by growth in the presence of the oxidant diamide (Frand & Kaiser, 1998, Molecular Cell, 1, 161-170), but diamide is not a "nutrient" addition of the type discussed above in respect of auxotrophic mutations. Diamide is not a commonly used component of growth media and an *ERO1* mutant that is transformed with a plasmid comprising the *ERO1* gene can be grown in rich media without loss of plasmid stability.

Accordingly, in a sixth aspect, the present invention also provides a host cell according to the invention comprising a plasmid (such as a plasmid as defined above by any of the previous aspects of the invention, including those described in the text relating the 'main aspect' of the invention), the plasmid comprising a gene that encodes an "essential" protein, such as a chaperone, wherein, in the absence of the plasmid, the host cell is unable to produce the "essential" protein. Preferably, in the absence of the plasmid, the host cell is inviable. Typically the host cell has been genetically modified to render it unable to produce a functional copy of the "essential" protein from a chromosomally-encoded (or otherwise endogenous) gene. The host cell may, or may not, further comprise a recombinant gene encoding a heterologous protein, such as those described above in respect of earlier aspects of the invention, including those described in the text relating the 'main aspect' of the invention.

The is also provided, in a seventh aspect, a plasmid comprising, as the sole yeast selectable marker, optionally as the sole selectable marker, a gene encoding an "essential" protein, such as an "essential" chaperone. The plasmid may, or may not, further comprise a gene encoding a heterologous protein. The plasmid may, or may not, be a 2µm-family plasmid.

The present invention also provides, in an eighth aspect, a method according to the invention for producing a desired protein (such as a desired heterologous protein) comprising the steps of: providing a host cell comprising a plasmid, the plasmid comprising a gene that encodes an "essential" protein, such as a chaperone, wherein, in the absence of the plasmid, the host cell is unable to produce the "essential" protein and wherein the host cell further comprises a gene, such as a recombinant gene, encoding a desired protein (such as a desired heterologous protein); culturing the host cell in a culture medium under conditions that allow the expression of the "essential" protein and the desired protein; and optionally purifying the thus expressed desired protein from the cultured host cell or the culture medium; and further optionally, lyophilising the thus purified protein. Thus, a host cell used in this method may, or may not, be a host cell according to the sixth aspect of the invention and/or the host call may, or may not, be transformed with a plasmid according to the seventh aspect.

The method may, or may not, further comprise the step of formulating the purified desired protein (such as a desired heterologous protein) with a carrier or diluent and optionally presenting the thus formulated protein in a unit dosage form, in the manner discussed above.

In one preferred embodiment, the step of "culturing the host cell in a culture medium under conditions that allow the expression of the "essential" protein and the desired protein" involves culturing the host cell in medium that is not specifically adapted to be selective for the presence of any genes on the plasmid, other than for the presence of the gene encoding the "essential" protein. Thus, in one embodiment, the step of culturing the host cells may, or may not, be performed in non-selective media, such as complex or rich media and/or under conditions (such as pH, temperature and/or oxygen levels) that are not specifically adapted to select for the presence of the "essential" protein. A medium can be described as non-selective for the purposes of the present situation if it is not specifically adapted to deprive the host cell of a product, typically a nutrient product, that is ordinarily provided to maximise, or otherwise allow, the growth of host cells that have not been modified to prevent the expression of the "essential" protein. For example, a medium (the "test medium") may be a non-selective medium, for the purposes of the present invention if, when one compares plasmid stability in a first host cell type grown in the "test medium" to plasmid stability in a second cell type grown in the "test medium" when each cell type is grown for 5, 10, 15, 20, 25 or 30 generations in the "test medium", wherein -
(i) the first host cell type is a host cell according to the sixth aspect of the present invention;
(ii) the second host cell type is a host cell according to the sixth aspect of the present invention except that it has been modified to restore the ability of the host cell to produce the "essential" protein in the absence of the plasmid (which is not to say that the second host cell type does not contain a plasmid encoding the "essential" protein, just that it can produce the "essential" protein even when the plasmid is not present);
then the plasmid stability observed in the second cell type is less than 99%, 98%, 95%, 90%, 80%, 70%, 60%, 50%, 40%, 30%, 20%, 15%, 10%, 5%, 4%, 3%, 2%, 1% or less of the plasmid stability observed in the first cell type. Thus, in this embodiment, and despite being grown under non-selective conditions, it is preferable for method of the eighth aspect of the present invention to produce host cells which display substantially 100% plasmid stability after 5, 10, 15, 20, 25 or 30 generations.

A ninth aspect also provides for the use of a polynucleotide that encodes an "essential" protein (as defined above) to increase the stability of a plasmid in a host cell, particularly under non-selective conditions, by integration of the polynucleotide into the plasmid to produce a modified plasmid, wherein the host cell is unable to produce the "essential" protein in the absence of the modified plasmid. The increase in stability may, or may not, be at least 1% (i.e. 1.01 times), 2%, 3%, 4%, 5%, 6%, 7%, 8%, 9%, 10%, 20%, 30%, 40%, 50%, 60%, 70%, 80%, 90%, 100% (i.e. 2-fold), 3-fold, 4-fold, 5-fold, 6-fold s, 7-fold, 8-fold, 9-fold, 10-fold or more greater than the level of stability of the unmodified plasmid in the same host cell that has been modified to produce the "essential" protein, when grown for at least five generations under non-selective conditions.

In one embodiment of the ninth aspect, the plasmid may, or may not, additionally comprise a gene that encodes a further desired heterologous protein, such as defined above in respect of earlier aspects of the present invention, including those described in the text relating the 'main aspect' of the invention. In that case, the use may, or may not, be to improve the productivity of the desired heterologous protein, such as when the host cell comprising the plasmid is grown under non-selective conditions.

In one preferred embodiment, the "essential" protein is a chaperone and may, or may not, be used to simultaneously increase the stability of a plasmid in the host cell and increase the ability of the host cell to produce a desired protein product. The desired protein product may, or may not, be an endogenously encoded protein or may, or may not, be a heterologous protein as defined by the earlier aspects of the invention, including those described in the text relating the 'main aspect' of the invention. Where the protein product is a heterologous protein, it may, or may not, be encoded by a recombinant gene that has been integrated into the chromosome of the host cell, or by a gene that is present on a plasmid in the host cell, such as the modified plasmid comprising the polynucleotide that encodes the "essential" protein as defined above.

We have also found that the effects of recombinantly-provided chaperones according to the other embodiments of the present invention can be modulated by modifying the promoters that control the expression levels of the chaperone(s).

Surprisingly we have found that, in some cases, shorter promoters result in increased expression of a desired protein. Without being bound by theory we believe that this is because the expression of a recombinant chaperone in host cells that already express desired proteins at high levels can cause the cells to overload themselves with desired protein (such as a desired heterologous protein), thereby achieving little or no overall increase in production of the desired protein. In those cases, it may, or may not, be beneficial to provide recombinant chaperone genes with truncated promoters.

Accordingly, in a tenth aspect there is provided a polynucleotide (such as a plasmid as defined above) comprising the sequence of a promoter operably connected to a coding sequence encoding a chaperone (such as those described above), for use in increasing the expression of a desired protein (such as a desired heterologous protein), such as those described above, in a host cell (such as those described above) by expression of the polynucleotide sequence within the host cell, wherein the promoter is characterised in that it achieves a modified, such as a higher or lower, level of expression of the chaperone than would be achieved if the coding sequence were to be operably connected to its naturally occurring promoter.

The present invention also provides, in a eleventh aspect, a method of the invention for producing a desired protein (such as a desired heterologous protein) comprising the steps of: providing a host cell comprising a recombinant gene that comprising the sequence of promoter operably connected to a coding sequence encoding a chaperone, the promoter being characterised in that it achieves a lower level of expression of the chaperone than would be achieved if the coding sequence were to be operably connected to its naturally occurring promoter, and the host cell further comprising a gene, such as a recombinant gene, encoding a desired protein (such as a desired heterologous protein); culturing the host cell under conditions that allow the expression of the chaperone and the desired protein; and optionally purifying the thus expressed desired protein from the cultured host cell or the culture medium; and further optionally, lyophilising the thus purified protein; and optionally further formulating the purified desired protein with a carrier or diluent; and optionally presenting the thus formulated protein in a unit dosage form, in the manner discussed above.

As is apparent from the examples of the present application, the combination of recombinantly expressed PDI and transferrin-based proteins provides a surprisingly high level of transferrin expression. For example, transferrin expression in a system that includes a chromosomally encoded recombinant PDI gene provided a 2-fold increase (compared to a control in which there is no chromosomally encoded recombinant PDI gene). This increase was 5-times greater than an equivalent system comprising a recombinant gene encoding human albumin in place of the recombinant transferrin gene.

The host may be any cell type, such as a procaryotic cell (e.g. bacterial cells such as *E. coli*) or a eucaryotic cell. Preferred eucaryotic cells include fungal cells, such as yeast cells, and mammalian cells. Exemplary yeast cells are discussed above. Exemplary mammalian cells include human cells.

Host cells as described above can be cultured to produce recombinant transferrin-based proteins. The thus produced transferrin-based proteins can be isolated from the culture and purified, optionally to a pharmaceutically acceptable level of purity, for example using techniques known in the art and/or as set out above. Purified transferrin-based proteins may, or may not, be formulated with a pharmaceutically acceptable carrier or diluent and may, or may not, be presented in unit dosage form.

The inventors also disclose:
(1) A method for producing a desired protein (such as a desired heterologous protein) comprising:
   (a) providing a host cell comprising a first recombinant gene encoding a protein comprising the sequence of a first chaperone protein, a second recombinant gene encoding a protein comprising the sequence of a second chaperone protein and a third gene, such a third recombinant gene, encoding a desired protein (such as a desired heterologous protein), wherein the first and second chaperones are different; and
   (b) culturing the host cell in a culture medium to obtain expression of the first, second and third genes.
(2) The method of (1) further comprising the step of purifying the thus expressed desired protein (such as a desired heterologous protein) from the cultured host cell or the culture medium.
(3) The method of (2) further comprising the step of lyophilising the thus purified protein.
(4) The method of (2) or (3) further comprising the step of formulating the purified desired protein (such as a desired heterologous protein) with a carrier or diluent and optionally presenting the thus formulated protein in a unit dosage form.
(5) A method according to any of (1) to (4) wherein one or both of the first or second chaperone proteins has a sequence of a fungal chaperone (optionally a yeast chaperone) or a mammalian chaperone (optionally a human chaperone).
(6) A method according to any one of (1) to (5) wherein one or both of the first and second chaperones each individually comprise the sequence of a protein encoded by any one of *AHA1*, *CCT2*, *CCT3*, *CCT4*, *CCT5*, *CCT6*, *CCT7*, *CCT8*, *CNS1*, *CPR3*, *CPR6*, *EPS1*, *ERO1*, *EUG1*, *FMO1*, *HCH1*, *HSP10*, *HSP12*, *HSP104*, *HSP26*, *HSP30*, *HSP42*, *HSP60*, *HSP78*, *HSP82*, *JEM1*, *MDJ1*, *MDJ2*, *MPD1*, *MPD2*, *PDI1*, *PFD1*, *ABC1*, *APJ1*, *ATP11*, *ATP12*, *BTT1*, *CDC37*, *CPR7*, *HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UBI4*, *ORM1*, *ORM2*, *PER1*, *PTC2*, *PSE1*, *HAC1* or truncated intronless *HAC1*, *TIM9*, *PAM18* or *TCP1* or a variant or fragment of any one of these.
(7) A method according to any one of (1) to (6) wherein the first chaperone is protein disulphide isomerase.
(8) A method according to any one of (1) to (7) wherein the second chaperone is Orm2p or a variant or fragment thereof.
(9) A method according to any one of (1) to (8) wherein at least one, preferably both, of the first or second chaperones is encoded by a chromosomally integrated recombinant gene.
(10) A method according to any one of (1) to (9) wherein at least one, preferably both, of the first or second chaperones is encoded by a gene on a plasmid.
(11) A method according to any of (1) to (10) wherein the third gene which encodes the desired protein (such as a desired heterologous protein) is integrated in the chromosome of the host cell, or is provided on a plasmid within the host cell.
(12) A method according to (10) or (11) wherein the plasmid is, or is not, a 2µm-family plasmid.
(13) A method according to (12) wherein the plasmid comprises a gene encoding a protein comprising the sequence of the first chaperone protein and/or a gene encoding a protein comprising the sequence of the second chaperone protein, and a gene encoding a desired heterologous protein.
(14) A method according to (12) or (13) wherein the plasmid is a disintegration vector.
(15) A method according to any of (1) to (14) wherein the desired protein (such as a desired heterologous protein) comprises a leader sequence effective to cause secretion from the host cell, such as yeast.
(16) A method according to any of (1) to (15) wherein the desired protein (such as a desired heterologous protein) is a eucaryotic protein, or a fragment or variant thereof, optionally a vertebrate or a fungal (such as a yeast) protein.
(17) A method according to any of (1) to (16) wherein the desired protein (such as a desired heterologous protein) is a commercially useful protein, such as a therapeutically, diagnostically, industrially, domestically or nutritionally useful protein.
(18) A method according to any of (1) to (17) wherein the desired protein (such as a desired heterologous protein) comprises a sequence selected from albumin, a monoclonal antibody, an etoposide, a serum protein (such as a blood clotting factor, e.g. Factor VII, Factor VIII, Factor IX, Factor X and Factor XIII), antistasin, a tick anticoagulant peptide, transferrin, lactoferrin, endostatin, angiostatin, collagens, immunoglobulins, or immunoglobulin-based molecules or fragment of either (e.g. a dAb, Fab' fragments, F(ab')₂, scAb, scFv or scFv fragment), a Kunitz domain protein, interferons, interleukins, IL10, IL11, IL2, interferon α species and sub-species, interferon β species and sub-species, interferon γ species and sub-species, leptin, CNTF, CNTF_{Ax15}'(Axokinc™), IL1-receptor antagonist, erythropoietin (EPO) and EPO mimics, thrombopoietin (TPO) and TPO mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, α₁-antitrypsin, plasminogen activators, nerve growth factor, LACI, platelet-derived endothelial cell growth factor (PD-ECGF), glucose oxidase, serum cholinesterase, aprotinin, amyloid precursor protein, inter-alpha trypsin inhibitor, antithrombin III, apo-lipoprotein species, Protein C, Protein S, a metabolite, an antibiotic, or a variant or fragment of any of the above.
(19) A method according to any of (1) to (18) wherein the desired protein (such as a desired heterologous protein) comprises the sequence of albumin or a variant or fragment thereof.
(20) A method according to any of (1) to (19) wherein the desired protein (such as a desired heterologous protein) comprises the sequence of a transferrin family member, optionally transferrin or lactoferrin, or a variant or fragment thereof.
(21) A method according to any of (1) to (20) wherein the desired protein is a desired heterologous protein that comprises a fusion protein, such as a fusion protein of albumin or a transferrin family member or a variant or fragment of either, fused directly or indirectly to the sequence of another protein.
(22) A plasmid comprising a first recombinant gene encoding a protein comprising the sequence of a first chaperone protein and a second recombinant gene encoding a protein comprising the sequence of a second chaperone protein.
(23) A plasmid according to (22) wherein at least one, and optionally both, of the first and second chaperones are chaperones as defined by any one of (5) to (8).
(24) A plasmid according to (22) or (23) further comprising a third recombinant gene, which third recombinant gene encodes a desired heterologous protein, optionally a desired heterologous protein as described by any one of (15)-(21).
(25) A plasmid according to any one of (22) to (24) wherein the plasmid is a plasmid as defined by any one of (12) to (14).
(26) A host cell comprising a first recombinant gene encoding a protein comprising the sequence of a first chaperone protein, a second recombinant gene encoding a protein comprising the sequence of a second chaperone protein and a third gene, such as a third recombinant gene, encoding a desired protein (such as a desired heterologous protein), wherein the first and second chaperones are different.
(27) A host cell according to (26), which host cell is a host cell as defined by any one of (5) to (21).
(28) A host cell comprising a plasmid as defined by any one of (22)-(25).
(29) A method according to any one of (1) to (21) or a host cell according to any one of (26) to (28) wherein the host cell is a bacterial or yeast host cell.
(30) A method or a host cell according to (29) wherein the host cell is a yeast cell, optionally a member of the *Saccharomyces*, *Kluyveromyces*, *Arxula*, *Yarrowia, Candida, Schizosaccharomyces, Debaryomyces, Xanthophyllomyces, Geotrichum, Ashbya, Hortaea, Schwanniomyces, Trichosporon, Xanthophyllomyces,* or *Pichia* genus, such as *Saccharomyces cerevisiae*, *Kluyveromyces lactis*, *Pichia pastoris, Pichia membranaefaciens, Zygosaccharomyces rouxii*, *Zygosaccharomyces bailii, Zygosaccharomyces fermentati*, *Kluyveromyces drosphilarum, Pichia methanolica, Hansenula polymorpha* (also known as *Pichia angusta*)*, Arxula adeninivorans, Yarrowia lipolytica, Candida boidinii Candida utilis* or *Schizosaccharomyces pombe.*
(31) A host cell according to (27), or as defined by (10) or (11), in which the plasmid is a 2µm-family plasmid and wherein:
   (a) the plasmid is based on pSR1, pSB3 or pSB4 and the host cell is *Zygosaccharomyces rouxii*;
   (b) the plasmid is based on pSB1 or pSB2 and the host cell is *Zygosaccharomyces bailli;*
   (c) the plasmid is based on pSM1 and the host cell is *Zygosaccharomyces fermentati;*
   (d) the plasmid is based on pKD1 and the host cell is *Kluyveromyces drosophilarum;*
   (e) the plasmid is based on pPM1 and the host cell is *Pichia membranaefaciens;* or
   (f) the plasmid is based on the 2µm plasmid and the host cell is *Saccharomyces cerevisiae* or *Saccharomyces carlsbergensis.*
(32) A host cell according to (31) in which the plasmid is based on the 2µm plasmid and the host cell is *Saccharomyces cerevisiae* or *Saccharomyces carlsbergensis.*
(33) A method for producing desired protein (such as a desired heterologous protein) comprising:
   (a) providing a host cell comprising a first recombinant gene encoding a protein comprising the sequence of Orm2p or a variant or fragment thereof and a second gene, such as a second recombinant gene, encoding a desired protein (such as a desired heterologous protein), with the proviso that the first and second genes are not present within the host cell on the same 2µm-family plasmid; and
   (b) culturing the host cell in a culture medium to obtain expression of the first and second genes.
(34) A host cell comprising a plasmid, the plasmid comprising a gene that encodes an essential chaperone wherein, in the absence of the plasmid, the host cell is unable to produce the chaperone, the plasmid further comprising a recombinant gene encoding a desired heterologous protein, such as a desired heterologous protein as defined in any one of (15) to (21).
(35) A host cell according to (34) wherein the essential chaperone is a eucaryotic chaperone.
(36) A host cell according to (34) wherein the essential chaperone is a yeast chaperone.
(37) A host cell according to (34) wherein the essential chaperone is a yeast chaperone comprises the sequence of a protein encoded by a gene selected from *CCT2*, *CCT3*, *CCT4*, *CCT5*, *CCT6*, *CCT7*, *CCT8*, *CNS1*, *ERO1*, *HSP10*, *HSP60*, *PDI1*, *CDC37, KAR2*, *MGE1*, *MRS11*, *NOB1*, *SSC1*, *PSE1*, *TIM9*, *PAM18* and *TCP1* or a variant or fragment of any one of these..
(38) A host cell according to (34) wherein the essential chaperone is protein disulphide isomerase.
(39) A host cell according to any one of (34) to (38) wherein, in the absence of the plasmid, the host cell is inviable.
(40) A host cell according to (39) wherein, in the absence of the plasmid, the host cell is inviable and cannot be made viable by growing the host cell in a growth medium and making nutrient additions or modifications to that growth medium, and preferably cannot be made viable by growing the host cell in a growth medium and making any additions or modifications to that growth medium.
(41) A plasmid comprising, as the sole yeast selectable marker, a gene encoding a chaperone that is essential to the viability of a yeast host cell, in the sense that when the gene or genes encoding the essential chaperone are deleted or inactivated in a yeast host cell, then the host cell is inviable in culture and the deficiency cannot be complemented by additions or modifications to the culture medium.
(42) A plasmid according to (41) wherein the gene encoding the essential chaperone is the sole selectable marker encoded by the plasmid.
(43) A plasmid according to (42) wherein the essential chaperone is a eucaryotic chaperone.
(44) A plasmid according to (42) wherein the essential chaperone is a yeast chaperone.
(45) A plasmid according to (42) wherein the essential chaperone is a yeast chaperone comprises the sequence of a protein encoded by a gene selected from *CCT2*, *CCT3*, *CCT4*, *CCT5*, *CCT6*, *CCT7*, *CCT8*, *CNS1*, *ERO1*, *HSP10*, *HSP60*, *PDI1*, *CDC37*, *KAR2*, *MGE1*, *MRS11*, *NOB1*, *SSC1*, *PSE1*, *TIM9*, *PAM18* and *TCP1* or a variant or fragment of any one of these.
(46) A plasmid according to (42), wherein the essential chaperone is protein disulphide isomerase or Pselp.
(47) A plasmid according to any one of (41) to (46) further comprising a gene encoding a desired heterologous protein, such as a desired heterologous protein defined by any one of (15) to (21).
(48) A plasmid according to any one of (41) to (47) which is a 2µm-family plasmid.
(49) A plasmid according to any one of (41) to (48) which is a plasmid as defined by any one of (22) to (25).
(50) A host cell according to any one of (34) to (40) in which the plasmid is a plasmid according to any one of (41) (49).
(51) A host cell according to (28) wherein a chaperone encoded by the plasmid is an essential gene.
(52) A host cell according to (50) wherein, in the absence of the plasmid, the host cell does not produce the chaperone.
(53) A method according to (1) wherein the host cell is a host cell as defined by (51) or (52).
(54) A method according to (53) wherein the step (b) of (1) involves culturing the host cell in non-selective media, such as rich or complex media.
(55) A method for producing a desired recombinant protein comprising the steps of: providing a host cell comprising a plasmid, the plasmid comprising a first recombinant gene that encodes a chaperone that is essential to the viability of the host cell (the "essential chaperone") and wherein, in the absence of the plasmid, the host cell is unable to produce the essential chaperone, which host cell is optionally a host cell as defined by any one of (34) to (40) or (50) to (52), and further in which the host cell comprises a gene that encodes a desired protein; culturing the host cell in a culture medium under conditions that allow the expression of the essential chaperone and the desired protein; and optionally isolating the thus expressed desired protein from the cultured host cell or the culture medium; and optionally purifying the thus isolated desired protein to a commercially acceptable level of purity; and further optionally, lyophilising the thus purified protein or formulating the purified desired protein with a carrier or diluent (such as a pharmaceutically acceptable carrier or diluent); and optionally presenting the thus formulated desired protein in a unit dosage form.
(56) The method of (55) wherein the step of culturing the host cell involves culturing the host cell in a non-selective media, such as a complex or rich media.
(57) A method for producing a desired protein (such as a desired heterologous protein) comprising the steps of:
   (a) providing a host cell as defined by any one of (34) to (40); and
   (b) culturing the host cell in a culture medium under conditions that allow the expression of the essential chaperone and the desired protein.
(58) The method of (57) wherein the host cell comprises a plasmid as defined by any one of (41) to (49).
(59) The method of (57) or (58) wherein step (b) of the method of (57) is performed by culturing the host cell in a non-selective medium, such as a rich or complex medium.
(60) Use of a polynucleotide comprising a sequence that encodes a chaperone that is essential to the viability of a host cell (the "essential chaperone"), to increase the stability of a plasmid in the host cell by integration of the polynucleotide into the plasmid to produce a modified plasmid, wherein the host cell is unable to produce the essential chaperone in the absence of the modified plasmid.
(61) Use according to (60) to increase the stability of a plasmid in the host cell when the host cell is grown under non-selective conditions, such as in a rich or complex medium.
(62) Use according to (60) or (61) wherein the plasmid is a 2µm-family plasmid.
(63) Use according to any one of (60) to (62) wherein the plasmid further comprises a gene encoding a desired heterologous protein, such as a desired heterologous protein as defined in any one of (15) to (21).
(64) Use according to any one of (60) to (63) wherein the essential chaperone is a eucaryotic chaperone.
(65) Use according to any one of (60) to (63) wherein the essential chaperone is a yeast chaperone.
(66) Use according to any one of (60) to (63) wherein the essential chaperone is a yeast chaperone comprises the sequence of a protein encoded by a gene selected from *CCT2*, *CCT3*, *CCT4*, *CCT5*, *CCT6*, *CCT7*, *CCT8*, *CNS1*, *ERO1*, *HSP10*, *HSP60*, *PDI1*, *CDC37*, *KAR2*, *MGE1*, *MRS11*, *NOB1*, *SSC1*, *PSE1*, *TIM9*, *PAM18* and *TCP1* or a variant or fragment of any one of these.
(67) Use according to any one of (60) to (63) wherein the essential protein is a chaperone, such as protein disulphide isomerase or Pselp.
(68) Use according to any one of (60) to (67) to simultaneously increase the stability of a plasmid in the host cell and increase the ability of the host cell to produce protein product.
(69) Use according to (68) wherein the protein product is an endogenously encoded protein or a heterologous protein, such as a heterologous protein as defined in any one of (15) to (21).
(70) Use according to (69) wherein the protein product is a heterologous protein that is encoded by a recombinant gene that has been integrated into the chromosome of the host cell.
(71) Use according to (69) wherein the protein product is a heterologous protein that is encoded by a recombinant gene that is present on a plasmid in the host cell.
(72) Use according to (71) wherein the plasmid that comprises the recombinant gene that encodes the heterologous protein is the same plasmid as the modified plasmid that comprises the polynucleotide that encodes the essential chaperone.
(73) A host cell, plasmid, method or use according to any one of (44) to (82) wherein the host cell is a bacterial or yeast host cell.
(74) A host cell, plasmid, method or use according to (73) wherein the host cell is a yeast cell, optionally a member of the *Saccharomyces, Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces,* or *Pichia* genus, such as *Saccharomyces cerevisiae*, *Kluyveromyces lactis, Pichia pastoris*, *Pichia membranaefaciens, Zygosaccharomyces rouxii*, *Zygosaccharomyces bailii*, *Zygosaccharomyces fermentati*, *Kluyveromyces drosphilarum, Pichia methanolica, Hansenula polymorpha* (also known as *Pichia angusta*)*, Arxula adeninivorans, Yarrowia lipolytica, Candida boidinii Candida utilis* or *Schizosaccharomyces pombe.*
(75) A host cell, plasmid, method or use according to (84), in which the plasmid is a 2µm-family plasmid and wherein:
   (a) the plasmid is based on pSR1, pSB3 or pSB4 and the host cell is *Zygosaccharomyces rouxii*;
   (b) the plasmid is based on pSB1 or pSB2 and the host cell is *Zygosaccharomyces bailli;*
   (c) the plasmid is based on pSM1 and the host cell is *Zygosaccharomyces fermentati;*
   (d) the plasmid is based on pKD1 and the host cell is *Kluyveromyces drosophilarum;*
   (e) the plasmid is based on pPM1 and the host cell is *Pichia membranaefaciens;* or
   (f) the plasmid is based on the 2µm plasmid and the host cell is *Saccharomyces cerevisiae* or *Saccharomyces carlsbergensis.*
(76) A host cell, plasmid, method or use according to (85) in which the plasmid is based on the 2 µm plasmid and the host cell is *Saccharomyces cerevisiae* or *Saccharomyces carlsbergensis.*
(77) Use of a polynucleotide comprising the sequence of a promoter operably connected to a coding sequence encoding a chaperone for increasing the expression of a desired protein (such as a desired heterologous protein) in a host cell by expression of the polynucleotide sequence within the host cell, wherein the promoter is characterised in that it achieves a lower level of expression of the chaperone than would be achieved if the coding sequence were to be operably connected to its naturally occurring promoter, optionally wherein the use is a use as defined in any one of (70) to (82).
(78) A method for producing a desired protein (such as a desired heterologous protein) comprising the steps of:
   (a) providing a host cell comprising a recombinant gene that comprising the sequence of promoter operably connected to a coding sequence encoding a chaperone, the promoter being characterised in that it achieves a lower level of expression of the chaperone than would be achieved if the coding sequence were to be operably connected to its naturally occurring promoter, and the host cell further comprising a gene, such as a recombinant gene, encoding a desired protein (such as a desired heterologous protein); and
   (b) culturing the host cell in a under conditions that allow the expression of the chaperone and the desired protein (such as a desired heterologous protein); optionally wherein the method is a method according to any preceding method claim.
(79) The method of (78) further comprising the step of purifying the desired protein (such as a desired heterologous protein) produced in step (b); and optionally lyophilising the thus purified protein.
(80) The method of (79) further comprising the step of formulating the purified or lyophilised desired protein (such as a desired heterologous protein) with a carrier or diluent.
(81) The method of (80) further comprising the step of presenting the thus formulated protein in a unit dosage form.

The present invention will now be exemplified with reference to the following non-limiting examples and figures.

### BRIEF DESCRIPTION OF THE FIGURES

**Figure 1** shows a map of a typical 2µm plasmid.

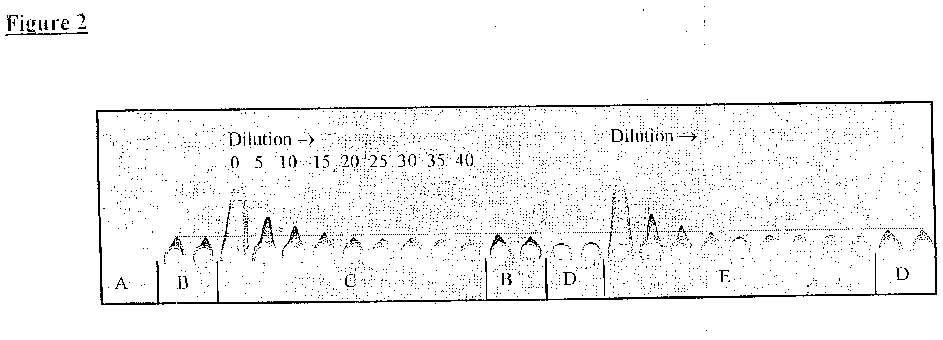

**Figure 2** shows the results of rocket immunoelectrophoresis (RIE) determination of increased recombinant transferrin (N413Q, N611Q) secretion with *PDI1* over-expression. Cryopreserved yeast stocks were grown for 4-days in 10mL BMMD shake flask cultures and supernatants were loaded at 5µL per well. Goat polyclonal anti-transferrin (human) antiserum (Calbiochem) was used at 40µL per rocket immunoelectrophoresis gel (50mL). A = Control strain [pSAC35], duplicate flasks; B = Control strain [pDB2536], duplicate flasks; C = Control strain [pDB2711], neat to 40-fold aqueous dilutions; D = Control strain [pDB2931], duplicate flasks; E = Control strain [pDB2929], neat to 40-fold aqueous dilutions.

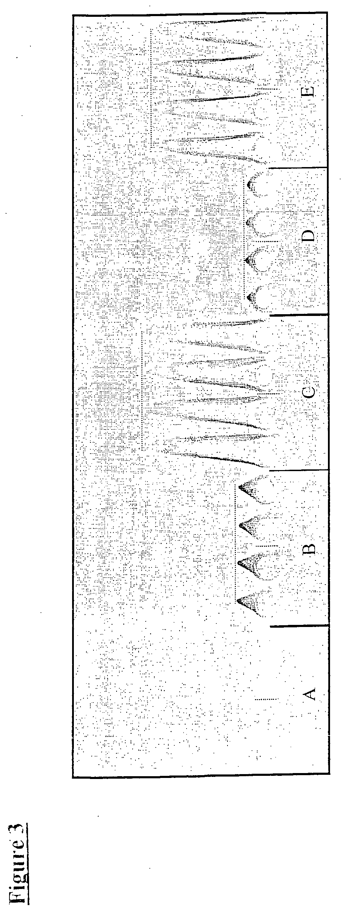

**Figure 3** shows the results of RIE analysis of recombinant transferrin (N413Q, N611Q) secretion with and without *PDI1* over-expression. Cryopreserved yeast stocks were grown for 4-days in 10mL BMMD shake flask cultures and supernatants were loaded at 5µL per well. Duplicate loadings were made of supernatants from two individual cultures of each strain. Goat polyclonal anti-transferrin (human) antiserum (Calbiochem) was used at 40µL per rocket immunoelectrophoresis gel (50mL). A = Control strain [pSAC35]; B = Control strain [pDB2536]; C = Control strain [pDB2711]; D = Control strain [pDB2931]; E = Control strain [pDB2929].

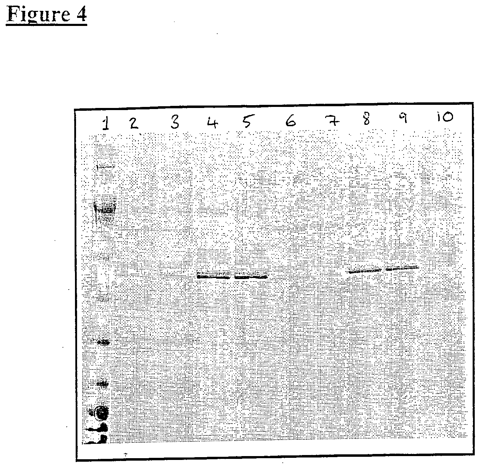

**Figure 4** shows the results of SDS-PAGE analysis of recombinant transferrin secretion with and without *PDI1* over-expression. BMMD shake flask cultures were grown for 4-days and 10µL supernatant analysed on non-reducing SDS-PAGE (4-12% NuPAGE^{®}, MOPS buffer, InVitrogen) with GelCode^{®} Blue reagent (Pierce). 1 = SeeBlue Plus2 Markers (InVitrogen). 2 = pDB2536; 3 = pDB2536; 4 = pDB2711; 5 = pDB2711; 6 = pDB2931; 7 = pDB2931; 8 = pDB2929; 9 = pDB2929; 10 = pSAC35 control.

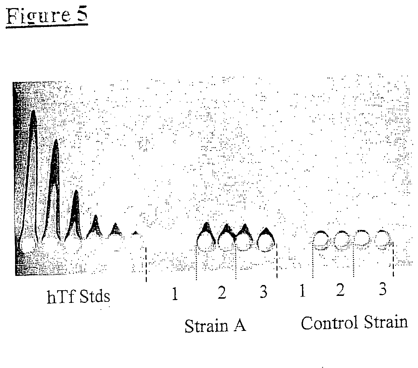

**Figure 5** shows RIE analysis of recombinant transferrin secretion from *S. cerevisiae* strains with an additional integrated copy of *PDI1.* 5-day BMMD shake flask culture supernatants were loaded at 5mL per well. Strains contained: 1) pSAC35 (negative control); 2) pDB2536 (recombinant non-glycosylated transferrin (N413Q, N611Q)) or 3) pDB2506 (same as plasmid pDB2536 but the transferrin ORF encodes transferrin without the N→Q mutations at positions 413 and 611, i.e. recombinant glycosylated transferrin). Each well contained a sample derived from an individual transformant. Standards were human plasma holo-transferrin (Calbiochem) at 100, 50, 20, 10, 5 and 2mg.L⁻¹.

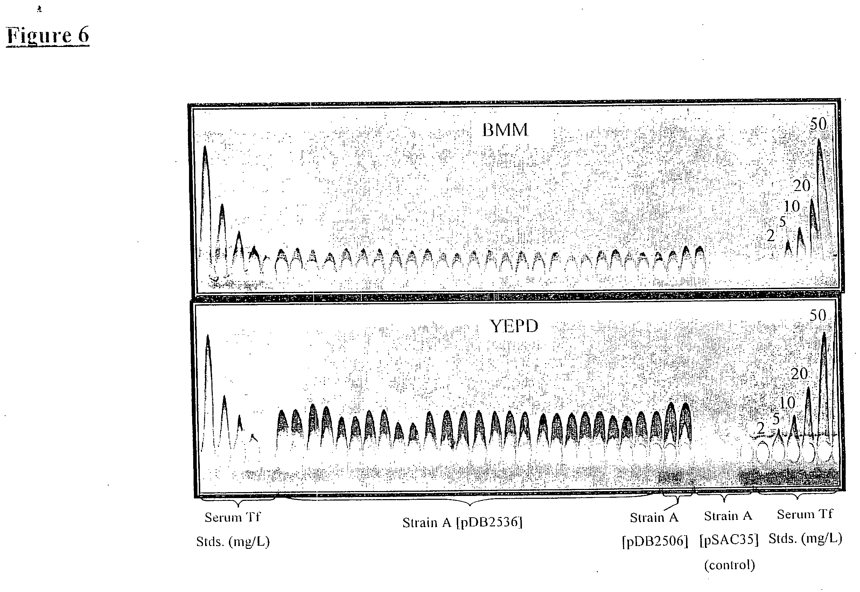

**Figure 6** shows RIE analysis of recombinant transferrin secretion from Strain A [pDB2536] and Strain A [pDB2506] grown in shake flask culture. 5-day BMMD or YEPD shake flask culture supernatants were loaded in duplicate at 5mL per well.

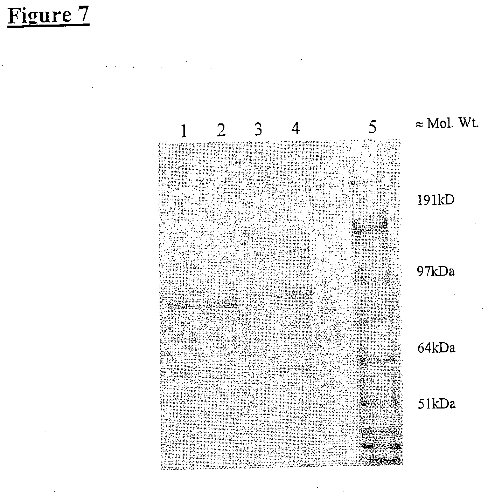

**Figure 7** shows SDS-PAGE analysis of recombinant transferrin secreted from Strain A [pDB2536] and Strain A [pDB2506] grown in shake flask culture. Cultures were grown for 5-days in BMMD and 30mL supernatants analysed on SDS-PAGE (4-12% NuPAGE^{™}, MOPS Buffer, InVitrogen) stained with GelCode, Blue Reagent (Pierce). 1) Strain A [pDB2536] transformant 1; 2) Strain A [pDB2536] transformant 2; 3) Strain A [pSAC35] control; 4) Strain A [pDB2506] transformant 1; 5) SeeBlue, Plus2 Protein Standards (approximate molecular weights only).

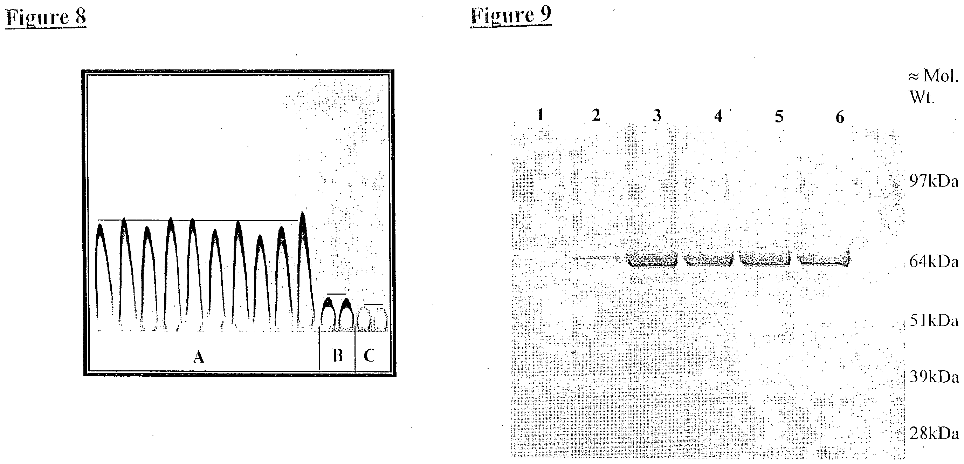

**Figure 8** shows RIE of recombinant transferrin secreted from *S. cerevisiae* strains with different *PDI1* copy numbers. 3-day BMMD shake flask culture supernatants were loaded at 5mL per well. Goat polyclonal anti-transferrin (human) antiserum (Calbiochem) was used at 30mL per rocket immunoelectrophoresis gel (50mL). (A) supernatant from *S. cerevisiae* control strain [pDB2711] or [pDB2712]; (B) supernatant from Strain A [pDB2536]; (C) supernatant from control strain [pDB2536].
**Figure 9** shows SDS-PAGE analysis of recombinant transferrin secreted from *S. cerevisiae* strains with different *PDI1* copy numbers. 4-12% NuPAGE reducing gel run with MOPS buffer (InVitrogen) after loading with 30mL of 3-day BMMD shake flask culture supernatant per lane; (lane 1) supernatant from control strain [pDB2536]; (lane 2) supernatant from Strain A [pDB2536]; (lanes 3-6) supernatant from control strain [pDB2711] or [pDB2712]; (lane 7) molecular weight markers (SeeBlue Plus2, InVitrogen).

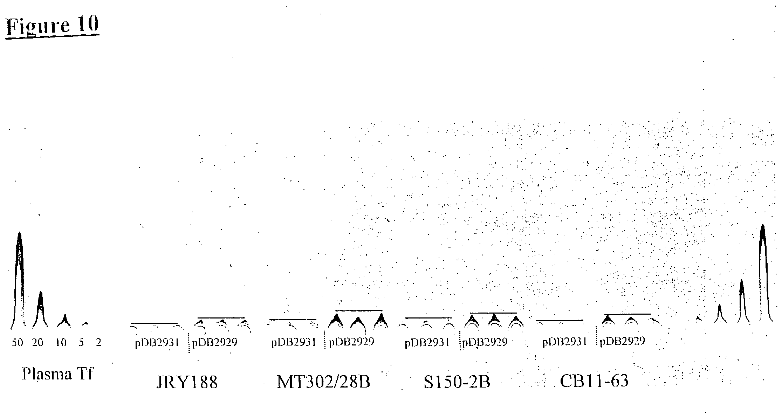

**Figure 10** shows RIE of recombinant transferrin secreted from different *S. cerevisiae* strains with and without additional *PDI1* gene co-expression. 10mL YEPD shake flasks were inoculated with yeast and incubated for 4-days at 30°C. 5µL culture supernatant loaded per well of a rocket immunoelectrophoresis gel. Plasma Tf standards concentrations are in µg/mL. 20µL goat anti-Tf / 50mL agarose. Precipin was stained with Coomassie blue.

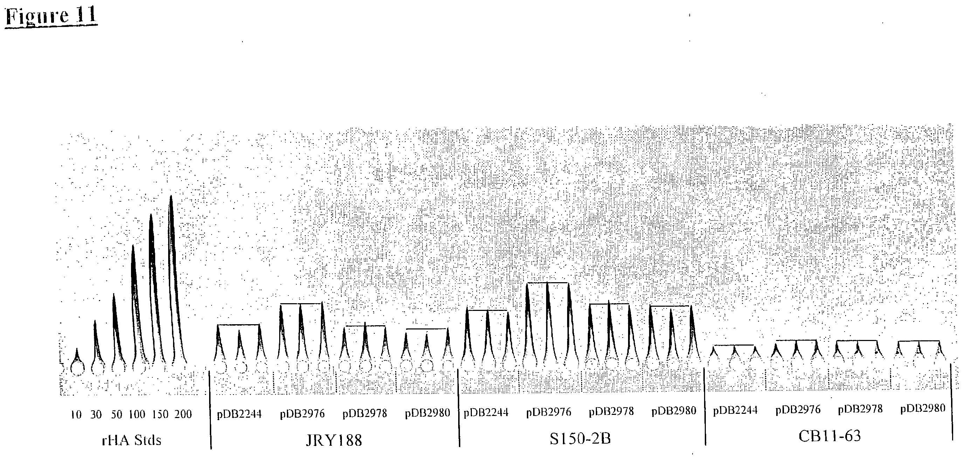

**Figure 11** shows RIE analysis of rHA expression in different *S. cerevisiae* strains when co-expressed with *PDI1* genes having different length promoters. 10mL YEPD shake flasks were inoculated with yeast and incubated for 4-days at 30°C. 4µL culture supernatant loaded per well of a rocket immunoelectrophoresis gel. rHA standards concentrations are in µg/mL. 400µL goat anti-HA (Sigma product A-1151 resuspended in 5mL water) /50mL agarose. Precipin was stained with Coomassie blue.

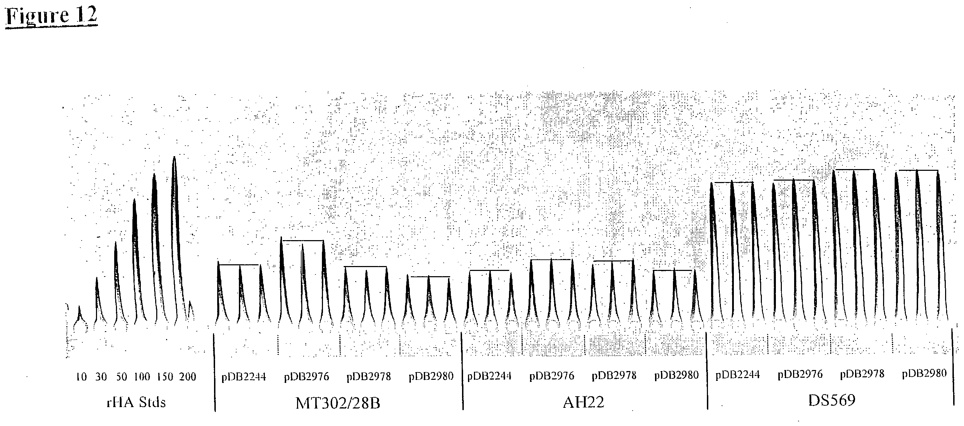

**Figure 12** shows RIE analysis of rHA expression in different *S. cerevisiae* strains when co-expressed with *PDI1* genes having different length promoters. 10mL YEPD shake flasks were inoculated with yeast and incubated for 4-days at 30°C. 4µL culture supernatant loaded per well of a rocket immunoelectrophoresis gel. rHA standards concentrations are in µg/mL. 400µL goat anti-HA (Sigma product A-1151 resuspended in 5mL water) /50mL agarose. Precipin was stained with Coomassie blue.

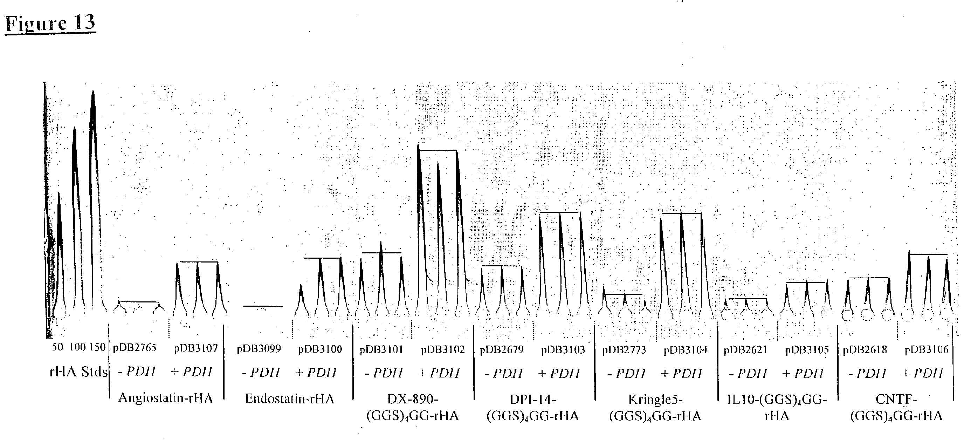

**Figure 13** shows RIE analysis of rHA fusion proteins with and without co-expressed recombinant PDI1. 10mL BMMD shake flasks were inoculated with YBX7 transformed with albumin fusion expression plasmids and incubated for 4-days at 30°C. 4µL culture supernatant loaded per well of a rocket immunoelectrophoresis gel. rHA standards concentrations are in µg/mL. 200µL goat anti-HA (Sigma product A-1151 resuspended in 5mL water) /50mL agarose. Precipin was stained with Coomassie blue.

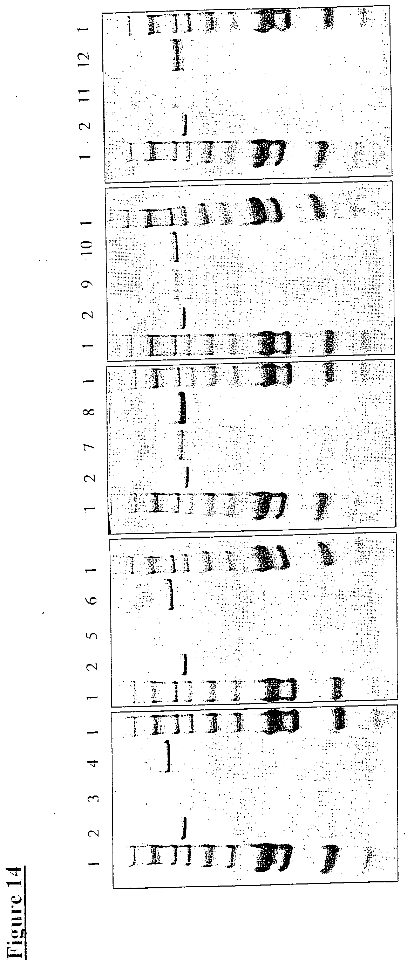

**Figure 14** shows SDS-PAGE analysis of recombinant albumin fusion secretion with and without *PDI1* present on the expression plasmid. 10mL BMMD shake flasks were inoculated with yeast and incubated for 4-days at 30°C, 200rpm. 30µL supernatant analysed on non-reducing SDS-PAGE (4-12% NuPAGE^{®}, MES buffer, InVitrogen) with GelCode^{®} Blue reagent (Pierce). 1 = SeeBlue Plus2 Markers (InVitrogen); 2 = 1 µg rHA; 3 = angiostatin-rHA; 4 = angiostatin-rHA + *PDI1;* 5 = endostatin-rHA; 6 = endostatin-rHA + *PDI1;* 7 = DX-890-(GGS)₄GG-rHA; 8 = DX-890-(GGS)₄GG-rHA + *PDI1;* 9 = DPI-14-(GGS)₄GG-rHA; 10 = DPI-14-(GGS)₄GG-rHA + *PDI1;* 11 = Axokine™ (CNTF_{Ax105})-(GGS)₄GG-rHA (Lambert et al, 2001, Proc. Natl. Acad. Sci. USA, 98, 4652-4657); 12 = Axokine™ (CNTF_{Ax15}) -(GGS)₄GG-rHA + *PDI1.*

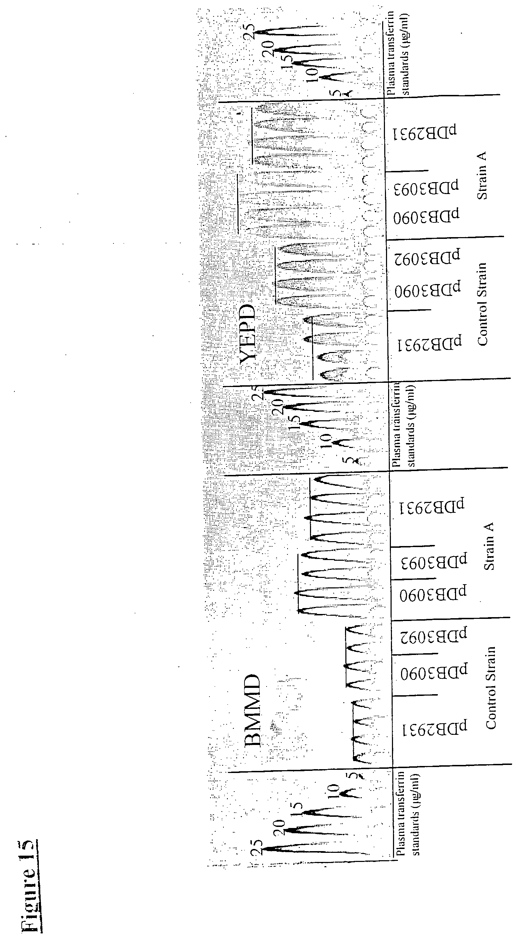

**Figure 15** shows RIE analysis demonstrating increased transferrin secretion from *S. cerevisiae* with *ORM2* co-expression from a 2µm-based plasmid. Four day shake flask culture supernantants were loaded at 5µl per well. Standards were human plasma holo-transferrin (Calbiochem), at 25, 20, 15, 10, 5 µg/ml, loaded 5µl per well. Goat polyclonal anti-transferrin (human) antiserum (Calbiochem) used at 20 µl per rocket immunoelectrophoresis gel (50 ml).

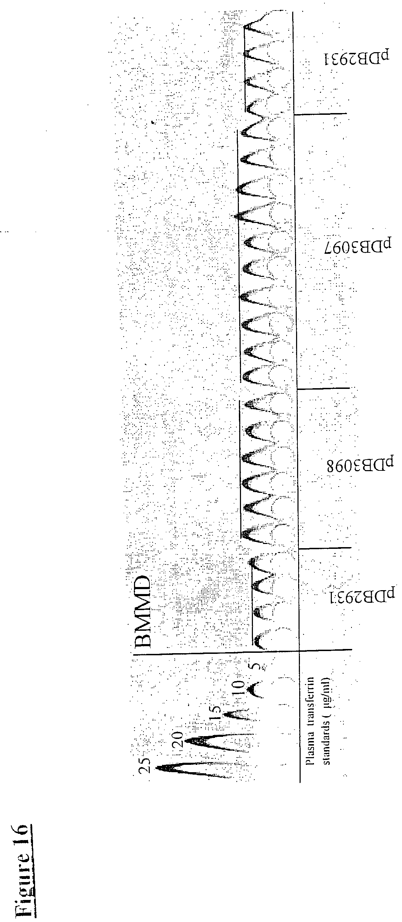

**Figure 16** shows RIE analysis demonstrating increased transferrin secretion from *S. cerevisiae* with *PSE1* co-expression from a 2µm-based plasmid. Four day shake flask culture supernantants were loaded at 5µl per well. Standards were human plasma holo-transferrin (Calbiochem), at 25, 20, 15, 10, 5 µg/ml, loaded 5µl per well. Goat polyclonal anti-transferrin (human) antiserum (Calbiochem) used at 20µl per rocket immunoelectrophoresis gel (50 ml).

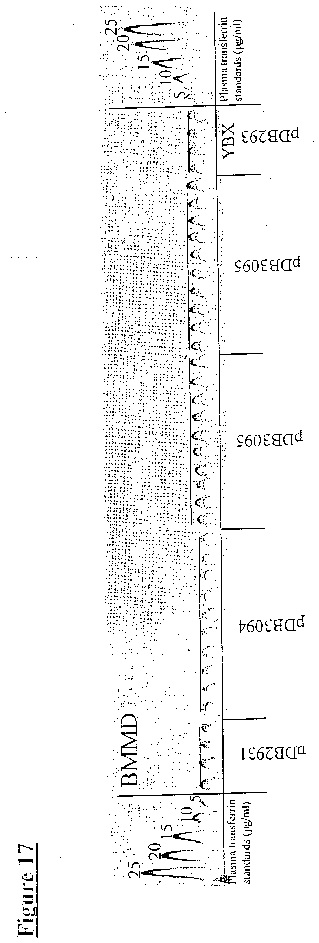

**Figure 17** shows RIE analysis demonstrating increased transferrin secretion from *S. cerevisiae* with *SSA1* co-expression from a 2µm-based plasmid. Four day shake flask culture supernantants were loaded at 5µl per well. Standards were human plasma holo-transferrin (Calbiochem), at 25, 20, 15, 10, 5 µg/ml, loaded 5µl per well. Goat polyclonal anti-transferrin (human) antiserum (Calbiochem) used at 20µl per rocket immunoelectrophoresis gel (50 ml).

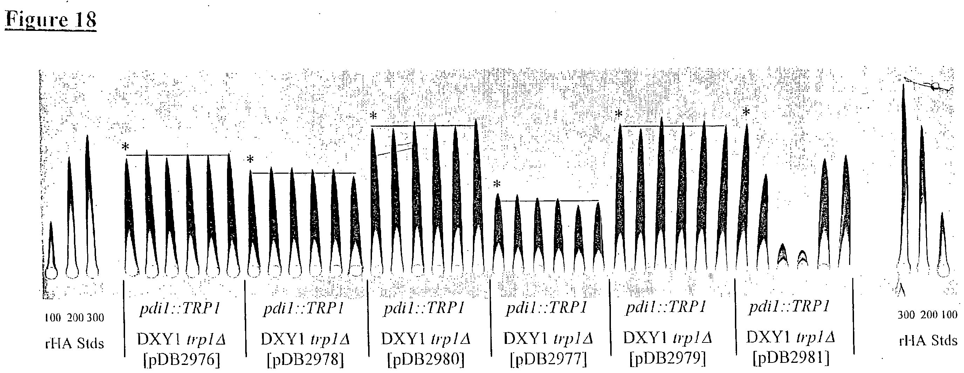

**Figure 18** shows the results of RIE. 10mL YEPD shake flasks were inoculated with DXY1 *trp1Δ* [pDB2976], DXY1 *trp1Δ* [pDB2977], DXY1 *trp1*Δ [pDB2978], DXY1 *trp1Δ* [pDB2979], DXY1 *trp1Δ* [pDB2980] or DXY1 *trp1*Δ [pDB2981] transformed to tryptophan prototrophy with a 1.41kb *Not*I/*Pst*I *pdi1::TRP1* disrupting DNA fragment was isolated from pDB3078. Transformants were grown for 4-days at 30°C, 200rpm. 4µL culture supernatant loaded per well of a rocket immunoelectrophoresis gel. rHA standards concentrations are in µg/mL. 700µL goat anti-HA (Sigma product A-1151 resuspended in 5mL water) /50mL agarose. Precipin was stained with Coomassie blue. Isolates selected for further analysis are indicated (*).

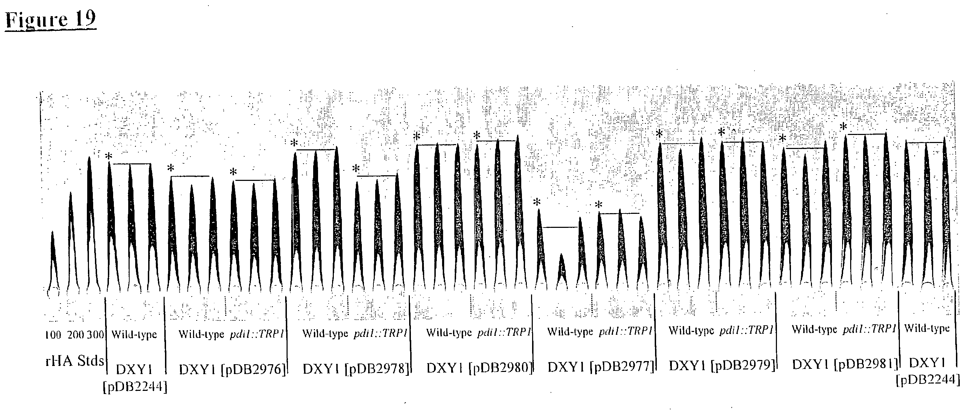

**Figure 19** shows the results of RIE. 10mL YEPD shake flasks were inoculated with DXY1 [pDB2244], DXY1 [pDB2976], DXY1 *trp1Δ pdi1::TRP1* [pDB2976], DXY1 [pDB2978], DXY1 *trp1Δ pdi1::TRP1* [pDB2978], DXY1 [pDB2980], DXY1 *trp1Δ pdi1::TRP1* [pDB2980], DXY1 [pDB2977], DXY1 *trp1Δ pdi1::TRP1* [pDB2977], DXY1 [pDB2979] DXY1 *trp1Δ pdi1::TRP1* [pDB2979], DXY1 [pDB2981] and DXY1 *trp1Δ pdi1::TRP1* [pDB2981], and were grown for 4-days at 30°C, 200rpm. 4µL culture supernatant loaded per well of a rocket immunoelectrophoresis gel. rHA standards concentrations are in µg/mL. 800µL goat anti-HA (Sigma product A-1151 resuspended in 5mL water) /50mL agarose. Precipin was stained with Coomassie blue. Isolates selected for further analysis are indicated (*)

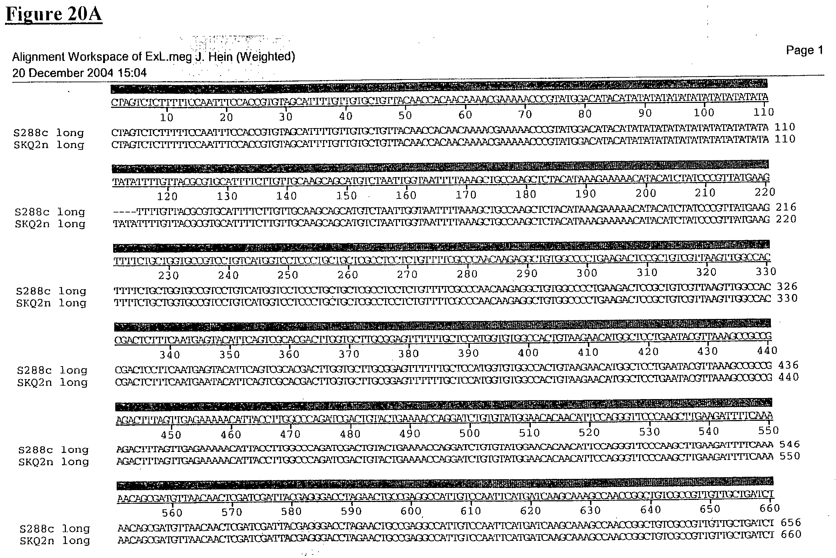

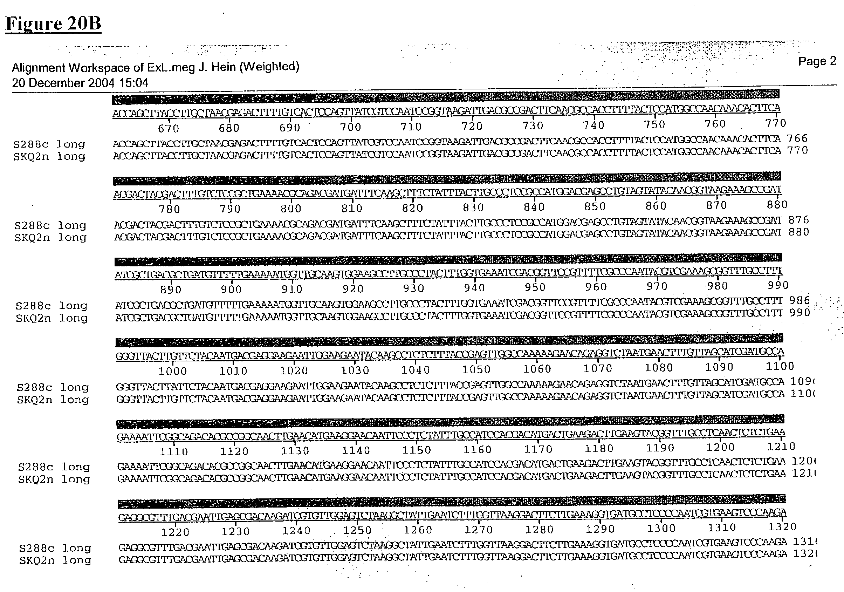

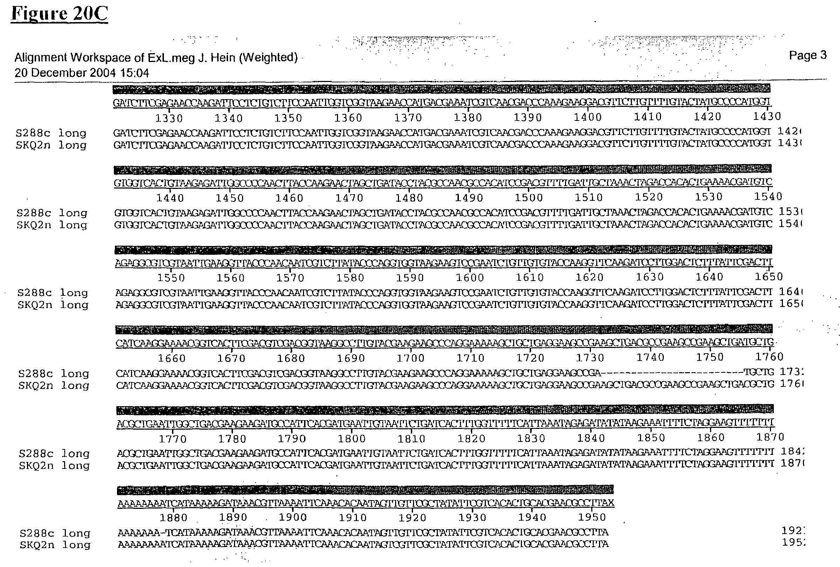

**Figure 20** shows a sequence alignment of the SKQ2n and S288c gene sequences with long promoters, as described in Example 6.

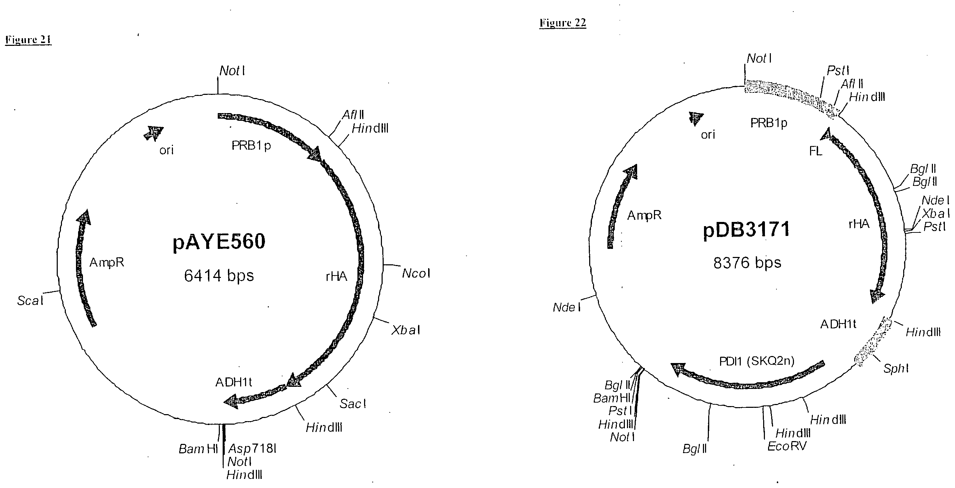

**Figures 21 to 33** show various plasmid maps.

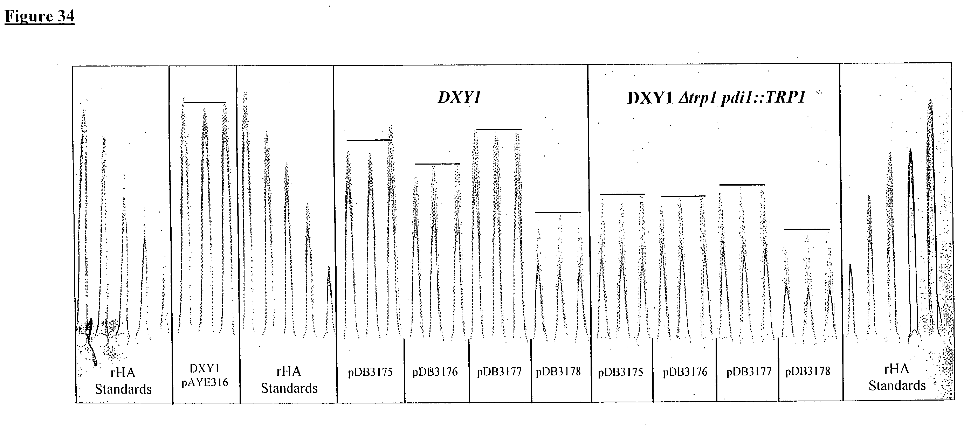

**Figure 34** shows Rocket Immunoelectrophoresis of YEPD shake flask culture supernatants from DXY1 and DXY1 *Δtrp1 pdi1::TRP1* containing pDB3175 to pDB3178. 10mL YEPD shake flasks were inoculated with DXY1 [pAYE316], DXY1 [pDB3175], DXY1 [pDB3176], DXY1 [pDB3177], DXY1 [pDB3178], DXY1 *Δtrp1 pdi1::TRP1* [pDB3175], DXY1 *Δtrp1 pdi1::TRP1* [pDB3176], DXY1 *Δtrp1 pdi1::TRP1* [pDB3177], and DXY1 *Δtrp1 pdi1::TRP1* [pDB3178] were grown for 4-days at 30°C, 200rpm. 5µL culture supernatant was loaded per well of a 50mL rocket immunoelectrophoresis gel in triplicate. rHA standard concentrations were 300, 200, 150, 100 and 50 µg/mL. 600µL goat anti-HSA (Sigma product A-1151 resuspended in 5mL water) per 50mL agarose gel. Precipitin was stained with Coomassie blue.

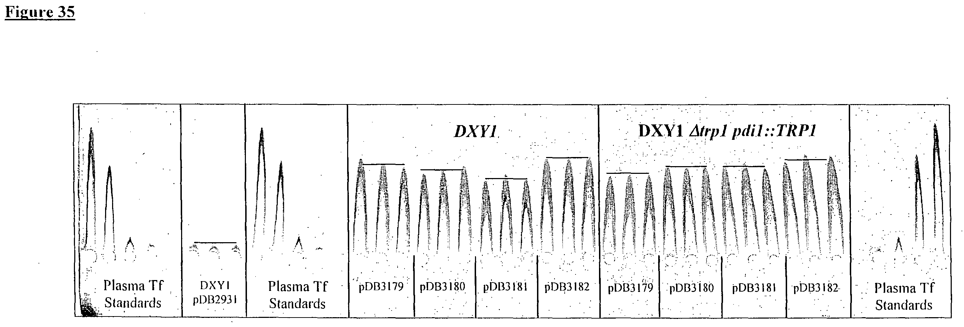

**Figure 35** shows Rocket Immunoelectrophoresis of YEPD shake flask culture supernatants from DXY1 and DXY1 *Δtrp1 pdi1::TRP1* containing pDB3179 to pDB3182. 10mL YEPD shake flasks were inoculated with DXY1 [pDB2931], DXY1 [pDB3179], DXY1 [pDB3180], DXY1 [pDB3181], DXY1 [pDB3182], DXY1 *Δtrp1 pdi1::TRP1* [pDB3179], DXY1 *Δtrp1 pdi1::TRP1* [pDB3180], DXY1 *Δtrp1 pdi1::TRP1* [pDB3181], and DXY1 *Δtrp1 pdi1::TRP1* [pDB3182] were grown for 4-days at 30°C, 200rpm. 5µL culture supernatant was loaded per well of a 50mL rocket immunoelectrophoresis gel in triplicate. Plasma transferrin standard concentrations were 100, 50, 20, 10 and 5 µg/mL. 40µL goat polyclonal anti human transferrin antiserum (Calbiochem) was used per 50mL agarose gel. Precipitin was stained with Coomassie blue.

### EXAMPLES

Two types of expression cassette have been used to exemplify secretion of a recombinant human transferrin mutant (N413Q, N611Q) from *S. cerevisiae.* One type uses a modified HSA(pre)/MFα (pro) leader sequence (named the "modified fusion leader" sequence). The second type of expression cassette uses only the modified HSA(pre) leader sequence.

The 24 amino acid sequence of the "modified fusion leader" is MKWVFIVSILFLFSSAYSRSLDKR.

The 18 amino acid sequence of the modified HSA(pre) leader sequence is MKWVFIVSILFLFSSAYS.

Transferrin (N413Q, N611Q) expression using these two cassettes has been studied in *S. cerevisiae* using the 2 µm expression vector with and without an additional copy of the *S. cerevisiae* PDI gene, *PDI1.*

### EXAMPLE 1

### Construction of expression plasmids

Plasmids pDB2515, pDB2529, pDB2536, pDB2688, pDB2690, pDB2711, pDB2921, pDB2928, pDB2929, pDB2930, pDB2931, pDB2932 and pDB2690 were constructed as described in Example 1 of WO 2005/061718.

### EXAMPLE 2

### Expression of transferrin

A *S. cerevisiae* control strain was transformed to leucine prototrophy with all the transferrin (N413Q, N611Q) expression plasmids, and cryopreserved stocks were prepared.

Strains were grown for four days at 30°C in 10mL BMMD cultures in 50mL conical flasks shaken at 200rpm. The titres of recombinant transferrin secreted into the culture supernatants were compared by rocket immunoelectrophoresis (RIE as described in Weeke, B., 1976, "Rocket immunoelectrophoresis" In N. H. Azelsen, J. Kroll, and B. Weeke [eds.], A manual of quantitative immunoelectrophoresis. Methods and applications. Universitetsforlaget, Oslo, Norway), reverse phase high performance liquid chromatography (RP-HPLC) (Table 1), and non-reducing SDS polyacrylamide electrophoresis stained with colloidal Coomassie blue stain (SDS-PAGE). The increase in recombinant transferrin secreted when *S. cerevisiae PDI1* was over-expressed was estimated to be greater than 10-fold.

By RP-HPLC analysis (using the method described in Example 2 of WO 2005/061718) the increase in transferrin secretion was determined to be 18-fold for the modified fusion leader sequence and 15-fold for the modified HSA-pre leader sequence (Table 1).

Figure 4 shows an SDS-PAGE comparison of the recombinant transferrin secreted by *S. cerevisiae* strains with and without additional *PDI1* expression.

**Table 1:**

| Plasmid | Secretory Leader | Additional *PDI1* | Average Transferrin Titre (µg.mL⁻¹) (n=2) | Estimated Increase due to Additional *PDI1* |
|---|---|---|---|---|
| pSAC35 | None | No | 0.4 | - |
| pDB2536 | Fusion Leader | No | 6.2 | - |
| pDB2711 | Fusion Leader | Yes | 112.8 | **18-fold** |
| pDB2931 | Modified HSA-pre Leader | No | 5.1 | - |
| pDB2929 | Modified HSA-pre Leader | Yes | 76.1 | **15-fold** |

RIE analysis indicated that the increased transferrin secretion in the presence of additional copies of *PDI1* was approximately 15-fold (Figure 2). By RIE analysis the increase appeared slightly larger for the modified HSA-pre leader sequence than for the modified fusion leader sequence (Figure 3).

### EXAMPLE 3

### Chromosomal over-expression of PDI

*S. cerevisiae* Strain A was selected to investigate the secretion of recombinant glycosylated transferrin expression from plasmid pDB2506 and recombinant non-glycosylated transferrin (N413Q, N611Q) from plasmid pDB2536. Strain A has the following characteristics -
- additional chromosomally integrated *PDI1* gene integrated at the host *PDI1* chromosomal location.
- the *URA3* gene and bacterial DNA sequences containing the ampicillin resistance gene were also integrated into the *S. cerevisiae* genome at the insertion sites for the above genes.

A control strain had none of the above insertions.

Control strain [cir⁰] and Strain A [cir⁰] were transformed to leucine prototrophy with pDB2506 (recombinant transferrin), pDB2536 (recombinant non-glycosylated transferrin (N413Q, N611Q)) or pSAC35 (control). Transformants were selected on BMMD-agar.

The relative level of transferrin secretion in BMMD shake flask culture was determined for each strain/plasmid combination by rocket immunoelectrophoresis (RIE). Figure 5 shows that both strains secreted both the glycosylated and non-glycosylated recombinant transferrins into the culture supernatant.

The levels of both the glycosylated and non-glycosylated transferrins secreted from Strain A [pDB2506] and Strain A [pDB2536] respectively, appeared higher than the levels secreted from the control strain. Hence, at least in shake flask culture, *PDI1* integrated into the host genome at the *PDI1* locus in Strain A has enhanced transferrin secretion.

Furthermore, the increase in transferrin secretion observed between control strain [pDB2536] and Strain A [pDB2536] appeared to be at least a 100% increase by RIE. In contrast, the increase in rHA monomer secretion between control strain [pDB2305] and Strain A [pDB2305] was approximately 20% (data not shown). Therefore, the increase in transferrin secretion due to the additional copy of *PDI1* in Strain A was surprising large considering that transferrin has 19 disulphide bonds, compared to rHA with 17 disulphide bonds. Additional copies of the *PDI1* gene may be particularly beneficial for the secretion from *S. cerevisiae* of proteins from the transferrin family, and their derivatives.

The levels of transferrin secreted from Strain A [pDB2536] and Strain A [pDB2506] were compared by RIE for transformants grown in BMMD and YEPD (Figure 6). Results indicated that a greater than 2-fold increase in titres of both non-glycosylated recombinant transferrin (N413Q, N611Q) and glycosylated recombinant transferrin was achieved by growth in YEPD (10-20 mg.L⁻¹ serum transferrin equivalent) compared to BMMD (2-5 mg.L⁻¹ serum transferrin equivalent). The increase in both glycosylated and non-glycosylated transferrin titre observed in YEPD suggested that both transferrin expression plasmids were sufficiently stable under non-selective growth conditions to allow the expected increased biomass which usually results from growth in YEPD to be translated into increased glycosylated and non-glycosylated transferrin productivity.

SDS-PAGE analysis of non-glycosylated transferrin (N413Q, N611Q) secreted from Strain A [pDB2536] and glycosylated transferrin from Strain A [pDB2506] grown in BMMD shake flask culture is shown in Figure 7. Strain A [pDB2536] samples clearly showed an additional protein band compared to the Strain A [pSAC35] control. This extra band migrated at the expected position for the recombinant transferrin (N413Q, N611Q) secreted from control strain [pDB2536]. Strain A [pDB2506] culture supernatants appeared to contain a diffuse protein band at the position expected for transferrin. This suggested that the secreted recombinant transferrin was heterogeneous, possibly due to hyper-mannosylation at Asp413 and/or Asp611.

### EXAMPLE 4

### Comparing transferrin secretion from S. cerevisiae control strain containing pDB2711 with transferrin secretion from S. cerevisiae Strain A

Plasmid pDB2711 is as described above. Plasmid pDB2712 (Figure 22 of WO 2005/061718) was also produced with the *Not*I cassette in the opposite direction to pDB2711.

Control strain *S. cerevisiae* [cir⁰] was transformed to leucine prototrophy with pDB2711 and pDB2712. Transformants were selected on BMMD-agar and cryopreserved trehalose stocks of control strain [pDB2711] were prepared.

Secretion of recombinant transferrin (N413Q, N611Q) by control strain [pDB2711], control strain [pDB2712], Strain A [pDB2536], control strain [pDB2536] and an alternative control strain [pDB2536] was compared in both BMMD and YEPD shake flask culture. RIE indicated that a significant increase in recombinant transferrin secretion had been achieved from control strain [pDB2711] with multiple episomal *PDI1* copies, compared to Strain A [pDB2536] with two chromosomal copies of *PDI1,* and control strain [pDB2536] with a single chromosomal copy of *PDI1* gene (Figure 8). Control strain [pDB2711] and control strain [pDB2712] appeared to secrete similar levels of rTf (N413Q, N611Q) into the culture media. The levels of secretion were relatively consistent between control strain [pDB2711] and control strain [pDB2712] transformants in both BMMD and YEPD media, suggesting that plasmid stability was sufficient for high-level transferrin secretion even under non-selective conditions. This is in contrast to the previous published data in relation to recombinant PDGF-BB and HSA where introduction of *PDI1* into multicopy 2 µm plasmids was shown to be detrimental to the host.

**Table 2: Recombinant transferrin titres from high cell density fermentations**

| **Strain** | **Supernatant (g.L⁻¹)** | |
|---|---|---|
| | **GP-HPLC** | **SDS-PAGE** |
| Control [pDB2536] | 0.5/0.4 | - |
| Alternative control [pDB2536] | 1.5/1.6 | 0.6 |
| | 0.9/0.9 | 0.4/0.4/0.5 |
| Strain A [pDB2536] | 0.7 | 0.6 |
| | 0.6 | - |
| Control [pDB2711] | 3.5 | 3.6 |
| | 3.4 | 2.7/3.1 |

Reducing SDS-PAGE analysis of transferrin secreted from control strain [pDB2711], control strain [pDB2712], Strain A [pDB2536], control strain [pDB2536] and alternative control strain [pDB2536] in BMMD shake flask culture is shown in Figure 9. This shows an abundant protein band in all samples from control strain [pDB2711] and control strain [pDB2712] at the position expected for transferrin (N413Q, N611Q). The relative stain intensity of the transferrin (N413Q, N611Q) band from the different strains suggested that Strain A [pDB2536] produced more than control strain [pDB2536] and alternative control strain [pDB2536], but that there was an even more dramatic increase in secretion from control strain [pDB2711] and control strain [pDB2712]. The increased recombinant transferrin secretion observed was concomitant with the increased *PDI1* copy number in these strains. This suggested that Pdi1p levels were limiting transferrin secretion in control strain, Strain A and the alternative control strain, and that elevated *PDI1* copy number was responsible for increased transferrin secretion. Elevated *PDI1* copy number could increase the steady state expression level of *PDI1* so increasing the amount of Pdi1p activity. There are a number of alternative methods by which this could be achieved without increasing the copy number of the *PDI1* gene, for example the steady state *PDI1* mRNA level could be increased by either increasing the transcription rate, say by use of a higher efficiency promoter, or by reducing the clearance rate of the *PDI1* mRNA. Alternatively, protein engineering could be used to enhance the specific activity or turnover number of the Pdi1p protein.

In high cell density fermentations control strain [pDB2711] recombinant transferrin (N413Q, N611Q) production was measured at approximately 3g.L⁻¹ by both GP-HPLC analysis and SDS-PAGE analysis (Table 2). This level of production is several fold-higher than control strain, the alternative control strain or Strain A containing pDB2536. Furthermore, for the production of proteins for therapeutic use in humans, expression systems such as control strain [pDB2711] have advantages over those using Strain A, as they do not contain bacterial DNA sequences.

### CONCLUSIONS

Secretion of recombinant transferrin from a multicopy expression plasmid (pDB2536) was investigated in *S. cerevisiae* strains containing an additional copy of the *PDI1* gene integrated into the yeast genome. Transferrin secretion was also investigated in *S. cerevisiae* transformed with a multicopy expression plasmid, in which the *PDI1* gene has been inserted into the multicopy episomal transferrin expression plasmid (pDB2711).

A *S. cerevisiae* strain with an additional copy of the *PDI1* gene integrated into the genome at the endogenous *PDI1* locus, secreted recombinant transferrin and non-glycosylated recombinant transferrin (N413Q, N611Q) at an elevated level compared to strains containing a single copy of *PDI1.* A further increase in *PDI1* copy number was achieved by using pDB2711 In high cell density fermentation of the strain transformed with pDB2711, recombinant transferrin (N413Q, N611Q) was secreted at approximately 3g.L⁻¹, as measured by SDS-PAGE and GP-HPLC analysis. Therefore, increased *PDI1* gene copy number has produced a large increase in the quantity of recombinant transferrins secreted from *S. cerevisiae.*

The following conclusions are drawn -
1. In shake flask analysis of recombinant transferrin expression from pDB2536 (non-glycosylated transferrin (N413Q, N611Q) and pDB2506 (glycosylated transferrin) the *S. cerevisiae* strain Strain A secreted higher levels of both recombinant transferrins into the culture supernatant than control strains. This was attributed to the extra copy of *PDI1* integrated at the *PDI1* locus.
2. Control strain [pDB2711], which contained the *PDI1* gene on the multicopy expression plasmid, produced a several-fold increase in recombinant transferrin (N413Q, N611Q) secretion compared to Strain A [pDB2536] in both shake flask culture and high cell density fermentation.
3. Elevated *PDI1* copy number in yeast such as *S. cerevisiae* will be advantageous during the production of desired proteins (such as a desired heterologous proteins), such as those from the transferrin family.
4. pSAC35-based plasmids containing additional copies of *PDI1* gene have advantages for the production of proteins from the transferrin family, and their derivatives, such as fusions, mutants, domains and truncated forms.

### EXAMPLE 5

### Insertion of a PDI1 gene into a 2µm-like plasmid increased secretion of recombinant transferrin from various different S. cerevisiae strains

The *S. cerevisiae* strain JRY188 cir⁺ (National Collection of Yeast Cultures) and MT302/28B cir⁺ (Finnis et al., 1993, Eur. J. Biochem., 212, 201-210) was cured of the native 2µm plasmid by galactose induced over-expression of *FLP* from Yep351*-GAL-FLP1,* as described by Rose and Broach (1990, *Meth. Enzymol.,* **185**, 234-279) to create the *S. cerevisiae* strains JRY188 cir⁰ and MT302/28B cir⁰, respectively.

The *S. cerevisiae* strains JRY188 cir⁰, MT302/28B cir⁰, S150-2B cir⁰ (Cashmore et al., 1986, Mol. Gen. Genet., 203, 154-162), CB11-63 cir⁰ (Zealey et al., 1988, Mol. Gen. Genet., 211, 155-159) were all transformed to leucine prototrophy with pDB2931 (Figure 14 of WO 2005/061718) and pDB2929 (Figure 12 of WO 2005/061718). Transformants were selected on appropriately supplemented minimal media lacking leucine. Transformants of each strain were inoculated into 10mL YEPD in 50mL shake flasks and incubated in an orbital shaker at 30°C, 200rpm for 4-days. Culture supernatants were harvested and the recombinant transferrin titres compared by rocket immunoelectrophoresis (Figure 10). The results indicated that the transferrin titres in supernatants from all the yeast strains were higher when *PDI1* was present in the 2µm plasmid (pDB2929) than when it was not (pDB2931)

### EXAMPLE 6

### The construction of expression vectors containing various PDI1 genes and the expression cassettes for various heterologous proteins on the same 2µm-like plasmid

### PCR amplification and cloning of PDI1 genes into YIplac211:

The *PDI1* genes from *S. cerevisiae* S288c and *S. cerevisiae* SKQ2n were amplified by PCR to produce DNA fragments with different lengths of the 5'-untranslated region containing the promoter sequence. PCR primers were designed to permit cloning of the PCR products into the *EcoR*I and *Bam*HI sites of YIplac211 (Gietz & Sugino, 1988, Gene, 74, 527-534). Additional restriction endonuclease sites were also incorporated into PCR primers to facilitate subsequent cloning. Table 3 describes the plasmids constructed and Table 4 gives the PCR primer sequences used to amplify the *PDI1* genes. Differences in the *PDI1* promoter length within these YIplac211-based plasmids are described in Table 3.

pDB2939 (Figure 27 of WO 2005/061718) was produced by PCR amplification of the *PDI1* gene from *S. cerevisiae* S288c genomic DNA with oligonucleotide primers DS248 and DS250 (Table 5), followed by digesting the PCR product with *Eco*RI and *Bam*HI and cloning the approximately 1.98-kb fragment into YIplac211 (Gietz & Sugino, 1988, Gene, 74, 527-534), that had been cut with *EcoR*I and *Bam*HI*.* DNA sequencing of pDB2939 identified a missing 'G' from within the DS248 sequence, which is marked in bold in Table 4. Oligonucleotide primers used for sequencing the *PDI1* gene are listed in Table 5, and were designed from the published S288c *PDI1* gene sequence (PDI1/YCL043C on chromosome III from coordinates 50221 to 48653 plus 1000 base pairs of upstream sequence and 1000 base pairs of downstream sequence. (http://www.yeastgenome.org/Genebank Accession number NC001135).

**Table 3: YIplac211-based Plasmids Containing PDI1 Genes**

| *Plasmid* | *Plasmid Base* | PDI1 *Gene* | | | *PCR Primers* |
|---|---|---|---|---|---|
| | | *Source* | *Promoter* | *Terminator* | |
| ***pDB2939*** | ***YIplac211*** | ***S288c*** | ***Long (∼210-bp)*** | **→ Bsu*36I*** | ***DS248* + *DS250*** |
| ***pDB2941*** | ***YIplac211*** | ***S288c*** | ***Medium (∼140-bp)*** | **→ Bsu*36I*** | ***DS251* + *DS250*** |
| ***pDB2942*** | ***YIplac211*** | ***S288c*** | ***Short (∼80-bp)*** | **→ Bsu*36I*** | ***DS252* + *DS250*** |
| ***pDB2943*** | ***YIplac211*** | ***SKQ2n*** | ***Long (∼210-bp)*** | **→ Bsu*36I*** | ***DS248* + *DS250*** |
| ***pDB2963*** | ***YIplac211*** | ***SKQ2n*** | ***Medium (∼140-bp)*** | **→ Bsu*36I*** | ***DS267* + *DS250*** |
| ***pDB2945*** | ***YIplac211*** | ***SKQ2n*** | ***Short (∼80-bp)*** | **→ Bsu*36I*** | ***DS252* + *DS250*** |

**Table 4: Oligonucleotide Primers for PCR Amplification of S. cerevisiae PDI1 Genes**

| **Primer** | **Sequence** |
|---|---|
| DS248 | |
| DS249 | |
| DS250 | |
| DS251 | |
| DS252 | |
| DS267 | |

**Table 5: Oligonucleotide Primers for DNA Sequencing S. cerevisiae PDI1 Genes**

| **Primer** | **Sequence** |
|---|---|
| DS253 | 5'-CCTCCCTGCTGCTCGCC-3' |
| DS254 | 5'-CTGTAAGAACATGGCTCC-3' |
| DS255 | 5'-CTCGATCGATTACGAGGG-3' |
| DS256 | 5'-AAGAAAGCCGATATCGC-3' |
| DS257 | 5'-CAACTCTCTGAAGAGGCG-3' |
| DS258 | 5'-CAACGCCACATCCGACG-3' |
| DS259 | 5'-GTAATTCTGATCACTTTGG-3' |
| DS260 | 5'-GCACTTATTATTACTACGTGG-3' |
| DS261 | 5'-GTTTTCCTTGATGAAGTCG-3' |
| DS262 | 5'-GTGACCACACCATGGGGC-3' |
| DS263 | 5'-GTTGCCGGCGTGTCTGCC-3' |
| DS264 | 5'-TTGAAATCATCGTCTGCG-3' |
| DS265 | 5'-CGGCAGTTCTAGGTCCC-3' |
| DS266 | 5'-CCACAGCCTCTTGTTGGG-3' |
| M13/pUC Primer (-40) | 5'-GTTTTCCCAGTCACGAC-3' |

Plasmids pDB2941 (Figure 28 of WO 2005/061718) and pDB2942 (Figure 29 of WO 2005/061718) were constructed similarly using the PCR primers described in Tables 3 and 4, and by cloning the approximately 1.90-kb and 1.85-kb *Eco*RI-*Bam*HI fragments, respectively, into YIplac211. The correct DNA sequences were confirmed for the *PDI1* genes in pDB2941 and pDB2942.

The *S. cerevisiae* SKQ2n *PDI1* gene sequence was PCR amplified from plasmid DNA containing the *PDI1* gene from pMA3a:C7 (US 6,291,205), also known as Clone C7 (Crouzet & Tuite, 1987, *supra;* Farquhar *et al,* 1991, *supra*)*.* The SKQ2n *PDI1* gene was amplified using oligonucleotide primers DS248 and DS250 (Tables 3 and 4). The approximately 2.01-kb PCR product was digested with *Eco*RI and *Bam*HI and ligated into YIplac211 (Gietz & Sugino, 1988, Gene, 74, 527-534) that has been cut with *EcoR*I and *Bam*HI*,* to produce plasmid pDB2943 (Figure 30 of WO 2005/061718). The 5' end of the SKQ2n *PDI1* sequence is analogous to a blunt-ended *Spe*I-site extended to include the *Eco*RI*, Sac*I*, Sna*BI, *Pac*I*, Fse*I*, Sfi*I and *Sma*I sites, the 3' end extends up to a site analogous to a blunt-ended *Bsu*36I site, extended to include a *Sma*I*, Sna*BI and *Bam*HI sites. The *PDI1* promoter length is approximately 210bp. The entire DNA sequence was determined for the *PDI1* fragment using oligonucleotide primers given in Table 5. This confirmed the presence of a coding sequence for the PDI protein of *S. cerevisiae* strain SKQ2n (NCBI accession number CAA38402), but with a serine residue at position 114 (not an arginine residue as previously published). Similarly, in the same way as in the *S. cerevisiae* S288c sequence in pDB2939, pDB2943 also had a missing 'G' from within the DS248 sequence, which is marked in bold in Table 4.

Plasmids pDB2963 (Figure 31 of WO 2005/061718) and pDB2945 (Figure 32 of WO 2005/061718) were constructed similarly using the PCR primers described in Tables 3 and 4, and by cloning the approximately 1.94-kb and 1.87-kb *Eco*RI-*Bam*HI fragments, respectively, into YIplac211. The expected DNA sequences were confirmed for the *PDI1* genes in pDB2963 and pDB2945, with a serine codon at the position of amino acid 114.

### The construction of pSAC35-based rHA expression plasmids with different PDI1 genes inserted at the XcmI-site after REP2:

pSAC35-based plasmids were constructed for the co-expression of rHA with different *PDI1* genes (Table 6).

**Table 6: pSAC35-based plasmids for co-expression of rHA with different PDI1 genes**

| ***Plasmid*** | ***Plasmid Base*** | **PDI1 *Gene at* Xcm*I-site after REP2*** | | | | ***Heterologous Protein Expression Cassette (at* Not*I-site)*** |
|---|---|---|---|---|---|---|
| | | ***Source*** | ***Promoter*** | ***Terminator*** | ***Orientation*** | |
| ***pDB2982*** | ***pSAC35*** | ***SKQ2n*** | ***Long*** | **→ Bsu*36I*** | ***A*** | ***rHA*** |
| ***pDB2983*** | ***pSAC35*** | ***SKQ2n*** | ***Long*** | **→ Bsu*36I*** | ***B*** | ***rHA*** |
| ***pDB2984*** | ***pSAC35*** | ***SKQ2n*** | ***Medium*** | **→ Bsu*36I*** | ***A*** | ***rHA*** |
| ***pDB2985*** | ***pSAC35*** | ***SKQ2n*** | ***Medium*** | **→ Bsu*36I*** | ***B*** | ***rHA*** |
| ***pDB2986*** | ***pSAC35*** | ***SKQ2n*** | ***Short*** | **→ Bsu*36I*** | ***A*** | ***rHA*** |
| ***pDB2987*** | ***pSAC35*** | ***SKQ2n*** | ***Short*** | **→ Bsu*36I*** | ***B*** | ***rHA*** |
| ***pDB2976*** | ***pSAC35*** | ***S288c*** | ***Long*** | **→ Bsu*36I*** | ***A*** | ***rHA*** |
| ***pDB2977*** | ***pSAC35*** | ***S288c*** | ***Long*** | **→ Bsu*36I*** | ***B*** | ***rHA*** |
| ***pDB2978*** | ***pSAC35*** | ***S288c*** | ***Medium*** | **→ Bsu*36I*** | ***A*** | ***rHA*** |
| ***pDB2979*** | ***pSAC35*** | ***S288c*** | ***Medium*** | **→ Bsu*36I*** | ***B*** | ***rHA*** |
| ***pDB2980*** | ***pSAC35*** | ***S288c*** | ***Short*** | **→ Bsu*36I*** | ***A*** | ***rHA*** |
| ***pDB2981*** | ***pSAC35*** | ***S288c*** | ***Short*** | **→ Bsu*36I*** | ***B*** | ***rHA*** |

The rHA expression cassette from pDB2243 (Figure 33 of WO 2005/061718, as also described in WO 00/44772) was first isolated on a 2,992-bp *Not*I fragment, which subsequently was cloned into the *Not*I-site of pDB2688 (Figure 4 of WO 2005/061718) to produce pDB2693 (Figure 34 of WO 2005/061718). pDB2693 was digested with *Sna*BI*,* treated with calf intestinal alkaline phosphatase, and ligated with *SnaBI* fragments containing the *PDI1* genes from pDB2943, pDB2963, pDB2945, pDB2939, pDB2941 and pDB2942. This produced plasmids pDB2976 to pDB2987 (Figures 35 to 46 of WO 2005/061718). *PDI1* transcribed in the same orientation as *REP2* was designated "orientation A", whereas *PDI1* transcribed in opposite orientation to *REP2* was designated "orientation B" (Table 6).

### The construction of pSAC35-based transferrin expression plasmids with different PDI1 genes inserted at the XcmI-site after REP2:

pSAC35-based plasmids were constructed for the co-expression of recombinant transferrin (N413Q, N611Q) with different *PDI1* genes (Table 7).

**Table 7: pSAC35-based plasmids for co-expression of transferrin with different PDI1 genes**

| ***Plasmid*** | ***Plasmid Base*** | **PDI1 *Gene at* Xcm*I-site after REP2*** | | | | ***Heterologous Protein Expression Cassette (at* Not*I-site)*** |
|---|---|---|---|---|---|---|
| | | ***Source*** | ***Promoter*** | ***Terminator*** | ***Orientation*** | |
| ***pDB2929*** | ***pSAC35*** | ***SKQ2n*** | ***Long*** | **→ Bsu*36I*** | ***A*** | ***rTf (N413Q, N611Q)*** |
| ***pDB3085*** | ***pSAC35*** | ***S288c*** | ***Long*** | **→ Bsu*36I*** | ***A*** | ***rTf (N413Q, N611Q)*** |
| ***pDB3086*** | ***pSAC35*** | ***S288c*** | ***Medium*** | **→ Bsu*36I*** | ***A*** | ***rTf (N413Q, N611Q)*** |
| ***pDB3087*** | ***pSAC35*** | ***S288c*** | ***Short*** | **→ Bsu*36I*** | ***A*** | ***rTf (N413Q, N611Q)*** |

In order to achieve this, the NotI expression cassettes for rHA expression were first deleted from pDB2976, pDB2978, and pDB2980 by NotI digestion and circularisation of the vector backbone. This produced plasmids pDB3081 (Figure 47 of WO 2005/061718), pDB3083 (Figure 48 of WO 2005/061718) and pDB3084 (Figure 49 of WO 2005/061718) as described in Table 8.

**Table 8: pSAC35-based plasmids with different PDI1 genes**

| ***Plasmid*** | ***Plasmid Base*** | **PDI1 *Gene at* Xcm*I-site after REP2*** | | | | ***Heterologous Protein Expression Cassette (at Not*I*-site)*** |
|---|---|---|---|---|---|---|
| | | ***Source*** | ***Promoter*** | ***Terminator*** | ***Orientation*** | |
| ***pDB2690*** | ***pSAC35*** | ***SKQ2n*** | ***Long*** | **→ Bsu*36I*** | ***A*** | ***None*** |
| ***pDB3081*** | ***pSAC35*** | ***S288c*** | ***Long*** | **→ Bsu*36I*** | ***A*** | ***None*** |
| ***pDB3083*** | ***pSAC35*** | ***S288c*** | ***Medium*** | **→ Bsu*36I*** | ***A*** | ***None*** |
| ***pDB3084*** | ***pSAC35*** | ***S288c*** | ***Short*** | **→ Bsu*36I*** | ***A*** | ***None*** |

The 3,256-bp NotI fragment from pDB2928 (Figure 11 of WO 2005/061718) was cloned into the NotI-sites of pDB3081, pDB3083 and pDB3084, such that transcription from the transferrin gene was in the same direction as LEU2. This produced plasmids pDB3085 (Figure 50 of WO 2005/061718), pDB3086 (Figure 51 of WO 2005/061718) and pDB3087 (Figure 52 of WO 2005/061718) as described in Table 7.

### EXAMPLE 7

### Insertion and optimisation of a PDI1 gene in the 2µm-like plasmid increased the secretion of recombinant human serum albumin by various different S. cerevisiae strains

The *S. cerevisiae* strains JRY188 cir⁰, MT302/28B cir⁰, S150-2B cir⁰ , CB11-63 cir⁰ (all described above), AH22 cir⁰ (Mead et al., 1986, Mol. Gen. Genet., 205, 417-421) and DS569 cir⁰ (Sleep et al., 1991, Bio/Technology, 9, 183-187) were transformed to leucine prototrophy with either pDB2244 (WO 00/44772), pDB2976 (Figure 35 of WO 2005/061718), pDB2978 (Figure 37 of WO 2005/061718) or pDB2980 (Figure 39 of WO 2005/061718) using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates with appropriate supplements, and were subsequently patched out on BMMD-agar plates with appropriate supplements.

Transformants of each strain were inoculated into 10mL YEPD in 50mL shake flasks and incubated in an orbital shaker at 30°C, 200rpm for 4-days. Culture supernatants were harvested and the recombinant albumin titres compared by rocket immunoelectrophoresis (Figures 11 and 12). The results indicated that the albumin titres in the culture supernatants from all the yeast strains were higher when *PDI1* was present in the 2µm plasmid than when it was not (pDB2244). The albumin titre in the culture supernatants in the absence of *PDI1* on the plasmid was dependent upon which yeast strain was selected as the expression host, however, in most examples tested the largest increase in expression was observed when *PDI1* with the long promoter (~210-bp) was present in the 2µm plasmid (pDB2976). Modifying the *PDI1* promoter by shortening, for example to delete regulation sites, had the affect of controlling the improvement. For one yeast strain, known to be a high rHA producing strain (DS569) a shorter promoter was preferred for optimal expression.

### EXAMPLE 8

### PDI1 on the 2µm-based plasmid enhanced the secretion of recombinant albumin fusions.

The affect of co-expression of the *S. cerevisiae* SKQ2n *PDI1* gene with the long promoter (~210-bp) upon the expression of recombinant albumin fusions was investigated.

The plasmids defined in Table 9 below were generated as described in Example 9 ofWO 2005/061718.

**Table 9: Summary of plasmids encoding albumin fusion proteins**

| **Albumin fusion** | **Plasmid name** | |
|---|---|---|
| | With *PDI1* | Without *PDI1* |
| endostatin-albumin | pDB3100 | PDB3099 |
| angio statin-albumin | pDB3107 | pDB2765 |
| Kringle5-(GGS)₄GG-albumin | pDB3104 | pDB2773 |
| DX-890-(GGS)₄GG-albumin | pDB3102 | pDB3101 |
| DPI-14-(GGS)₄GG-albumin | pDB3103 | pDB2679 |
| Axokine™-(GGS)4GG-albumin | pDB3106 | pDB2618 |
| human IL10-(GGS)4-GG-albumin | pDB3105 | pDB2621 |

The same control yeast strain as used in previous examples was transformed to leucine prototrophy using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates, and were subsequently patched out on BMMD-agar plates. Cryopreserved trehalose stocks were prepared from 10mL BMMD shake flask cultures (24 hrs, 30°C, 200rpm).

Transformants of each strain were inoculated into 10mL BMMD in 50mL shake flasks and incubated in an orbital shaker at 30°C, 200rpm for 4-days. Culture supernatants were harvested and the recombinant albumin fusion titres compared by rocket immunoelectrophoresis (Figure 13). The results indicated that the albumin fusion titre in the culture supernatants from yeast strain was higher when *PDI1* was present in the 2µm plasmid than when it was not.

The increase in expression of the albumin fusions detected by rocket immunoelectrophoresis was further studied by SDS-PAGE analysis. BMMD shake flask cultures of YBX7 expressing various albumin-fusions were grown for 4-days in an orbital shaker at 30°C, 200rpm. A sample of the culture supernatant was analysed by SDS-PAGE (Figure 14). A protein band of the expected size for the albumin fusion under study was observed increase in abundance.

### EXAMPLE 9

### Co-expression of S. cerevisiae ORM2 and recombinant Transferrin on a 2µm-based plasmid

The *S. cerevisiae* Control Strain and Strain A (as described in Example 3) were selected to investigate the effect on transferrin secretion when the transferrin and *ORM2* genes were co-expressed from the 2µm-based plasmids. The Control Strain and Strain A were transformed to leucine prototrophy by plasmids pDB3090, pDB3092 and pBD3093 (containing expression cassettes for rTf (N413Q, N611Q) and for *ORM2),* as well as a control plasmid pDB2931 (containing the rTf (N413Q, N611Q) expression cassette without *ORM2*). The construction of these plasmids is described in Example 10 of WO 2005/061718. Transformants were selected on BMMD agar and patched out on BMMD agar for subsequent analysis.

To investigate the effect of *ORM2* co-expression on transferrin secretion, 10mL selective (BMMD) and non-selective (YEPD) liquid media were inoculated with strains containing the ORM2/transferrin co-expression plasmids. The shake flask culture was then incubated at 30°C with shaking (200 rpm) for 4 days. The relative level of transferrin secretion was determined by rocket gel immunoelectrophoresis (RIE) (Figure 15).

Levels of transferrin secreted from Control Strain [pDB3090] and Control Strain [pDB3092] were greater than the levels from Control Strain [pDB2931] in both BMMD and YEPS media. Similarly, the levels of transferrin secreted from both Strain A [pDB3090] and Strain A [pDB3093] were greater than the levels from Strain A [pDB2931] in both BMMD and YEPS media. Transferrin secretion from all Strain A transformants was higher than the Control Strain transformants grown in the same media. Strain A contains an additional copy of *PDI1* in the genome, which enhanced transferrin secretion. Therefore in Strain A, the increased expression of *ORM2* and *PDI1* had a cumulative effect on the secretion of transferrin.

### EXAMPLE 10

### Co-expression of S. cerevisiae PSE1 and recombinant Transferrin on a 2µm-based plasmid

The *S. cerevisiae* Control Strain was transformed to leucine prototrophy by plasmids, pDB3097 and pBD3098 (containing expression cassettes for rTf (N413Q, N611Q) and for *PSE1*)*,* as well as a control plasmid pDB2931 (containing the rTf (N413Q, N611Q) expression cassette without *PSE1*)*.* The construction of these plasmids are described in Example 11 of WO 2005/061718. Transformants were selected on BMMD agar and patched out on BMMD agar for subsequent analysis.

To investigate the effect of *PSE1* expression on transferrin secretion, flasks containing 10mL selective (BMMD) liquid media were inoculated with strains containing the *PSE1*/transferrin co-expression plasmids. The shake flask culture was then incubated at 30°C with shaking (200 rpm) for 4 days. The relative level of transferrin secretion was determined by rocket gel immunoelectrophoresis (RIE) (Figure 16).

Levels of transferrin secreted from Control Strain [pDB3097] and Control Strain [pDB3098] were greater than the levels from Control Strain [pDB2931] in BMMD media. Therefore, expression of *PSE1* from the 2µm-based plasmids had enhanced transferrin secretion from *S. cerevisiae.* Transferrin secretion was improved with the *PSE1* gene transcribed in either direction relative to the *REP2* gene in pDB3097 and pDB3098.

### EXAMPLE 11

### Co-expression of S. cerevisiae SSA1 and recombinant Transferrin on a 2µm-based plasmid

The *S. cerevisiae* Control Strain was transformed to leucine prototrophy by plasmids, pDB3094 and pBD3095 (containing expression cassettes for rTf (N413Q, N611Q) and for *SSA1*), as well as a control plasmid pDB2931 (containing the rTf (N413Q, N611Q) expression cassette without *SSA1*). The construction of these plasmids are described in Example 12 of WO 2005/061718. Transformants were selected on BMMD agar and patched out on BMMD agar for subsequent analysis.

To investigate the effect of *SSA1* expression on transferrin secretion, flasks containing 10mL selective (BMMD) liquid media were inoculated with strains containing the SSA*1*/transferrin co-expression plasmids. The shake flask cultures were incubated at 30°C with shaking (200 rpm) for 4 days. The relative level of transferrin secretion was determined by rocket gel immunoelectrophoresis (RIE) (Figure 17).

Levels of transferrin secreted from Control Strain [pDB3095] were greater than the levels from Control Strain [pDB2931] and Control Strain [pDB3094] in BMMD media. Therefore, expression of *SSA1* from the 2µm-based plasmids had enhanced transferrin secretion from *S. cerevisiae.* Transferrin secretion was improved with the *SSA1* gene transcribed in the opposite direction relative to the *REP2* gene in pDB3094.

### EXAMPLE 12

### PDI1 gene disruption, combined with a PDI1 gene on the 2µm-based plasmid enhanced the secretion of recombinant albumin and plasmid stability.

Single stranded oligonucleotide DNA primers listed in Table 11 were designed to amplify a region upstream of the yeast *PDI1* coding region and another a region downstream of the yeast *PDI1* coding region.

**Table 11: Oligonucleotide primers**

| **Primer** | **Description** | **Sequence** |
|---|---|---|
| DS299 | 5' *PDI1* primer, 38mer | |
| DS300 | 5' *PDI1* primer, 40mer | |
| DS301 | 3' *PDI1* primer, 38mer | |
| DS302 | 3' *PDI1* primer, 41 mer | |
| DS303 | 18mer | 5'-GGAGCGACAAACCTTTCG-3' |
| DS304 | 20mer | 5'-ACCGTAATAAAAGATGGCTG-3' |
| DS305 | 24mer | 5'-CATCTTGTGTGTGAGTATGGTCGG-3' |
| DS306 | 14mer | 5'-CCCAGGATAATTTTCAGG-3' |

Primers DS299 and DS300 amplified the 5' region of *PDI1* by PCR, while primers DS301 and DS302 amplified a region 3' of *PDI1,* using genomic DNA derived S288c as a template. The PCR conditions were as follows: 1µL S288c template DNA (at 0.01ng/µL, 0.1ng/µL, 1ng/µL, 10ng/µL and 100ng/µL), 5µL 10XBuffer (Fast Start Taq+Mg, (Roche)), 1µL 10mM dNTP's, 5µL each primer (2µM), 0.4µL Fast Start Taq, made up to 50µL with H₂O. PCRs were performed using a Perkin-Elmer Thermal Cycler 9700. The conditions were: denature at 95°C for 4min [HOLD], then [CYCLE] denature at 95°C for 30 seconds, anneal at 45°C for 30 seconds, extend at 72°C for 45 seconds for 20 cycles, then [HOLD] 72°C for 10min and then [HOLD] 4°C. The 0.22kbp *PDI1* 5' PCR product was cut with *Not*I and *Hin*dIII, while the 0.34kbp *PDI1* 3' PCR product was cut with *Hind*III and *Pst*I*.*

Plasmid pMCS5 (Hoheisel, 1994, Biotechniques 17, 456-460) (Figure 85 of WO 2005/061718) was digested to completion with *Hin*dIII, blunt ended with T4 DNA polymerase plus dNTPs and religated to create pDB2964 (Figure 86 of WO 2005/061718).

Plasmid pDB2964 was *Hind*III digested, treated with calf intestinal phosphatase, and ligated with the 0.22kbp *PDI1* 5' PCR product digested with *Not*I and *Hind*III and the 0.34kbp *PDI1* 3' PCR product digested with *Hind*III and *Pst*I to create pDB3069 (Figure 87 of WO 2005/061718) which was sequenced with forward and reverse universal primers and the DNA sequencing primers DS303, DS304, DS305 and DS306 (Table 11).

Primers DS234 and DS235 (Table 12) were used to amplify the modified *TRP1* marker gene from YIplac204 (Gietz & Sugino, 1988, Gene, 74, 527-534), incorporating *Hind*III restriction sites at either end of the PCR product. The PCR conditions were as follows: 1µL template YIplac204 (at 0.01ng/µL, 0.1ng/µL, 1ng/µL, 10ng/µL and 100ng/µL), 5µL 10XBuffer (Fast Start Taq+Mg, (Roche)), 1µL 10mM dNTP's, 5µL each primer (2µM), 0.4µL Fast Start Taq, made up to 50µL with H₂O. PCRs were performed using a Perkin-Elmer Thermal Cycler 9600. The conditions were: denature at 95°C for 4min [HOLD], then [CYCLE] denature at 95°C for 30 seconds, anneal for 45 seconds at 45°C, extend at 72°C for 90sec for 20 cycles, then [HOLD] 72°C for 10min and then [HOLD] 4°C. The 0.86kbp PCR product was digested with *Hind*III and cloned into the *Hind*III site of pMCS5 to create pDB2778 (Figure 88 of WO 2005/061718). Restriction enzyme digestions and sequencing with universal forward and reverse primers as well as DS236, DS237, DS238 and DS239 (Table 12) confirmed that the sequence of the modified *TRP1* gene was correct.

**Table 12: Oligonucleotide primers**

| **Primer** | **Description** | **Sequence** |
|---|---|---|
| DS230 | *TRP1* 5' UTR | 5'-TAGCGAATTC AATCAGTAAAAATCAACGG-3' |
| DS231 | *TRP1* 5' UTR | 5'-GTCAAAGCTTCAAAAAAAGA AAAGCTCCGG-3' |
| DS232 | *TRP1* 3' UTR | |
| DS233 | *TRP1* 3' UTR | 5'-GTCAAAGCTTTAAAGATAATGCTAAATCATTTGG-3' |
| DS234 | *TRP1* | |
| DS235 | *TRP1* | 5'-TGACAAGCTTGATCTTTTATGCTTGCTTTTC-3' |
| DS236 | *TRP1* | 5'-AATAGTTCAGGCACTCCG-3' |
| DS237 | *TRP1* | 5'-TGGAAGGCAAGAGAGCC-3' |
| DS238 | *TRP1* | 5'-TAAAATGTAAGCTCTCGG-3' |
| DS239 | *TRP1* | 5'-CCAACCAAGTATTTCGG-3' |
| CED005 | Δ*TRP1* | 5'-GAGCTGACAGGGAAATGGTC-3' |
| CED006 | Δ*TRP1* | 5'-TACGAGGATACGGAGAGAGG-3' |

The 0.86kbp *TRP1* gene was isolated from pDB2778 by digestion with *Hind*III and cloned into the *Hind*III site of pDB3069 to create pDB3078 (Figure 89 of WO 2005/061718) and pDB3079 (Figure 90 of WO 2005/061718). A 1.41kb *pdi1::TRP1* disrupting DNA fragment was isolated from pDB3078 or pDB3079 by digestion with *Not*I/*Pst*I*.*

Yeast strains incorporating a *TRP1* deletion (*trp1*Δ) were to be constructed in such a way that no homology to the *TRP1* marker gene (pDB2778) should left in the genome once the *trp1*Δ had been created, so preventing homologous recombination between future *TRP1* containing constructs and the *TRP1* locus. In order to achieve the total removal of the native *TRP1* sequence from the genome of the chosen host strains, oligonucleotides were designed to amplify areas of the 5' UTR and 3' UTR of the *TRP1* gene outside of *TRP1* marker gene present on integrating vector YIplac204 (Gietz & Sugino, 1988, Gene, 74, 527-534). The YIplac204 *TRP1* marker gene differs from the native/chromosomal *TRP1* gene in that internal *Hin*dIII, *Pst*I and *Xba*I sites were removed by site directed mutagenesis (Gietz & Sugino, 1988, Gene, 74, 527-534). The YIplac204 modified *TRP1* marker gene was constructed from a 1.453kbp blunt-ended genomic fragment *EcoR*I fragment, which contained the *TRP1* gene and only 102bp of the *TRP1* promoter (Gietz & Sugino, 1988, Gene, 74, 527-534). Although this was a relatively short promoter sequence it was clearly sufficient to complement *trp1* auxotrophic mutations (Gietz & Sugino, 1988, Gene, 74, 527-534). Only DNA sequences upstream of the *Eco*RI site, positioned 102bp 5' to the start of the *TRP1* ORF were used to create the 5' *TRP1* UTR. The selection of the 3' UTR was less critical as long as it was outside the 3' end of the functional modified *TRP1* marker, which was chosen to be 85bp downstream of the translation stop codon.

Single stranded oligonucleotide DNA primers were designed and constructed to amplify the 5' UTR and 3' UTR regions of the *TRP1* gene so that during the PCR amplification restriction enzyme sites would be added to the ends of the PCR products to be used in later cloning steps. Primers DS230 and DS231 (Table 12) amplified the 5' region of *TRP1* by PCR, while primers DS232 and DS233 (Table 12) amplified a region 3' of *TRP1*, using S288c genomic DNA as a template. The PCR conditions were as follows: 1µL template S288c genomic DNA (at 0.01ng/µL, 0.1ng/µL, 1ng/µL, 10ng/µL and 100ng/µL), 5µL 10XBuffer (Fast Start Taq+Mg, (Roche)), 1µL 10mM dNTP's, 5µL each primer (2µM), 0.4µL Fast Start Taq, made up to 50µL with H₂O. PCRs were performed using a Perkin-Elmer Thermal Cycler 9600. The conditions were: denature at 95°C for 4min [HOLD], then [CYCLE] denature at 95°C for 30 seconds, anneal for 45 seconds at 45°C, extend at 72°C for 90sec for 20 cycles, then [HOLD] 72°C for 10min and then [HOLD] 4°C.

The 0.19kbp *TRP1* 5' UTR PCR product was cut with *Eco*RI and *Hin*dIII, while the 0.2kbp *TRP1* 3' UTR PCR product was cut with *Bam*HI and *Hind*III and ligated into pAYE505 linearised with *Bam*HI/*Eco*RI to create plasmid pDB2777 (Figure 91 of WO 2005/061718). The construction of pAYE505 is described in WO 95/33833. DNA sequencing using forward and reverse primers, designed to prime from the plasmid backbone and sequence the cloned inserts, confirmed that in both cases the cloned 5' and 3' UTR sequences of the *TRP1* gene had the expected DNA sequence. Plasmid pDB2777 contained a *TRP1* disrupting fragment that comprised a fusion of sequences derived from the 5' and 3' UTRs of *TRP1.* This 0.383kbp *TRP1* disrupting fragment was excised from pDB2777 by complete digestion with *Eco*RI*.*

Yeast strain DXY1 (Kerry-Williams et al., 1998, Yeast, 14, 161-169) was transformed to leucine prototrophy with the albumin expression plasmid pDB2244 using a modified lithium acetate method (Sigma yeast transformation kit, YEAST-1, protocol 2; (Ito et al, 1983, J. Bacteriol., 153, 163; Elble, 1992, Biotechniques, 13, 18)) to create yeast strain DXY1 [pDB2244]. The construction of the albumin expression plasmid pDB2244 is described in WO 00/44772. Transformants were selected on BMMD-agar plates, and were subsequently patched out on BMMD-agar plates. Cryopreserved trehalose stocks were prepared from 10mL BMMD shake flask cultures (24 hrs, 30°C, 200rpm).

DXY1 [pDB2244] was transformed to tryptophan autotrophy with the 0.383kbp *Eco*RI *TRP1* disrupting DNA fragment from pDB2777 using a nutrient agar incorporating the counter selective tryptophan analogue, 5-fluoroanthranilic acid (5-FAA), as described by Toyn et al., (2000 Yeast 16, 553-560). Colonies resistant to the toxic effects of 5-FAA were picked and streaked onto a second round of 5-FAA plates to confirm that they really were resistant to 5-FAA and to select away from any background growth. Those colonies which grew were then were re-patched onto BMMD and BMMD plus tryptophan to identify which were tryptophan auxotrophs.

Subsequently colonies that had been shown to be tryptophan auxotrophs were selected for further analysis by transformation with YCplac22 (Gietz & Sugino, 1988, Gene, 74, 527-534) to ascertain which isolates were *trp1.*

PCR amplification across the *TRP1* locus was used to confirm that the trp⁻ phenotype was due to a deletion in this region. Genomic DNA was prepared from isolates identified as resistant to 5-FAA and unable to grow on minimal media without the addition of tryptophan. PCR amplification of the genomic *TRP1* locus with primers CED005 and CED006 (Table 12) was achieved as follows: 1µL template genomic DNA, 5µL 10XBuffer (Fast Start Taq+Mg, (Roche)), 1µL 10mM dNTP's, 5µL each primer (2µM), 0.4uL Fast Start Taq, made up to 50µL with H₂O. PCRs were performed using a Perkin-Elmer Thermal Cycler 9600. The conditions were: denature at 94°C for 10min [HOLD], then [CYCLE] denature at 94°C for 30 seconds, anneal for 30 seconds at 55°C, extend at 72°C for 120sec for 40 cycles, then [HOLD] 72°C for 10min and then [HOLD] 4°C. PCR amplification of the wild type *TRP1* locus resulted in a PCR product of 1.34kbp in size, whereas amplification across the deleted *TRP1* region resulted in a PCR product 0.84kbp smaller at 0.50kbp. PCR analysis identified a DXY1 derived trp⁻ strain (DXY1 *trp1Δ* [pDB2244]) as having the expected deletion event.

The yeast strain DXY1 *tp1Δ* [pDB2244] was cured of the expression plasmid pDB2244 as described by Sleep et al., (1991, Bio/Technology, 9, 183-187). DXY1 *tp1Δ* cir⁰ was re-transformed the leucine prototrophy with either pDB2244, pDB2976, pDB2977, pDB2978, pDB2979, pDB2980 or pDB2981 using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates supplemented with tryptophan, and were subsequently patched out on BMMD-agar plates supplemented with tryptophan. Cryopreserved trehalose stocks were prepared from 10mL BMMD shake flask cultures supplemented with tryptophan (24 hrs, 30°C, 200rpm).

The yeast strains DXY1 *tp1Δ* [pDB2976], DXY1 *tp1Δ* [pDB2977], DXY1 *tp1Δ* [pDB2978], DXY1 *tp1Δ* [pDB2979], DXY1 *trp1Δ* [pDB2980] or DXY1 *tp1Δ* [pDB2981] was transformed to tryptophan prototrophy using the modified lithium acetate method (Sigma yeast transformation kit, YEAST-1, protocol 2; (Ito et al, 1983, J. Bacteriol., 153, 163; Elble, 1992, Biotechniques, 13, 18)) with a 1.41kb *pdi1::TRP1* disrupting DNA fragment was isolated from pDB3078 by digestion with *Not*I/*Pst*I*.* Transformants were selected on BMMD-agar plates and were subsequently patched out on BMMD-agar plates.

Six transformants of each strain were inoculated into 10mL YEPD in 50mL shake flasks and incubated in an orbital shaker at 30°C, 200rpm for 4-days. Culture supernatants and cell biomass were harvested. Genomic DNA was prepared (Lee, 1992, Biotechniques, 12, 677) from the tryptophan prototrophs and DXY1 [pDB2244]. The genomic *PDI1* locus amplified by PCR of with primers DS236 and DS303 (Table 11 and 12) was achieved as follows: 1µL template genomic DNA, 5µL 10XBuffer (Fast Start Taq+Mg, (Roche)), 1µL 10mM dNTP's, 5µL each primer (2µM), 0.4µL Fast Start Taq, made up to 50µL with H₂O. PCRs were performed using a Perkin-Elmer Thermal Cycler 9700. The conditions were: denature at 94°C for 4min [HOLD], then [CYCLE] denature at 94°C for 30 seconds, anneal for 30 seconds at 50°C, extend at 72°C for 60sec for 30 cycles, then [HOLD] 72°C for 10min and then [HOLD] 4°C. PCR amplification of the wild type *PDI1* locus resulted in no PCR product, whereas amplification across the deleted *PDI1* region resulted in a PCR product 0.65kbp. PCR analysis identified that all 36 potential *pdi1::TRP1* strains tested had the expected *pdi1::TRP1* deletion.

The recombinant albumin titres were compared by rocket immunoelectrophoresis (Figure 18). Within each group, all six *pdi1::TRP1* disruptants of DXY1 *trp1Δ* [pDB2976], DXY1 *tp1Δ* [pDB2978], DXY1 *tp1Δ* [pDB2980], DXY1 *tp1Δ* [pDB2977] and DXY1 *tp1Δ* [pDB2979] had very similar rHA productivities. Only the six *pdi1::TRP1* disruptants of DXY1 *tp1Δ* [pDB2981] showed variation in rHA expression titre. The six *pdi1::TRP1* disruptants indicated in Figure 18 were spread onto YEPD agar to isolate single colonies and then re-patched onto BMMD agar.

Three single celled isolates of DXY1 *trp1Δ pdi1::TPP1* [pDB2976], DXY1 *trp1Δ pdi1::TRP1* [pDB2978], DXY1 *tp1Δ pdi1::TRP1* [pDB2980], DXY1 *tp1Δ pdi1::TRP1* [pDB2977], DXY1 *trp1Δ pdi1::TRP1* [pDB2979] and DXY1 *tp1Δ pdi1::TRP1* [pDB2981] along with DXY1 [pDB2244], DXY1 [pDB2976], DXY1 [pDB2978], DXY1 [pDB2980], DXY1 [pDB2977], DXY1 [pDB2979] and DXY1 [pDB2981] were inoculated into 10mL YEPD in 50mL shake flasks and incubated in an orbital shaker at 30°C, 200rpm for 4-days. Culture supernatants were harvested and the recombinant albumin titres were compared by rocket immunoelectrophoresis (Figure 19). The thirteen wild type *PDI1* and *pdi1::TRP1* disruptants indicated in Figure 19 were spread onto YEPD agar to isolate single colonies. One hundred single celled colonies from each strain were then re-patched onto BMMD agar or YEPD agar containing a goat anti-HSA antibody to detect expression of recombinant albumin (Sleep et al., 1991, Bio/Technology, 9, 183-187) and the Leu+/rHA+, Leu+/rHA-, Leu-/rHA+ or Leu-/rHA- phenotype of each colony scored (Table 13).

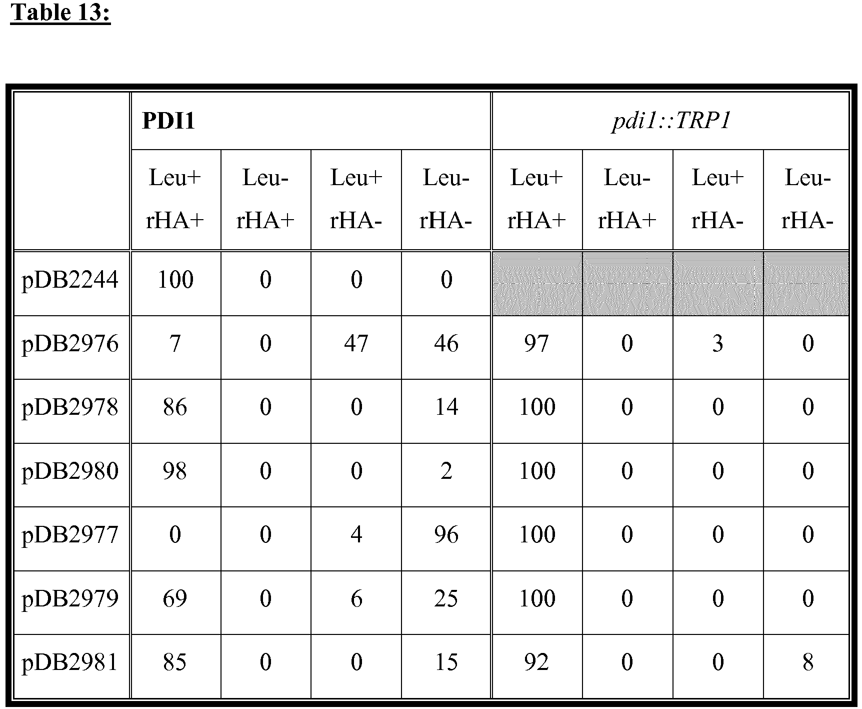

These data indicate plasmid retention is increased when the *PDI1* gene is used as a selectable marker on a plasmid in a host strain having no chromosomally encoded PDI, even in a non-selective medium such as the exemplified rich medium.

### Example 13

### Construction of the pSAC35-based expression vectors pDB3175-pDB3182 for co-expression of PDI1 with recombinant human albumin or transferrin (N413Q, N611Q) from the SnaBI/NotI-site in the 2µm UL-region

The *Not*I expression cassette from the pSAC35-based expression vector, pAYE316 (Sleep et al, 1991, Biotechnology (N Y), 9, 183-187), designed for the secretion of recombinant human albumin, was cloned into the unique *Not*I-site of the *E. coli* cloning vector pBST(+) (Sleep et al, 2001, Yeast, 18, 403-421) to produce plasmid pQC262e. pQC262e was subsequently modified by site-directed mutagenesis (Kunkel et al, 1987, Methods Enzymol., 154, 367-382) with oligonucleotide LRE49 (Table 14) to introduce a unique *Asp*718I-site immediately upstream of the *Not*I-site at the *ADH1* terminator region of the expression cassette.

**Table 14: Mutagenic Oligonucleotide**

| **Primer** | **Description** | **Sequence** |
|---|---|---|
| LRE49 | Mutagenic 45mer *ADH1* terminator region | |

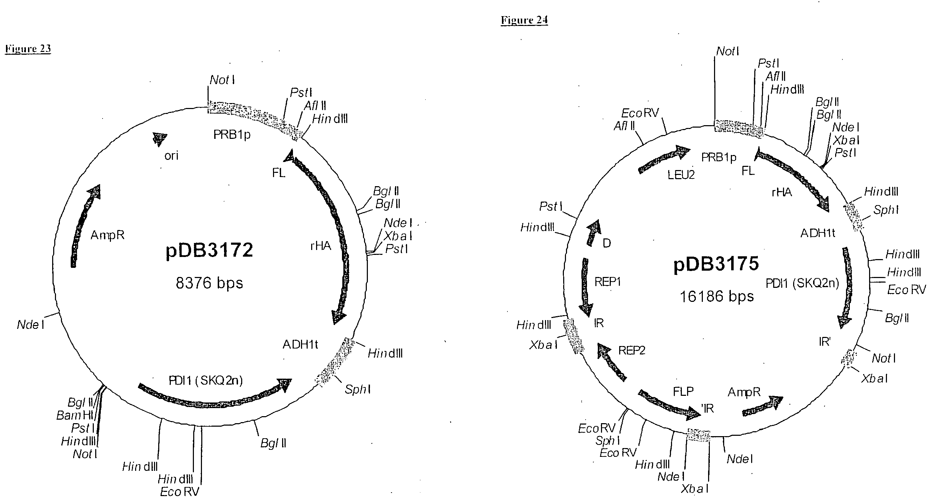

This produced plasmid pAYE560 (Figure 21). The *S. cerevisiae* SKQ2n *PDI1* gene with the long (~210-bp) promoter was isolated on 1.96-kb *Sma*I fragment from pDB2952 (Figure 46 in WO 2005/061719). The pDB2952 *PDI1* fragment was cloned into the unique *Asp*718I-site of pAYE560, following digestion with *Asp*718I, filling the 3'-recessed ends using T4 DNA polymerase, and calf intestinal alkaline phosphatase treatment of the blunt-ended product. This produced plasmids pDB3171 (Figure 22) with the *PDI1* gene transcribed in the same direction as rHA, and pDB3172 (Figure 23) with converging transcription of the *PDI1* and rHA genes.

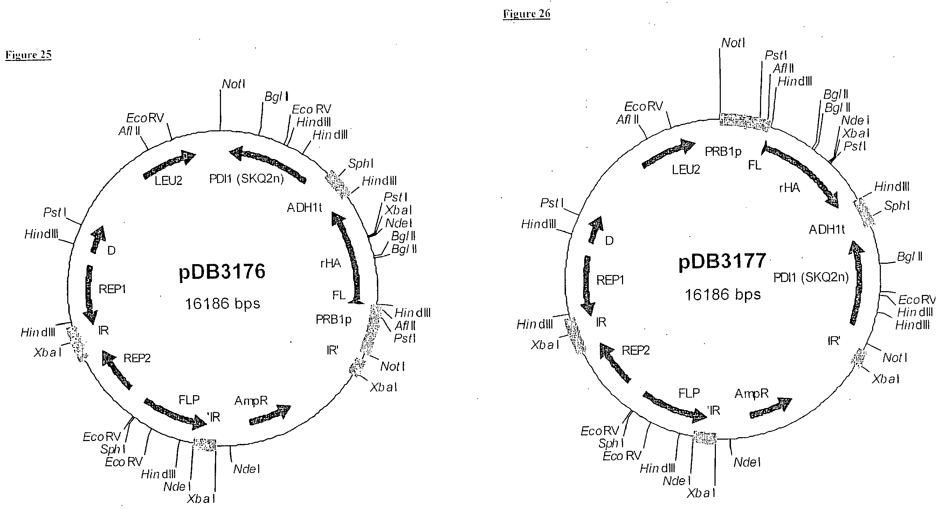

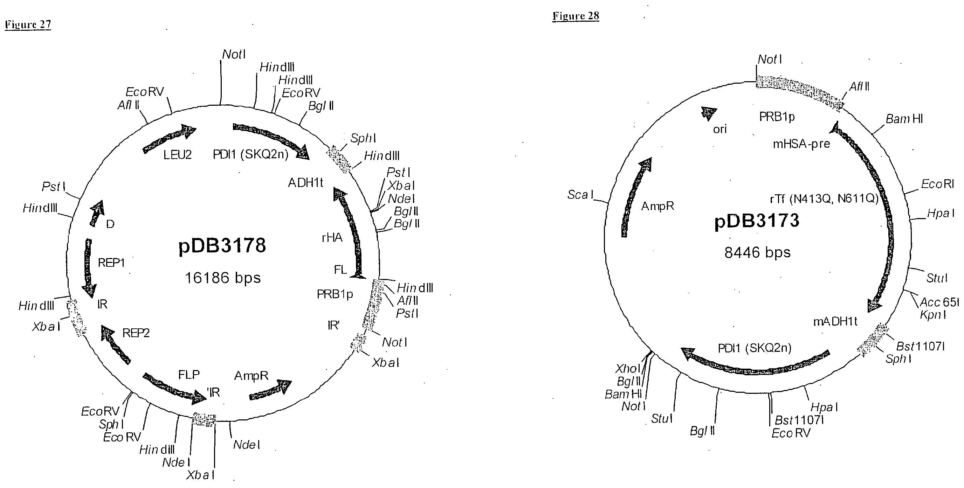

pDB3175 and pDB3176 (Figures 24 and 25) were produced by cloning the ~ 5.1-kb rHA→*PDI1*→ *Not*I expression cassette from pDB3171 into pSAC35, which had been digested with *Not*I and calf intestinal alkaline phosphatase. pDB3177 and pDB3178 (Figures 26 and 27) were produced similarly by cloning the ~ 5.1-kb *Not*I rHA→←*PDI1* expression cassette from pDB3172 into pSAC35 digested with *Not*I and calf intestinal alkaline phosphatase.

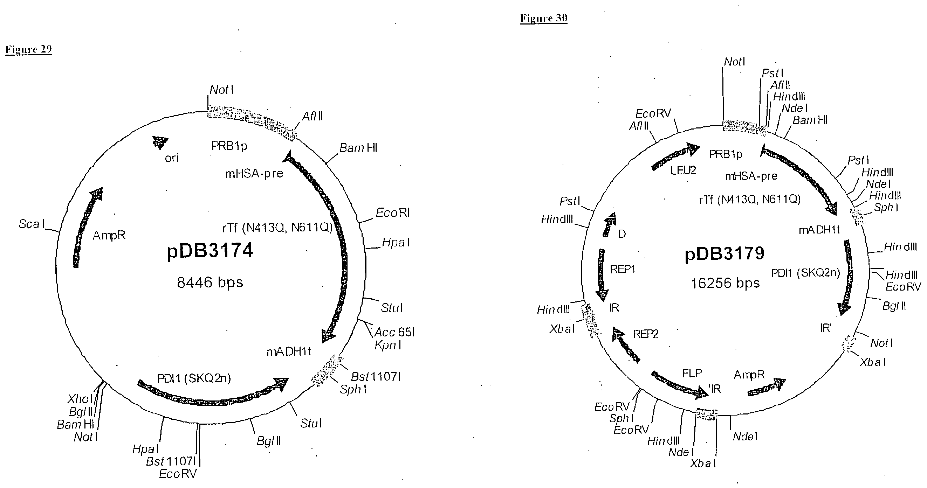

To construct *Not*I expression cassettes for co-expression of recombinant unglycosylated human transferrin (N413Q, N611Q) and the *S. cerevisiae* SKQ2n *PDI1* gene with the long (~210-bp) promoter, the ~ 6.1-kb *Afl*II*-Sph*I fragment from pDB3171 was ligated with the ~ 2.4-kb *Afl*II*-Sph*I fragment from pDB2928 (Figure 11 of WO 2005/061718), thus replacing the rHA coding and adjacent sequences with those for transferrin (N413Q, N611Q) secretion. This produced plasmids pDB3173 (Figure 28) with the *PDI1* gene transcribed in the same direction as rTf (N413Q, N611Q), and pDB3174 (Figure 29) with converging transcription of the *PDI1* and rTf (N413Q, N611Q) genes.

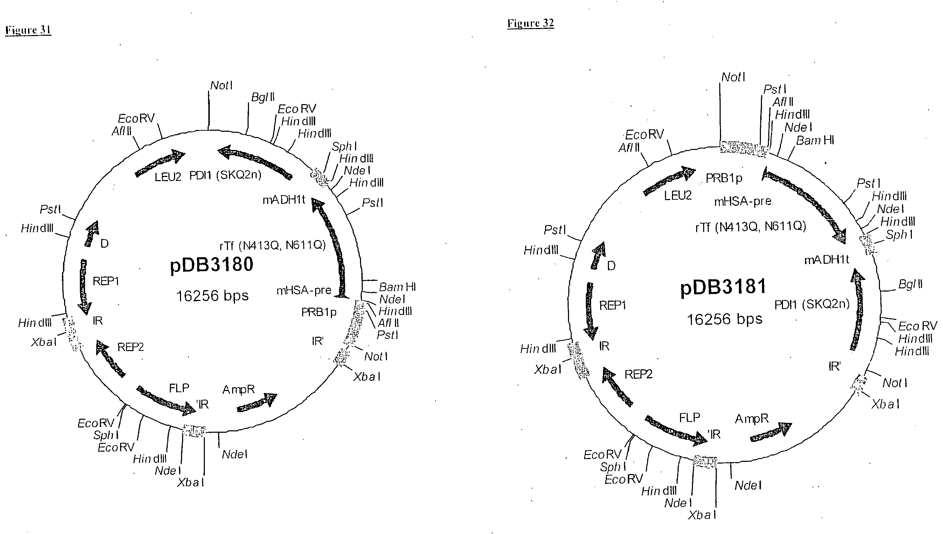

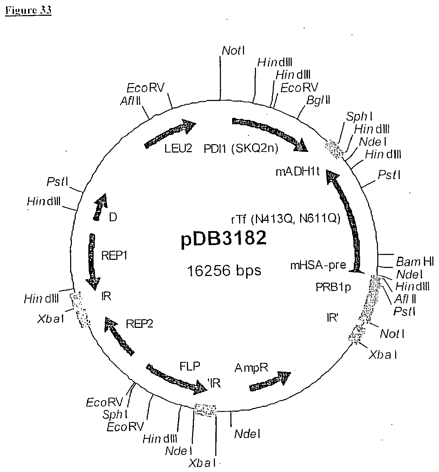

pDB3179 and pDB3180 (Figures 30 and 31) were produced by cloning the ~ 5.2-kb rTf→ *PDI1→ Not*I expression cassette from pDB3173 into pSAC35, which had been digested with *Not*I and calf intestinal alkaline phosphatase. pDB3181 and pDB3182 (Figures 32 and 33) were produced by cloning the ~ 5.2-kb *Not*I rTf→←*PDI1* expression cassette from pDB3174 into pSAC35 digested with *Not*I and calf intestinal alkaline phosphatase.

### Example 14

### S. cerevisiae PDI1 at the SnaBI/NotI-site in the UL-region of pSAC35-based expression vectors as a selectable marker in S. cerevisiae strain DXY1 Δtrp1 pdi1::TRP1

The yeast strains DXY1 (Kerry-Williams et al., 1998, Yeast, 14, 161-169) and DXY1 *Δtrp1* (see Example 13 of WO 2005/061718) were transformed to leucine prototrophy with pSAC35-based plasmids pAYE316, pDB3175, pDB3176, pDB3177, pDB3178, pDB2931, pDB3179, pDB3180, pDB3181 and pDB3182 using a modified lithium acetate method (Sigma yeast transformation kit, YEAST-1, protocol 2; (Ito et al, 1983, J. Bacteriol., 153, 163; Elble, 1992, Biotechniques, 13, 18)). Transformants were selected on BMMD-agar plates with appropriate supplements, and were subsequently patched out on BMMD-agar plates with appropriate supplements.

DXY1 *Δtrp1* [pDB3175], DXY1 *Δtrp1* [pDB3176], DXY1 *Δtrp1* [pDB3177], DXY1 *Δtrp1* [pDB3178], DXY1 *Δtrp1* [pDB3179], DXY1 *Δtrp1* [pDB3180], DXY1 *Δtrp1* [pDB3181], and DXY1 *Δtrp1* [pDB3182] were transformed to tryptophan prototrophy using the modified lithium acetate method (Sigma yeast transformation kit, YEAST-1, protocol 2; (Ito et al, 1983, J. Bacteriol., 153, 163; Elble, 1992, Biotechniques, 13, 18)) with a 1.41-kb *pdi1::TRP1* disrupting DNA fragment isolated from pDB3078 by digestion with *Not*I/*Pst*I (see Example 13 of WO 2005/061718). Transformants were selected on BMMD-agar plates and were subsequently patched out on BMMD-agar plates.

Disruption of the *PDI1* gene with the *TRP1* marker was confirmed by diagnostic PCR amplification of an approximately 810-bp product using oligonucleotide primers CF247 and DS236 (Table 15). CF247 binds in the *PDI1* promoter region upstream of the disruption site and DS236 binds within the *TRP1* gene.

**Table 15: Oligonucleotide Sequencing Primers**

| **Primer** | **Description** | **Sequence** |
|---|---|---|
| CF247 | *PDI1* promoter region, 20mer | 5'-GCGCGTTTTCATTAGTGCCC-3' |
| DS236 | *TRP1* coding region, 19mer | 5'-AAATAGTTCAGGCACTCCG-3' |

DXY1 *Δtrp1*, DXY1 *Δtrp1* containing pDB3175-pDB3182 and putative *pdi1::TRP1*-disruptants were inoculated into 10mL YEPD in 50mL shake flasks, and incubated in an orbital shaker at 30°C, 200rpm for 4-days. Genomic DNA was prepared (Lee, 1992, Biotechniques, 12, 677) from the biomass for subsequent use as template DNA in diagnostic PCR.

0.5µL template genomic DNA, 2.5µL 10xBuffer (Fast Start Taq+Mg, (Roche)), 0.5µL 10mM dNTP's, 2.5µL each primer (2µM), 0.2µL Fast Start Taq were mixed and made up to 25µL with H₂O. PCRs were performed using a Dyad™ DNA Engine Peltier thermal cycler (GRI) as follows; denaturation at 95°C for 4 mins, then 25 cycles of 95°C for 30s, 55°C for 30s and 72°C for 1 min, followed by extension at 72°C for 10 mins. A PCR product of ~810-bp was only amplified from DNA containing the *TRP1* gene integrated at the *pdi1* locus. DXY1 *Δtrp1 pdi1::TRP1* containing pDB3175-pDB3182 were successfully identified. As expected, no PCR product was generated for the controls DXY1 *Δtrp1* or DXY1 *Δtrp1* containing pDB3175-pDB3182.

DXY1 containing pDB3175-pDB3182 were compared with DXY1 *Δtrp1 pdi1::TRP1* containing pDB3175-pDB3182 for plasmid stability and secretion of recombinant human albumin or transferrin (N413Q, N611Q) in YEPD shake flask culture. 10mL YEPD shake flasks were inoculated with the above strains and grown for 4-days at 30°C, 200rpm. Samples were spread onto YEPD-agar plates and grown to single colonies. Fifty randomly selected colonies were patched out in replica onto BMMD and YEPD plates and incubated at 30°C. Plasmid stability was scored as the percentage of colonies able to grow on both media. The results shown in Tables 16 and 17 demonstrated that all of the plasmids pDB3175-pDB3182 were less than 100% stable in DXY1, regardless of the relative orientations of the *PDI1* and rTf/rHA genes cloned at the *Sna*BI/*Not*I-site. However, 100% plasmid stability was determined in all cases in DXY1 *Δtrp1 pdi1::TRP1* containing pDB3175-pDB3182. Hence, use of *PDI1* as the sole selectable marker at the *Sna*BI/*Not*I-site of pSAC35-based vectors resulted in 100% plasmid stability in rich media.

**Table 16: Plasmid stability of pSAC35-based vectors containing a recombinant albumin gene and the S. cerevisiae PDI1 gene at the SnaBI/NotI-site in the UL-region in strains DXY1 and DXY1 Δtrp1 pdi1::TRP1. 10mL YEPD shake flasks were inoculated with DXY1 [pDB3175], DXY1 [pDB3176], DXY1 [pDB3177], DXY1 [pDB3178], DXY1 Δtrp1 pdi1::TRP1 [pDB3175], DXY1 Δtrp1 pdi1::TRP1 [pDB3176], DXY1 Δtrp1 pdi1::TRP1 [pDB3177], and DXY1 Δtrp1 pdi1::TRP1 [pDB3178] were grown for 4-days at 30°C, 200rpm. Samples were spread onto YEPD-agar plates and grown to single colonies. Fifty randomly selected colonies were patched out in replica onto BMMD and YEPD plates and incubated at 30°C. Plasmid stability was scored as the percentage of colonies able to grow on both media.**

| **Plasmid** | ***SnaB*I/*Not*I Cassette*** | **Plasmid Stability (% Prototrophs)** | | |
|---|---|---|---|---|
| | | DXY1 | DXY1 *Δtrp1* | DXY1 *Δtrp1 pdi1::TRP1* |
| pDB3175 | *LEU2* rHA *PDI1* | 96 | 0 | 100 |
| | → → → | | | |
| pDB3176 | *LEU2 PDI1* rHA | 68 | 0 | 100 |
| | → ← ← | | | |
| pDB3177 | *LEU2* rHA *PDI1* | 86 | 0 | 100 |
| | → → ← | | | |
| pDB3178 | *LEU2 PDI1* rHA | 28 | 0 | 100 |
| | → → ← | | | |

| | | | | |
|---|---|---|---|---|
| * Arrows indicate the direction of transcription relative to the *LEU2* gene. | | | | |

**Table 17: Plasmid stability of pSAC35-based vectors containing a recombinant transferrin gene and the S. cerevisiae PDI1 gene at the SnaBI/NotI-site in the UL-region in strains DXY1 and DXY1 Δtrp1 pdi1::TRP1. 10mL YEPD shake flasks were inoculated with DXY1 [pDB3179], DXY1 [pDB3180], DXY1 [pDB3181], DXY1 [pDB3182], DXY1 Δtrp1 pdi1::TRP1 [pDB3179], DXY1 Δtrp1 pdi1::TRP1 [pDB3180], DXY1 Δtrp1 pdi1::TRP1 [pDB3181], and DXY1 Δtrp1 pdi1::TRP1 [pDB3182] were grown for 4-days at 30°C, 200rpm. Samples were spread onto YEPD-agar plates and grown to single colonies. Fifty randomly selected colonies were patched out in replica onto BMMD and YEPD plates and incubated at 30°C. Plasmid stability was scored as the percentage of colonies able to grow on both media.**

| **Plasmid** | ***Sna*BI/*Not*I Cassette** | **Plasmid Stability (% Prototrophs)** | | |
|---|---|---|---|---|
| | | DXY1 | DXY1 *Δtrp1* | DXY1 *pdi1::TRP1* |
| pDB3179 | *LEU2* rTf *PDI1* | 56 | 0 | 100 |
| | → → ← | | | |
| pDB3180 | *LEU2 PDI1* rTf | 36 | 0 | 100 |
| pDB3181 | *LEU2* rTf *PDI1* | 28 | 0 | 100 |
| | → → ← | | | |
| pDB3182 | *LEU2 PDI1* rTf | 15 | 0 | 100 |
| | → → ← | | | |

| | | | | |
|---|---|---|---|---|
| * Arrows indicate the direction of transcription relative to the *LEU2* gene. | | | | |

DXY1 containing pDB3175-pDB3182 were compared with DXY1 *Δtrp1 pdi1::TRP1* containing pDB3175-pDB3182 for the secretion of recombinant human albumin and transferrin (N413Q, N611Q). Figure 34 shows that following disruption of the genomic *PDI1* gene with *TRP1* to increase plasmid stability to 100% (Table 16), there was a reduced recombinant human albumin titre for *PDI1* inserted at the *Sna*BI-site. However, this is more than compensated for by the increased genetic stability following disruption of *PDI1* in the genome, which increases the reproducibility and reliability of heterologous protein secretion, resulting in a more useful industrial organism for application in consistent protein production, especially in prolonged cultivation, such as fill and draw fermentation or continuous culture fermentation campaigns. Furthermore, when *PDI1* was located at the *Xcm*I-site after *REP2* of pSAC35 (Example 12) there was no significant decrease in rHA titre following disruption of the genomic *PDI1* gene with *TRP1* to increase plasmid stability (Table 13). This suggests that the *Xcm*I-site was preferred (but not essential) compared to the *Sna*BI-site for *PDI1* on 2µm-based plasmids expressing rHA. However, *PDI1* expression can be modulated, for example by altering the length of the *PDI1* promoter (Example 7) such that an increase in recombinant protein secretion is observed. In the above experiment the long *PDI1* promoter was used, which was not the preferred promoter length for optimal rHA secretion in the closely related high rHA producing strain, DS569, where a short promoter resulted in increased rHA secretion. In the case of genomic *PDI1* disruption in DXY1 [pDB2977] containing a long *PDI1* promoter in *PDI1* at the *Xcm*I-site after *REP2* of pSAC35 (Figure 19), the analysis included three individual isolates (not triplicates of a single isolate as shown in Figure 34), and demonstrated no decrease in rHA titre. Furthermore, the increased consistency and reproducibility is clearly demonstrated for these cultures following disruption of the genomic *PDI1* gene.

In Figure 35 a large increase in recombinant transferrin secretion was observed between DXY1 [pDB2931] without *PDI1* on the pSAC35-based vector (which itself was not 100% stable), and strains containing plasmids pDB3179-pDB3182, with *PDI1* at the *Sna*BI/*Not*I-site. In DXY1 *Δtrp1 pdi1::TRP1* containing plasmids pDB3179-pDB3182, *PDI1* on the plasmid was acting as the sole selectable marker and resulted in improved genetic stability (see Table 17) where 100% plasmid stability was observed without any decrease in recombinant transferrin secretion. Hence, by placing *PDI1* on the transferrin expression plasmid and disrupting *PDI1* in the genome, the overall effect has been to increase secretion of the recombinant protein and also to improve the genetic stability of the production organism.

## Claims

1. A method for producing desired protein comprising:
(a) providing a fungal or yeast host cell comprising a first recombinant gene encoding a protein comprising the sequence of Orm2p or a variant or fragment thereof having equivalent chaperone-like activity and a second gene, such as a second recombinant gene, encoding a desired protein, with the proviso that the first and second genes are not present within the host cell on the same 2µm-family plasmid; and
(b) culturing the host cell in a culture medium to obtain expression of the first and second genes,
wherein
Orm2p has the following sequence: and the variant of Orm2p has at least 70% sequence identity to: and the fragment of Orm2p has at least 70% of the complete sequence of:

2. Use of a recombinant gene encoding a protein comprising the sequence of Orm2p or a variant or fragment thereof having equivalent chaperone-like activity to increase the expression of a desired protein in a fungal or yeast host cell, with the proviso that the recombinant gene encoding a protein comprising the sequence of Orm2p or a variant or fragment thereof and the gene encoding the desired protein are not co-expressed within the host cell from the same 2µm-family plasmid
wherein
Orm2p has the following sequence: and the variant of Orm2p has at least 70% sequence identity to: and the fragment of Orm2p has at least 70% of the complete sequence of:

3. A method or use according to claim 1 or 2 wherein either or both of the first and second genes are located on a plasmid.

4. A method or use according to any preceding claim wherein the first recombinant gene encoding a protein comprising the sequence of Orm2p or a variant or fragment thereof is located on a plasmid.

5. A method or use according to claim 1 or 2 wherein either or both of the first and second genes are integrated into the chromosome of the host cell.

6. A method or use according to any one of claims 1, 2 or 5 wherein the first recombinant gene encoding a protein comprising the sequence of Orm2p or a variant or fragment thereof is integrated into the chromosome of the host cell.

7. A method or use according to any one of claims 1 to 6 further comprising the step of purifying the thus expressed desired protein from the cultured host cell or the culture medium.

8. A method or use according to claim 7 further comprising the step of formulating the purified desired protein with a carrier or diluent and optionally presenting the thus formulated protein in a unit dosage form.

9. A method or use according to any preceding claim wherein the fungal or yeast host cell further comprises an additional chaperone protein or variant or fragment thereof, or gene encoding an additional chaperone protein or variant or fragment thereof.

10. A method or use according to claim 9 wherein the additional chaperone is selected from *PDI1, JEM1, AHA1*, *CCT2*, *CCT3*, *CCT4*, *CCT5*, *CCT6, CCT7*, *CCT8*, *CNS1*, *CPR3*, *CPR6*, *EPS1*, *ERO1*, *EUG1*, *FMO1*, *HCH1*, *HSP10*, *HSP12*, *HSP104*, *HSP26*, *HSP30*, *HSP42, HSP60*, *HSP78, HSP82, MDJ1*, *MDJ2*, *MPD1*, *MPD2*, *PFD1*, *ABC1*, *APJ1*, *ATP11*, *ATP12*, *BTT1*, *CDC37, CPR7*, *HSC82, KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UBI4*, *ORM1, PER1*, *PTC2, PSE1, HAC1* or truncated intronless *HAC1, TIM9, PAM18* or *TCP1* or a variant or fragment of any one of these.

11. A method or use according to any one of claims 1 to 10 wherein the desired protein:
(i) comprises a leader sequence effective to cause secretion from the host cell, such as yeast; and/or
(ii) is a eucaryotic protein, or a fragment or variant thereof, optionally a vertebrate or a fungal protein; and/or
(iii) comprises a sequence selected from albumin, a monoclonal antibody, an etoposide, a serum protein, antistasin, a tick anticoagulant peptide, transferrin, lactoferrin, endostatin, angiostatin, collagens, immunoglobulins, or immunoglobulin-based molecules or fragment of either (e.g. a dAb, Fab' fragments, F(ab')₂, scAb, scFv or scFv fragment), a Kunitz domain protein, interferons, interleukins, IL10, IL11, IL2, interferon α species and sub-species, interferon β species and sub-species, interferon γ species and sub-species, leptin, CNTF, CNTF_{Ax15}', IL1-receptor antagonist, erythropoietin and EPO mimics, thrombopoietin and thrombopoietin mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, α₁-antitrypsin, plasminogen activators, nerve growth factor, LACI, platelet-derived endothelial cell growth factor, glucose oxidase, serum cholinesterase, aprotinin, amyloid precursor protein, inter-alpha trypsin inhibitor, antithrombin III, apo-lipoprotein species, Protein C, Protein S, a metabolite, an antibiotic, or a variant or fragment of any of the above.

12. A method or use according to claim 11 wherein the desired protein comprises the sequence of albumin or a variant or fragment thereof.

13. A method or use according to claim 11 wherein the desired protein comprises the sequence of a transferrin family member, optionally transferrin or lactoferrin, or a variant or fragment thereof.

14. A method or use according to claim 11 wherein the desired protein is a desired heterologous protein that comprises a fusion protein, such as a fusion protein of albumin or a transferrin family member or a variant or fragment of either, fused directly or indirectly to the sequence of another protein.

15. A method or use according to any one of claims 1 to 14 wherein the host cell is a yeast cell, optionally a member of the *Saccharomyces*, *Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces,* or *Pichia* genus, such as *Saccharomyces cerevisiae*, *Kluyveromyces lactis*, *Pichia pastoris, Pichia membranaefaciens, Zygosaccharomyces rouxii*, *Zygosaccharomyces bailii*, *Zygosaccharomyces fermentati*, *Kluyveromyces drosphilarum, Pichia methanolica, Hansenula polymorpha, Arxula adeninivorans, Yarrowia lipolytica, Candida boidinii*, *Candida utilis* or
*Schizosaccharomyces pombe.*

16. A method or use according to any one of claims 1 to 15 where, when a 2µm-family plasmid is used:
(a) the plasmid is based on pSR1, pSB3 or pSB4 and the host cell is *Zygosaccharomyces rouxii;*
(b) the plasmid is based on pSB1 or pSB2 and the host cell is *Zygosaccharomyces bailli;*
(c) the plasmid is based on pSM1 and the host cell is *Zygosaccharomyces fermentati;*
(d) the plasmid is based on pKD1 and the host cell is *Kluyveromyces drosophilarum;*
(e) the plasmid is based on pPM1 and the host cell is *Pichia membranaefaciens;* or
(f) the plasmid is based on the 2µm plasmid and the host cell is *Saccharomyces cerevisiae* or *Saccharomyces carlsbergensis.*

17. A method or use according to claim 16 in which the plasmid is based on the 2µm plasmid and the host cell is *Saccharomyces cerevisiae* or *Saccharomyces carlsbergensis.*

18. A fungal or yeast host cell comprising (i) a plasmid comprising a first recombinant gene encoding a protein comprising the sequence of Orm2p or a variant or fragment thereof having equivalent chaperone-like activity and (ii) a second gene encoding a desired heterologous protein, with the proviso that the first and second recombinant genes are not present within the host cell on the same 2µm-family plasmid
wherein
Orm2p has the following sequence: and the variant of Orm2p has at least 70% sequence identity to: and the fragment of Orm2p has at least 70% of the complete sequence of:

19. A host cell according to claim 18 wherein the fungal or yeast host cell further comprises an additional chaperone protein or variant or fragment thereof, or gene encoding an additional chaperone protein or variant or fragment thereof.

20. A host cell according to claim 19 wherein the additional chaperone is selected from *PDI1, JEM1, AHA1*, *CCT2*, *CCT3*, *CCT4*, *CCT5*, *CCT6, CCT7*, *CCT8*, *CNS1*, *CPR3*, *CPR6*, *EPS1*, *ERO1*, *EUG1*, *FMO1*, *HCH1*, *HSP10*, *HSP12*, *HSP104*, *HSP26*, *HSP30*, *HSP42*, *HSP60*, *HSP78*, *HSP82*, *MDJ1*, *MDJ2*, *MPD1*, *MPD2*, *PFD1*, *ABC1*, *APJ1*, *ATP11*, *ATP12*, *BTT1*, *CDC37, CPR7*, *HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UB14*, *ORM1*, *PER1*, *PTC2, PSE1, HAC1* or truncated intronless *HAC1, TIM9, PAM18* or *TCP1* or a variant or fragment of any one of these.

21. A host cell according to any one of claims 18 to 20 wherein the desired protein:
(i) comprises a leader sequence effective to cause secretion from the host cell, such as yeast; and/or
(ii) is a eucaryotic protein, or a fragment or variant thereof, optionally a vertebrate or a fungal protein; and/or
(iii) comprises a sequence selected from albumin, a monoclonal antibody, an etoposide, a serum protein, antistasin, a tick anticoagulant peptide, transferrin, lactoferrin, endostatin, angiostatin, collagens, immunoglobulins, or immunoglobulin-based molecules or fragment of either (e.g. a dAb, Fab' fragments, F(ab')₂, scAb, scFv or scFv fragment), a Kunitz domain protein, interferons, interleukins, IL10, IL11, IL2, interferon α species and sub-species, interferon β species and sub-species, interferon γ species and sub-species, leptin, CNTF, CNTF_{Ax15}', IL1-receptor antagonist, erythropoietin and EPO mimics, thrombopoietin and thrombopoietin mimics, prosaptide, cyanovirin-N, 5-helix, T20 peptide, T1249 peptide, HIV gp41, HIV gp120, urokinase, prourokinase, tPA, hirudin, platelet derived growth factor, parathyroid hormone, proinsulin, insulin, glucagon, glucagon-like peptides, insulin-like growth factor, calcitonin, growth hormone, transforming growth factor β, tumour necrosis factor, G-CSF, GM-CSF, M-CSF, FGF, coagulation factors in both pre and active forms, including but not limited to plasminogen, fibrinogen, thrombin, pre-thrombin, pro-thrombin, von Willebrand's factor, α₁-antitrypsin, plasminogen activators, nerve growth factor, LACI, platelet-derived endothelial cell growth factor, glucose oxidase, serum cholinesterase, aprotinin, amyloid precursor protein, inter-alpha trypsin inhibitor, antithrombin III, apo-lipoprotein species, Protein C, Protein S, a metabolite, an antibiotic, or a variant or fragment of any of the above.

22. A host cell according to claim 21 wherein the desired protein comprises the sequence of albumin or a variant or fragment thereof.

23. A host cell according to claim 21 wherein the desired protein comprises the sequence of a transferrin family member, optionally transferrin or lactoferrin, or a variant or fragment thereof.

24. A host cell according to claim 21 wherein the desired protein is a desired heterologous protein that comprises a fusion protein, such as a fusion protein of albumin or a transferrin family member or a variant or fragment of either, fused directly or indirectly to the sequence of another protein.

25. A host cell according to any one of claims 18 to 24 wherein the host cell is a yeast cell, optionally a member of the *Saccharomyces, Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces*, or *Pichia* genus, such as *Saccharomyces cerevisiae*, *Kluyveromyces lactis, Pichia pastoris, Pichia membranaefaciens, Zygosaccharomyces rouxii*, *Zygosaccharomyces bailii, Zygosaccharomyces fermentati*, *Kluyveromyces drosphilarum, Pichia methanolica, Hansenula polymorpha, Arxula adeninivorans, Yarrowia lipolytica, Candida boidinii*, *Candida utilis* or *Schizosaccharomyces*
*pombe.*

26. A host cell according to any one of claims 18 to 25 where, when a 2µm-family plasmid is used:
(a) the plasmid is based on pSR1, pSB3 or pSB4 and the host cell is *Zygosaccharomyces rouxii;*
(b) the plasmid is based on pSB1 or pSB2 and the host cell is *Zygosaccharomyces bailli;*
(c) the plasmid is based on pSM1 and the host cell is *Zygosaccharomyces fermentati;*
(d) the plasmid is based on pKD1 and the host cell is *Kluyveromyces drosophilarum;*
(e) the plasmid is based on pPM1 and the host cell is *Pichia membranaefaciens;* or
(f) the plasmid is based on the 2µm plasmid and the host cell is *Saccharomyces cerevisiae* or *Saccharomyces carlsbergensis.*

27. A host cell according to claim 26 in which the plasmid is based on the 2µm plasmid and the host cell is *Saccharomyces cerevisiae* or *Saccharomyces carlsbergensis.*

## Patentansprüche

1. Verfahren zum Herstellen von gewünschtem Protein, umfassend:
(a) Bereitstellen einer Pilz- oder Hefewirtszelle, die ein erstes rekombinantes Gen, das ein Protein kodiert, das die Sequenz von Orm2p oder eine Variante oder ein Fragment davon mit äquivalenter chaperonartiger Aktivität und ein zweites Gen umfasst, wie ein zweites rekombinantes Gen, das ein gewünschtes Protein kodiert, mit der Maßgabe, dass die ersten und zweiten Gene nicht innerhalb der Wirtszelle auf dem gleichen 2µm-Familie-Plasmid vorhanden sind; und
(b) Kultivieren der Wirtszelle in einem Kulturmedium, um Expression der ersten und zweiten Gene zu erhalten,
wobei
Orm2p die folgende Sequenz aufweist: und die Variante von Orm2p mindestens 70% Sequenzidentität aufweist zu: und das Fragment von Orm2p mindestens 70% der vollständigen Sequenz aufweist von:

2. Verwendung eines rekombinanten Gens, das ein Protein kodiert, das die Sequenz von Orm2p oder eine Variante oder ein Fragment davon mit äquivalenter chaperonartiger Aktivität umfasst, um die Expression eines gewünschten Proteins in einer Pilz- oder Hefewirtszelle zu erhöhen, mit der Maßgabe, dass das rekombinante Gen, das ein Protein kodiert, das die Sequenz von Orm2p oder eine Variante oder ein Fragment davon umfasst und das Gen, das das gewünschte Protein kodiert, nicht innerhalb der Wirtszelle vom gleichen 2µm-Familie-Plasmid koexprimiert werden
wobei
Orm2p die folgende Sequenz aufweist: und die Variante von Orm2p mindestens 70% Sequenzidentität aufweist zu: und das Fragment von Orm2p mindestens 70% der vollständigen Sequenz aufweist von:

3. Verfahren oder Verwendung nach Anspruch 1 oder 2, wobei eines oder beide der ersten und zweiten Gene auf einem Plasmid gelegen sind.

4. Verfahren oder Verwendung nach einem beliebigen voranstehenden Anspruch, wobei das erste rekombinante Gen, das ein Protein kodiert, das die Sequenz von Orm2p oder eine Variante oder ein Fragment davon umfasst, auf einem Plasmid gelegen ist.

5. Verfahren oder Verwendung nach Anspruch 1 oder 2, wobei eines oder beide der ersten und zweiten Gene in das Chromosom der Wirtszelle integriert sind.

6. Verfahren oder Verwendung nach einem beliebigen von Ansprüchen 1, 2 oder 5, wobei das erste rekombinante Gen, das ein Protein kodiert, das die Sequenz von Orm2p oder eine Variante oder ein Fragment davon umfasst, in das Chromosom der Wirtszelle integriert ist.

7. Verfahren oder Verwendung nach einem beliebigen der Ansprüche 1 bis 6, weiterhin umfassend den Schritt des Reinigens des so exprimierten gewünschten Proteins aus der kultivierten Wirtszelle oder dem Kulturmedium.

8. Verfahren oder Verwendung nach Anspruch 7, weiterhin umfassend den Schritt des Formulierens des gereinigten gewünschten Proteins mit einem Träger oder Verdünnungsmittel, und gegebenenfalls Präsentieren des so formulierten Proteins in einer Einheitsdosisform.

9. Verfahren oder Verwendung nach einem beliebigen voranstehenden Anspruch, wobei die Pilz- oder Hefewirtszelle weiterhin ein zusätzliches Chaperonprotein oder eine Variante oder ein Fragment davon, oder ein Gen, das ein zusätzliches Chaperonprotein oder eine Variante oder ein Fragment davon kodiert, umfasst.

10. Verfahren oder Verwendung nach Anspruch 9, wobei das zusätzliche Chaperon ausgewählt ist aus *PDI1, JEM1, AHA1*, *CCT2*, *CCT3*, *CCT4, CCT5*, *CCT6*, *CCT7*, *CCT8*, *CNS1*, *CPR3, CPR6, EPS1*, *ERO1*, *EUG1*, *FMO1*, *HCH1*, *HSP10*, *HSP12*, *HSP104*, *HSP26*, *HSP30, HSP42*, *HSP60, HSP78*, *HSP82, MDJ1*, *MDJ2*, *MPD1*, *MPD2*, *PFD1*, *ABC1*, *APJ1*, *ATP11*, *ATP12*, *BTT1*, *CDC37, CPR7, HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UBI4*, *ORM1*, *PER1*, *PTC2, PSE1, HAC1* oder trunkiertem intronlosen *HAC1, TIM9, PAM18* oder *TCP1* oder einer Variante oder einem Fragment eines beliebigen dieser.

11. Verfahren oder Verwendung nach einem beliebigen der Ansprüche 1 bis 10, wobei das gewünschte Protein:
(i) eine Leader-Sequenz umfasst, die wirksam ist, Sekretion aus der Wirtszelle, wie Hefe, zu verursachen; und/oder
(ii) ein eukaryotisches Protein, oder ein Fragment oder eine Variante davon, gegebenenfalls ein Vertebraten- oder ein Pilzprotein ist; und/oder
(iii) eine Sequenz umfasst ausgewählt aus Albumin, einem monoklonalen Antikörper, einem Etoposid, einem Serumprotein, Antistasin, einem Zecken-Antigerinnungspeptid, Transferrin, Lactoferrin, Endostatin, Angiostatin, Kollagenen, Immunoglobulinen, oder auf Immunoglobulin basierenden Molekülen oder einem Fragment von einem davon (z.B. ein dAb, Fab'-Fragmente, F(ab')₂, scAb, scFv oder scFv-Fragment), einem Kunitz-Domänenprotein, Interferonen, Interleukinen, IL10, IL11, IL2, Interferon α-Arten und Unterarten, Interferon β-Arten und Unterarten, Interferon γ-Arten und Unterarten, Leptin, CNTF, CNTF_{Ax15}', IL1-Rezeptorantagonist, Erythropoietin und EPO-Imitatoren, Thrombopoietin und Thrombopoietin-Imitatoren, Prosaptid, Cyanovirin-N, 5-Helix, T20-Peptid, T1249-Peptid, HIV gp41, HIV gp120, Urokinase, Prourokinase, tPA, Hirudin, Plättchen-abgeleitetem Wachstumsfakor, Parathyroidhormon, Proinsulin, Insulin, Glucagon, glucagonartigen Peptiden, insulinartigem Wachstumsfaktor, Calcitonin, Wachstumshormon, transformierendem Wachstumsfaktor β, Tumornekrosefaktor, G-CSF, GM-CSF, M-CSF, FGF, Gerinnungsfaktoren in sowohl prä- als auch aktiven Formen, einschließlich, aber nicht beschränkt auf Plasminogen, Fibrinogen, Thrombin, Präthrombin, Prothrombin, von Willebrand-Faktor, α₁-Antitrypsin, Plasminogenaktivatoren, Nervenwachstumsfaktor, LACI, Plättchen-abgeleitetem Endothelzellen-Wachstumsfaktor, Glucoseoxidase, Serum-Cholinesterase, Aprotinin, amyloidem Vorläuferprotein, Inter-alpha-Trypsininhibitor, Antithrombin III, Apolipoproteinarten, Protein C, Protein S, einem Metabolit, einem Antibiotikum, oder einer Variante oder einem Fragment eines beliebigen der Vorstehenden.

12. Verfahren oder Verwendung nach Anspruch 11, wobei das gewünschte Protein die Sequenz von Albumin oder einer Variante oder eines Fragments davon umfasst.

13. Verfahren oder Verwendung nach Anspruch 11, wobei das gewünschte Protein die Sequenz eines Mitglieds der Transferrinfamilie, gegebenenfalls Transferrin oder Lactoferrin, oder eine Variante oder ein Fragment davon umfasst.

14. Verfahren oder Verwendung nach Anspruch 11, wobei das gewünschte Protein ein gewünschtes heterologes Protein ist, das ein Fusionsprotein umfasst, wie ein Fusionsprotein von Albumin oder einem Mitglied der Transferrinfamilie oder einer Variante oder einem Fragment einer davon, direkt oder indirekt mit der Sequenz eines anderen Proteins fusioniert.

15. Verfahren oder Verwendung nach einem beliebigen der Ansprüche 1 bis 14, wobei die Wirtszelle eine Hefezelle ist, gegebenenfalls ein Mitglied der Gattung *Saccharomyces, Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces*, oder *Pichia*, wie *Saccharomyces cerevisiae*, *Kluyveromyces lactis*, *Pichia pastoris, Pichia membranaefaciens, Zygosaccharomyces rouxii*, *Zygosaccharomyces bailii*, *Zygosaccharomyces fermentati*, *Kluyveromyces drosphilarum, Pichia methanolica, Hansenula polymorpha, Arxula adeninivorans, Yarrowia lipolytica, Candida boidinii, Candida utilis* oder *Schizosaccharomyces pombe.*

16. Verfahren oder Verwendung nach einem beliebigen der Ansprüche 1 bis 15, wobei, wenn ein 2µm-Familie-Plasmid verwendet wird:
(a) das Plasmid auf pSR1, pSB3 oder pSB4 basiert, und die Wirtszelle *Zygosaccharomyces rouxii* ist;
(b) das Plasmid auf pSB1 oder pSB2 basiert, und die Wirtszelle *Zygosaccharomyces bailli* ist;
(c) das Plasmid auf pSM1 basiert, und die Wirtszelle *Zygosaccharomyces fermentati* ist;
(d) das Plasmid auf pKD1 basiert, und die Wirtszelle *Kluyveromyces drosophilarum* ist;
(e) das Plasmid auf pPM1 basiert, und die Wirtszelle *Pichia membranaefaciens* ist; oder
(f) das Plasmid auf dem 2µm-Plasmid basiert, und die Wirtszelle *Saccharomyces cerevisiae* oder *Saccharomyces carlsbergensis* ist.

17. Verfahren oder Verwendung nach Anspruch 16, worin das Plasmid auf dem 2µm-Plasmid basiert, und die Wirtszelle *Saccharomyces cerevisiae* oder *Saccharomyces carlsbergensis* ist.

18. Pilz- oder Hefewirtszelle, umfassend (i) ein Plasmid, das ein erstes rekombinantes Gen umfasst, das ein Protein kodiert, das die Sequenz von Orm2p oder eine Variante oder ein Fragment davon mit äquivalenter chaperonartiger Aktivität umfasst, und (ii) ein zweites Gen, das ein gewünschtes heterologes Protein kodiert, mit der Maßgabe, dass die ersten und zweiten rekombinanten Gene nicht innerhalb der Wirtszelle auf dem gleichen 2µm-Familie-Plasmid vorhanden sind,
wobei
Orm2p die folgende Sequenz aufweist: und die Variante von Orm2p mindestens 70% Sequenzidentität aufweist zu: und das Fragment von Orm2p mindestens 70% der vollständigen Sequenz aufweist von:

19. Wirtszelle nach Anspruch 18, wobei die Pilz- oder Hefewirtszelle ein zusätzliches Chaperonprotein oder eine Variante oder ein Fragment davon, oder ein Gen, das ein zusätzliches Chaperonprotein oder eine Variante oder ein Fragment davon kodiert, umfasst.

20. Wirtszelle nach Anspruch 19, wobei das zusätzliche Chaperon ausgewählt ist aus *PDI1, JEM1, AHA1*, *CCT2*, *CCT3*, *CCT4*, *CCT5*, *CCT6*, *CCT7, CCT8*, *CNS1*, *CPR3, CPR6*, *EPS1*, *ERO1*, *EUG1*, *FMO1*, *HCH1*, *HSP10*, *HSP12*, *HSP104*, *HSP26*, *HSP30*, *HSP42*, *HSP60*, *HSP78*, *HSP82*, *MDJ1*, *MDJ2*, *MPD1*, *MPD2*, *PFD1*, *ABC1*, *APJ1*, *ATP11*, *ATP12*, *BTT1*, *CDC37, CPR7, HSC82*, *KAR2*, *LHS1*, *MGE1*, *MRS11*, *NOB1*, *ECM10*, *SSA1*, *SSA2*, *SSA3*, *SSA4*, *SSC1*, *SSE2*, *SIL1*, *SLS1*, *UBI4*, *ORM1*, *PER1*, *PTC2, PSE1, HAC1* oder trunkiertem intronlosen *HAC1, TIM9, PAM18* oder *TCP1* oder einer Variante oder einem Fragment eines beliebigen dieser.

21. Wirtszelle nach einem beliebigen der Ansprüche 18 bis 20, wobei das gewünschte Protein:
(i) eine Leader-Sequenz umfasst, die wirksam ist, Sekretion aus der Wirtszelle, wie Hefe, zu verursachen; und/oder
(ii) ein eukaryotisches Protein, oder ein Fragment oder eine Variante davon, gegebenenfalls ein Vertebraten- oder ein Pilzprotein ist; und/oder
(iii) eine Sequenz umfasst ausgewählt aus Albumin, einem monoklonalen Antikörper, einem Etoposid, einem Serumprotein, Antistasin, einem Zecken-Antigerinnungspeptid, Transferrin, Lactoferrin, Endostatin, Angiostatin, Kollagenen, Immunoglobulinen, oder auf Immunoglobulin basierenden Molekülen oder einem Fragment von einem davon (z.B. ein dAb, Fab'-Fragmente, F(ab')₂, scAb, scFv oder scFv-Fragment), einem Kunitz-Domänenprotein, Interferonen, Interleukinen, IL10, IL11, IL2, Interferon α-Arten und Unterarten, Interferon β-Arten und Unterarten, Interferon γ- Arten und Unterarten, Leptin, CNTF, CNTF_{Ax15}', IL1-Rezeptorantagonist, Erythropoietin und EPO-Imitatoren, Thrombopoietin und Thrombopoietin-Imitatoren, Prosaptid, Cyanovirin-N, 5-Helix, T20-Peptid, T1249-Peptid, HIV gp41, HIV gp120, Urokinase, Prourokinase, tPA, Hirudin, Plättchen-abgeleitetem Wachstumsfaktor, Parathyroidhormon, Proinsulin, Insulin, Glucagon, glucagonartigen Peptiden, insulinartigem Wachstumsfaktor, Calcitonin, Wachstumshormon, transformierendem Wachstumsfaktor β, Tumornekrosefaktor, G-CSF, GM-CSF, M-CSF, FGF, Gerinnungsfaktoren in sowohl prä- als auch aktiven Formen, einschließlich, aber nicht beschränkt auf Plasminogen, Fibrinogen, Thrombin, Präthrombin, Prothrombin, von Willebrand-Faktor, α₁-Antitrypsin, Plasminogenaktivatoren, Nervenwachstumsfaktor, LACI, Plättchen-abgeleitetem Endothelzellen-Wachstumsfaktor, Glucoseoxidase, Serum-Cholinesterase, Aprotinin, amyloidem Vorläuferprotein, Inter-alpha-Trypsininhibitor, Antithrombin III, Apolipoproteinarten, Protein C, Protein S, einem Metabolit, einem Antibiotikum, oder einer Variante oder einem Fragment eines beliebigen der Vorstehenden.

22. Wirtszelle nach Anspruch 21, wobei das gewünschte Protein die Sequenz von Albumin oder einer Variante oder einem Fragment davon umfasst.

23. Wirtszelle nach Anspruch 21, wobei das gewünschte Protein die Sequenz eines Mitglieds der Transferrinfamilie, gegebenenfalls Transferrin oder Lactoferrin, oder eine Variante oder ein Fragment davon umfasst.

24. Wirtszelle nach Anspruch 21, wobei das gewünschte Protein ein gewünschtes heterologes Protein ist, das ein Fusionsprotein umfasst, wie ein Fusionsprotein von Albumin oder einem Mitglied der Transferrinfamilie oder einer Variante oder einem Fragment einer davon, direkt oder indirekt mit der Sequenz eines anderen Proteins fusioniert.

25. Wirtszelle nach einem beliebigen der Ansprüche 18 bis 24, wobei die Wirtszelle eine Hefezelle ist, gegebenenfalls ein Mitglied der Gattung *Saccharomyces*, *Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces*, oder *Pichia*, wie *Saccharomyces cerevisiae*, *Kluyveromyces lactis*, *Pichia pastoris, Pichia membranaefaciens, Zygosaccharomyces rouxii*, *Zygosaccharomyces bailii*, *Zygosaccharomyces fermentati*, *Kluyveromyces drosphilarum, Pichia methanolica, Hansenula polymorpha, Arxula adeninivorans, Yarrowia lipolytica, Candida boidinii, Candida utilis* oder *Schizosaccharomyces pombe.*

26. Wirtszelle nach einem beliebigen der Ansprüche 18 bis 25, wobei, wenn ein 2µm-Familie-Plasmid verwendet wird:
(a) das Plasmid auf pSR1, pSB3 oder pSB4 basiert, und die Wirtszelle *Zygosaccharomyces rouxii* ist;
(b) das Plasmid auf pSB1 oder pSB2 basiert, und die Wirtszelle *Zygosaccharomyces bailli* ist;
(c) das Plasmid auf pSM1 basiert, und die Wirtszelle *Zygosaccharomyces fermentati* ist;
(d) das Plasmid auf pKD1 basiert, und die Wirtszelle *Kluyveromyces drosophilarum* ist;
(e) das Plasmid auf pPM1 basiert, und die Wirtszelle *Pichia membranaefaciens* ist; oder
(f) das Plasmid auf dem 2µm-Plasmid basiert, und die Wirtszelle *Saccharomyces cerevisiae* oder *Saccharomyces carlsbergensis* ist.

27. Wirtszelle nach Anspruch 26, worin das Plasmid auf dem 2µm-Plasmid basiert, und die Wirtszelle *Saccharomyces cerevisiae* oder *Saccharomyces carlsbergensis* ist.

## Revendications

1. Méthode pour la production d'une protéine désirée, comprenant :
(a) fournir une cellule hôte fongique ou de levure comprenant un premier gène recombinant codant pour une protéine comprenant la séquence d'Orm2p ou un variant ou un fragment de celle-ci, ayant une activité de type chaperon équivalente et un deuxième gène, tel qu'un deuxième gène recombinant, codant pour une protéine désirée, à condition que les premier et deuxième gènes ne soient pas présents à l'intérieur de la cellule hôte sur le même plasmide de la famille 2µm ; et
(b) cultiver la cellule hôte dans un milieu de culture pour obtenir l'expression des premier et deuxième gènes,
dans laquelle
Orm2p possède la séquence suivante : et le variant d'Orm2p possède une identité de séquence d'au moins 70 % avec : et le fragment d'Orm2p possède au moins 70 % de la séquence complète :

2. Utilisation d'un gène recombinant codant pour une protéine comprenant la séquence d'Orm2p ou un variant ou un fragment de celle-ci, ayant une activité de type chaperon équivalente, afin d'augmenter l'expression d'une protéine désirée dans une cellule hôte fongique ou de levure, à condition que le gène recombinant codant pour une protéine comprenant la séquence d'Orm2p ou un variant ou un fragment de celle-ci et le gène codant pour la protéine désirée ne soient pas co-exprimés à l'intérieur de la cellule hôte provenant du même plasmide de la famille 2µm
dans laquelle
Orm2p possède la séquence suivante : et le variant d'Orm2p possède une identité de séquence d'au moins 70 % avec : et le fragment d'Orm2p possède au moins 70 % de la séquence complète :

3. Méthode ou utilisation selon la revendication 1 ou 2, dans laquelle l'un ou l'autre des premier et deuxième gènes sont localisés sur un plasmide.

4. Méthode ou utilisation selon l'une quelconque des revendications précédentes, dans laquelle le premier gène recombinant codant pour une protéine comprenant la séquence d'Orm2p ou un variant ou un fragment de celle-ci est localisé sur un plasmide.

5. Méthode ou utilisation selon la revendication 1 ou 2, dans laquelle l'un ou l'autre des premier et deuxième gènes sont intégrés dans le chromosome de la cellule hôte.

6. Méthode ou utilisation selon l'une quelconque des revendications 1, 2 ou 5, dans laquelle le premier gène recombinant codant pour une protéine comprenant la séquence d'Orm2p ou un variant ou un fragment de celle-ci est intégré dans le chromosome de la cellule hôte.

7. Méthode ou utilisation selon l'une quelconque des revendications 1 à 6, comprenant en outre l'étape consistant à purifier la protéine désirée ainsi exprimée à partir de la cellule hôte cultivée ou du milieu de culture.

8. Méthode ou utilisation selon la revendication 7, comprenant en outre l'étape consistant à formuler la protéine désirée purifiée avec un véhicule ou un diluant et éventuellement à présenter la protéine ainsi formulée dans une forme galénique unitaire.

9. Méthode ou utilisation selon l'une quelconque des revendications précédentes, dans laquelle la cellule hôte fongique ou de levure comprend en outre une protéine chaperon additionnelle, ou un variant ou un fragment de celle-ci, ou un gène codant pour une protéine chaperon additionnelle, ou un variant ou un fragment de celle-ci.

10. Méthode ou utilisation selon la revendication 9, dans laquelle la protéine chaperon additionnelle est choisie parmi *PDI1, JEM1, AHA1, CCT2, CCT3, CCT4, CET5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, MDJ1, MDJ2, MPD1, MPD2, PFDI, ABC1, APJ1, ATP11, ATP12, BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, PER1, PTC2, PSE1, HACI* ou *HAC1, TIM9, PAM18* ou *TCPI* tronquées sans intron, ou un variant ou un fragment de l'une quelconque de celles-ci.

11. Méthode ou utilisation selon l'une quelconque des revendications 1 à 10, dans laquelle la protéine désirée :
(i) comprend une séquence de tête efficace pour susciter la sécrétion à partir de la cellule hôte, telle qu'une levure ; et/ou
(ii) est une protéine eucaryote, ou un fragment ou un variant de celle-ci, éventuellement une protéine de vertébré ou une protéine fongique ; et/ou
(iii) comprend une séquence choisie parmi l'albumine, un anticorps monoclonal, un étoposide, une protéine sérique, l'antistasine, un peptide anticoagulant de tique, la transferrine, la lactoferrine, l'endostatine, l'angiostatine, les collagènes, les immunoglobulines, ou les molécules à base d'immunoglobuline ou un fragment de l'une de celles-ci (par exemple un dAb, des fragments Fab', F(ab')₂, scAb, scFv ou un fragment de scFv), une protéine du domaine de Kunitz, les interférons, les interleukines, IL10, IL11, IL2, les espèces et sous-espèces de l'interféron α, les espèces et sous-espèces de l'interféron β, les espèces et sous-espèces de l'interféron y, la leptine, CNTF, CNTF_{Ax15}, l'antagoniste du récepteur d'IL1, l'érythropoïétine, et les mimétiques d'EPO, la thrombopoïétine et les mimétiques de thrombopoïétine, le prosaptide, la cyanovirine-N, l'hélice 5, le peptide T20, le peptide T1249, gp41 du VIH, gp120 du VIH, l'urokinase, la pro-urokinase, tPA, l'hirudine, le facteur de croissance dérivé des plaquettes, l'hormone parathyroïdienne, la proinsuline, l'insuline, le glucagon, les peptides de type glucagon, le facteur de croissance de type insuline, la calcitonine, l'hormone de croissance, le facteur de croissance transformant β, le facteur de nécrose tumorale, G-CSF, GM-CSF, M-CSF, FGF, les facteurs de coagulation à la fois sous une préforme et sous une forme active, y compris mais sans y être limité, le plasminogène, le fibrinogène, la thrombine, la pré-thrombine, la pro-thrombine, le facteur de von Willebrand, l'α₁-antitrypsine, les activateurs du plasminogène, le facteur de croissance des nerfs, LACI, le facteur de croissance des cellules endothéliales dérivé des plaquettes, la glucose oxydase, la cholinestérase sérique, l'aprotinine, la protéine du précurseur amyloïde, l'inhibiteur de l'inter-alpha trypsine, l'antithrombine III, les espèces d'apo-lipoprotéine, la protéine C, la protéine S, un métabolite, un antibiotique, ou un variant ou un fragment de l'un quelconque des précédents.

12. Méthode ou utilisation selon la revendication 11, dans laquelle la protéine désirée comprend la séquence de l'albumine ou un variant ou un fragment de celle-ci.

13. Méthode ou utilisation selon la revendication 11, dans laquelle la protéine désirée comprend la séquence d'un membre de la famille de la transferrine, éventuellement la transferrine ou lactoferrine, ou un variant ou un fragment de celle-ci.

14. Méthode ou utilisation selon la revendication 11, dans laquelle la protéine désirée est une protéine hétérologue désirée qui comprend une protéine de fusion, telle qu'une protéine de fusion de l'albumine ou d'un membre de la famille de la transferrine ou un variant ou un fragment de l'un des deux, directement ou indirectement fusionnée à la séquence d'une autre protéine.

15. Méthode ou utilisation selon l'une quelconque des revendications 1 à 14, dans laquelle la cellule hôte est une cellule de levure, éventuellement un membre du genre *Saccharomyces, Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces,* ou *Pichia,* comme *Saccharomyces cerevisiae, Kluyveromyces lactis, Pichia pastoris, Pichia membranaefaciens, Zygosaccharomyces rouxii, Zygosaccharomyces bailii, Zygosaccharomyces fermentati, Kluyveromyces drosphilarum, Pichia methanolica, Hansenula polymorpha, Arxula adeninivorans, Yarrowia lipolytica, Candida boidinii, Candida utilis* ou *Schizosaccharomyces pombe.*

16. Méthode ou utilisation selon l'une quelconque des revendications 1 à 15, dans laquelle, quand un plasmide de la famille 2µm est utilisé :
(a) le plasmide est basé sur pSR1, pSB3 ou pSB4 et la cellule hôte est *Zygosaccharomyces rouxii* ;
(b) le plasmide est basé sur pSB1 ou pSB2 et la cellule hôte est *Zygosaccharomyces bailli* ;
(c) le plasmide est basé sur pSM1 et la cellule hôte est *Zygosaccharomyces fermentati* ;
(d) le plasmide est basé sur pKD1 et la cellule hôte est *Kluyveromyces drosophilarum* ;
(e) le plasmide est basé sur pPM1 et la cellule hôte est *Pichia membranaefaciens* _{;} ou
(f) le plasmide est basé sur le plasmide 2µm et la cellule hôte est *Saccharomyces cerevisiae* ou *Saccharomyces carlsbergensis.*

17. Méthode ou utilisation selon la revendication 16, dans laquelle le plasmide est basé sur le plasmide 2µm et la cellule hôte est *Saccharomyces cerevisiae* ou *Saccharomyces carlsbergensis.*

18. Cellule hôte fongique ou de levure comprenant (i) un plasmide comprenant un premier gène recombinant codant pour une protéine comprenant la séquence d'Orm2p ou un variant ou un fragment de celle-ci, ayant une activité de type chaperon équivalente, et (ii) un deuxième gène codant pour une protéine hétérologue désirée, à condition que les premier et deuxième gènes recombinants ne soient pas présents à l'intérieur de la cellule hôte sur le même plasmide de la famille 2µm
dans laquelle
Orm2p possède la séquence suivante : et le variant d'Orm2p possède une identité de séquence d'au moins 70 % avec : et le fragment d'Orm2p possède au moins 70 % de la séquence complète :

19. Cellule hôte selon la revendication 18, dans laquelle la cellule hôte fongique ou de levure comprend en outre une protéine chaperon additionnelle ou un variant ou un fragment de celle-ci, ou un gène codant pour une protéine chaperon additionnelle ou un variant ou un fragment de celle-ci.

20. Cellule hôte selon la revendication 19, dans laquelle la protéine chaperon additionnelle est choisie parmi *PDI1, JEM1, AHA1, CCT2, CCT3, CCT4, CET5, CCT6, CCT7, CCT8, CNS1, CPR3, CPR6, EPS1, ERO1, EUG1, FMO1, HCH1, HSP10, HSP12, HSP104, HSP26, HSP30, HSP42, HSP60, HSP78, HSP82, MDJ1, MDJ2, MPD1, MPD2, PFDI, ABC1, APJ1, ATP11, ATP12, BTT1, CDC37, CPR7, HSC82, KAR2, LHS1, MGE1, MRS11, NOB1, ECM10, SSA1, SSA2, SSA3, SSA4, SSC1, SSE2, SIL1, SLS1, UBI4, ORM1, PER1, PTC2, PSE1, HACI* ou *HAC1, TIM9, PAM18* ou *TCPI* tronquées sans intron, ou un variant ou un fragment de l'une quelconque de celles-ci.

21. Cellule hôte selon l'une quelconque des revendications 18 à 20, dans laquelle la protéine désirée :
(i) comprend une séquence de tête efficace pour susciter la sécrétion à partir de la cellule hôte, telle qu'une levure ; et/ou
(ii) est une protéine eucaryote, ou un fragment ou un variant de celle-ci, éventuellement une protéine de vertébré ou une protéine fongique ; et/ou
(iii) comprend une séquence choisie parmi l'albumine, un anticorps monoclonal, un étoposide, une protéine sérique, l'antistasine, un peptide anticoagulant de tique, la transferrine, la lactoferrine, l'endostatine, l'angiostatine, les collagènes, les immunoglobulines, ou les molécules à base d'immunoglobuline ou un fragment de l'une de celles-ci (par exemple un dAb, des fragments Fab', F(ab')₂, scAb, scFv ou un fragment de scFv), une protéine du domaine de Kunitz, les interférons, les interleukines, IL10, IL11, IL2, les espèces et sous-espèces de l'interféron α, les espèces et sous-espèces de l'interféron β, les espèces et sous-espèces de l'interféron γ, la leptine, CNTF, CNTF_{Ax15}, l'antagoniste du récepteur d'IL1, l'érythropoïétine, et les mimétiques d'EPO, la thrombopoïétine et les mimétiques de thrombopoïétine, le prosaptide, la cyanovirine-N, l'hélice 5, le peptide T20, le peptide T1249, gp41 du VIH, gp120 du VIH, l'urokinase, la pro-urokinase, tPA, l'hirudine, le facteur de croissance dérivé des plaquettes, l'hormone parathyroïdienne, la proinsuline, l'insuline, le glucagon, les peptides de type glucagon, le facteur de croissance de type insuline, la calcitonine, l'hormone de croissance, le facteur de croissance transformant β, le facteur de nécrose tumorale, G-CSF, GM-CSF, M-CSF, FGF, les facteurs de coagulation à la fois sous une préforme et sous une forme active, y compris mais sans y être limité, le plasminogène, le fibrinogène, la thrombine, la pré-thrombine, la pro-thrombine, le facteur de von Willebrand, l'α₁-antitrypsine, les activateurs du plasminogène, le facteur de croissance des nerfs, LACI, le facteur de croissance des cellules endothéliales dérivé des plaquettes, la glucose oxydase, la cholinestérase sérique, l'aprotinine, la protéine du précurseur amyloïde, l'inhibiteur de l'inter-alpha trypsine, l'antithrombine III, les espèces d'apo-lipoprotéine, la protéine C, la protéine S, un métabolite, un antibiotique, ou un variant ou un fragment de l'un quelconque des précédents.

22. Cellule hôte selon la revendication 21, dans laquelle la protéine désirée comprend la séquence de l'albumine ou un variant ou un fragment de celle-ci.

23. Cellule hôte selon la revendication 21, dans laquelle la protéine désirée comprend la séquence d'un membre de la famille de la transferrine, éventuellement la transferrine ou lactoferrine, ou un variant ou un fragment de celle-ci.

24. Cellule hôte selon la revendication 21, dans laquelle la protéine désirée est une protéine hétérologue désirée qui comprend une protéine de fusion, comme une protéine de fusion de l'albumine ou d'un membre de la famille de la transferrine ou un variant ou un fragment de l'un des deux, directement ou indirectement fusionnée à la séquence d'une autre protéine.

25. Cellule hôte selon l'une quelconque des revendications 18 à 24, dans laquelle la cellule hôte est une cellule de levure, éventuellement un membre du genre *Saccharomyces, Kluyveromyces, Arxula, Yarrowia, Candida, Schizosaccharomyces, Debaryomyces, Xanthophyllomyces, Geotrichum, Ashbya, Hortaea, Schwanniomyces, Trichosporon, Xanthophyllomyces,* ou *Pichia,* comme *Saccharomyces cerevisiae, Kluyveromyces lactis, Pichia pastoris, Pichia membranaefaciens, Zygosaccharomyces rouxii, Zygosaccharomyces bailii, Zygosaccharomyces fermentati, Kluyveromyces drosphilarum, Pichia methanolica, Hansenula polymorpha, Arxula adeninivorans, Yarrowia lipolytica, Candida boidinii, Candida utilis* ou *Schizosaccharomyces pombe.*

26. Cellule hôte selon l'une quelconque des revendications 18 à 25, dans laquelle, quand un plasmide de la famille 2µm est utilisé :
(a) le plasmide est basé sur pSR1, pSB3 ou pSB4 et la cellule hôte est *Zygosaccharomyces rouxii* ;
(b) le plasmide est basé sur pSB1 ou pSB2 et la cellule hôte est *Zygosaccharomyces bailli* ;
(c) le plasmide est basé sur pSM1 et la cellule hôte est *Zygosaccharomyces fermentati* ;
(d) le plasmide est basé sur pKD1 et la cellule hôte est *Kluyveromyces drosophilarum* ;
(e) le plasmide est basé sur pPM1 et la cellule hôte est *Pichia membranaefaciens* ; ou
(f) le plasmide est basé sur le plasmide 2µm et la cellule hôte est *Saccharomyces cerevisiae* ou *Saccharomyces carlsbergensis.*

27. Cellule hôte selon la revendication 26, dans laquelle le plasmide est basé sur le plasmide 2µm et la cellule hôte est *Saccharomyces cerevisiae* ou *Saccharomyces carlsbergensis.*


### SEQUENCE LISTING

<110> NOVOZYMES BIOPHARMA DK A/S
<120> GENE EXPRESSION TECHNIQUE
<130> 11079-EP-ETD
<150> PCT/GB2005/005085
   <151> 2004-12-23
<150> EP05823505.2
   <151> 2004-12-23
<160> 45
<170> PatentIn version 3.5
<210> 1
   <211> 24
   <212> PRT
   <213> Artificial
<220>
   <223> Modified fusion leader
<400> 1
   Met Lys Trp Val Phe Ile Val Ser Ile Leu Phe Leu Phe Ser Ser Ala
   1 5 10 15
   Tyr Ser Arg Ser Leu Asp Lys Arg
   20
<210> 2
   <211> 18
   <212> PRT
   <213> Artificial
<220>
   <223> Modified HSA(pre) leader sequence
<400> 2
   Met Lys Trp Val Phe Ile Val Ser Ile Leu Phe Leu Phe Ser Ser Ala
   1 5 10 15
   Tyr Ser
<210> 3
   <211> 90
   <212> DNA
   <213> Artificial
<220>
   <223> DS248
<400> 3
   gtcagaattc gagctctacg tattaattaa ggccggccag gcccgggcta gtctcttttt 60
   ccaatttgcc accgtgtagc attttgttgt 90
<210> 4
   <211> 59
   <212> DNA
   <213> Artificial
<220>
   <223> DS249
<400> 4
   gtcaggatcc tacgtacccg gggatatcat tatcatcttt gtcgtggtca tcttgtgtg 59
<210> 5
   <211> 54
   <212> DNA
   <213> Artificial
<220>
   <223> DS250
<400> 5
   gtcaggatcc tacgtacccg ggtaaggcgt tcgtgcagtg tgacgaatat agcg 54
<210> 6
   <211> 99
   <212> DNA
   <213> Artificial
<220>
   <223> DS251
<400> 6
   gtcagaattc gagctctacg tattaattaa ggccggccag gcccgggccc gtatggacat 60
   acatatatat atatatatat atatatattt tgttacgcg 99
<210> 7
   <211> 90
   <212> DNA
   <213> Artificial
<220>
   <223> DS252
<400> 7
   gtcagaattc gagctctacg tattaattaa ggccggccag gcccgggctt gttgcaagca 60
   gcatgtctaa ttggtaattt taaagctgcc 90
<210> 8
   <211> 103
   <212> DNA
   <213> Artificial
<220>
   <223> DS267
<400> 8
   gtcagaattc gagctctacg tattaattaa ggccggccag gcccgggccc gtatggacat 60
   acatatatat atatatatat atatatatat attttgttac gcg 103
<210> 9
   <211> 17
   <212> DNA
   <213> Artificial
<220>
   <223> DS253
<400> 9
   cctccctgct gctcgcc 17
<210> 10
   <211> 18
   <212> DNA
   <213> Artificial
<220>
   <223> DS254
<400> 10
   ctgtaagaac atggctcc 18
<210> 11
   <211> 18
   <212> DNA
   <213> Artificial
<220>
   <223> DS255
<400> 11
   ctcgatcgat tacgaggg 18
<210> 12
   <211> 17
   <212> DNA
   <213> Artificial
<220>
   <223> DS256
<400> 12
   aagaaagccg atatcgc 17
<210> 13
   <211> 18
   <212> DNA
   <213> Artificial
<220>
   <223> DS257
<400> 13
   caactctctg aagaggcg 18
<210> 14
   <211> 17
   <212> DNA
   <213> Artificial
<220>
   <223> DS258
<400> 14
   caacgccaca tccgacg 17
<210> 15
   <211> 19
   <212> DNA
   <213> Artificial
<220>
   <223> DS259
<400> 15
   gtaattctga tcactttgg 19
<210> 16
   <211> 21
   <212> DNA
   <213> Artificial
<220>
   <223> DS260
<400> 16
   gcacttatta ttactacgtg g 21
<210> 17
   <211> 19
   <212> DNA
   <213> Artificial
<220>
   <223> DS261
<400> 17
   gttttccttg atgaagtcg 19
<210> 18
   <211> 18
   <212> DNA
   <213> Artificial
<220>
   <223> DS262
<400> 18
   gtgaccacac catggggc 18
<210> 19
   <211> 18
   <212> DNA
   <213> Artificial
<220>
   <223> DS263
<400> 19
   gttgccggcg tgtctgcc 18
<210> 20
   <211> 18
   <212> DNA
   <213> Artificial
<220>
   <223> DS264
<400> 20
   ttgaaatcat cgtctgcg 18
<210> 21
   <211> 17
   <212> DNA
   <213> Artificial
<220>
   <223> DS265
<400> 21
   cggcagttct aggtccc 17
<210> 22
   <211> 18
   <212> DNA
   <213> Artificial
<220>
   <223> DS266
<400> 22
   ccacagcctc ttgttggg 18
<210> 23
   <211> 17
   <212> DNA
   <213> Artificial
<220>
   <223> M13/pUC Primer (-40)
<400> 23
   gttttcccag tcacgac 17
<210> 24
   <211> 38
   <212> DNA
   <213> Artificial
<220>
   <223> DS299
<400> 24
   cgtagcggcc gcctgaaagg ggttgaccgt ccgtcggc 38
<210> 25
   <211> 39
   <212> DNA
   <213> Artificial
<220>
   <223> DS300
<400> 25
   cgtaaagctt cgccgcccga cagggtaaca tattatcac 39
<210> 26
   <211> 38
   <212> DNA
   <213> Artificial
<220>
   <223> DS301
<400> 26
   cgtaaagctt gaccacgtag taataataag tgcatggc 38
<210> 27
   <211> 41
   <212> DNA
   <213> Artificial
<220>
   <223> DS302
<400> 27
   cgtactgcag attggatagt gattagagtg tatagtcccg g 41
<210> 28
   <211> 18
   <212> DNA
   <213> Artificial
<220>
   <223> DS303
<400> 28
   ggagcgacaa acctttcg 18
<210> 29
   <211> 20
   <212> DNA
   <213> Artificial
<220>
   <223> DS304
<400> 29
   accgtaataa aagatggctg 20
<210> 30
   <211> 24
   <212> DNA
   <213> Artificial
<220>
   <223> DS305
<400> 30
   catcttgtgt gtgagtatgg tcgg 24
<210> 31
   <211> 18
   <212> DNA
   <213> Artificial
<220>
   <223> DS306
<400> 31
   cccaggataa ttttcagg 18
<210> 32
   <211> 29
   <212> DNA
   <213> Artificial
<220>
   <223> DS230
<400> 32
   tagcgaattc aatcagtaaa aatcaacgg 29
<210> 33
   <211> 30
   <212> DNA
   <213> Artificial
<220>
   <223> DS231
<400> 33
   gtcaaagctt caaaaaaaga aaagctccgg 30
<210> 34
   <211> 38
   <212> DNA
   <213> Artificial
<220>
   <223> DS232
<400> 34
   tagcggatcc gaattcggcg gttgtttgca agaccgag 38
<210> 35
   <211> 34
   <212> DNA
   <213> Artificial
<220>
   <223> DS233
<400> 35
   gtcaaagctt taaagataat gctaaatcat ttgg 34
<210> 36
   <211> 35
   <212> DNA
   <213> Artificial
<220>
   <223> DS234
<400> 36
   tgacaagctt tcggtcgaaa aaagaaaagg agagg 35
<210> 37
   <211> 31
   <212> DNA
   <213> Artificial
<220>
   <223> DS235
<400> 37
   tgacaagctt gatcttttat gcttgctttt c 31
<210> 38
   <211> 18
   <212> DNA
   <213> Artificial
<220>
   <223> DS236
<400> 38
   aatagttcag gcactccg 18
<210> 39
   <211> 17
   <212> DNA
   <213> Artificial
<220>
   <223> DS237
<400> 39
   tggaaggcaa gagagcc 17
<210> 40
   <211> 18
   <212> DNA
   <213> Artificial
<220>
   <223> DS238
<400> 40
   taaaatgtaa gctctcgg 18
<210> 41
   <211> 17
   <212> DNA
   <213> Artificial
<220>
   <223> DS239
<400> 41
   ccaaccaagt atttcgg 17
<210> 42
   <211> 20
   <212> DNA
   <213> Artificial
<220>
   <223> CED005
<400> 42
   gagctgacag ggaaatggtc 20
<210> 43
   <211> 20
   <212> DNA
   <213> Artificial
<220>
   <223> CED006
<400> 43
   tacgaggata cggagagagg 20
<210> 44
   <211> 44
   <212> DNA
   <213> Artificial
<220>
   <223> LRE49
<400> 44
   gctagcgtcg acaagcttgc ggcgcggtac cgtgtggaag aacg 44
<210> 45
   <211> 20
   <212> DNA
   <213> Artificial
<220>
   <223> CF247
<400> 45
   gcgcgttttc attagtgccc 20